(19) Europäisches Patentamt
European Patent Office
Office européen des brevets

(11) Veröffentlichungsnummer: **0 257 378 B1**

(12) # EUROPÄISCHE PATENTSCHRIFT

(45) Veröffentlichungstag der Patentschrift: **03.07.91**

(51) Int. Cl.5: **C07H 19/06**, C07D 405/04, C07H 19/073, C07H 19/09, //A61K31/70,A61K31/505, A61K31/34

(21) Anmeldenummer: **87111280.1**

(22) Anmeldetag: **04.08.87**

(54) **Pyrimidinderivate, ihre Herstellung und Medikamente, die diese Derivate enthalten.**

(30) Priorität: **18.08.86 GB 8620070**
**14.05.87 GB 8711336**

(43) Veröffentlichungstag der Anmeldung:
**02.03.88 Patentblatt 88/09**

(45) Bekanntmachung des Hinweises auf die Patenterteilung:
**03.07.91 Patentblatt 91/27**

(84) Benannte Vertragsstaaten:
**AT BE CH DE ES FR GB GR IT LI LU NL SE**

(56) Entgegenhaltungen:
**DE-A- 3 045 342**

**Chemical Abstracts, Band 100, Nr. 19, 7. Mai 1984, Columbus, Ohio, USA Seite 565, Zusammenfassung-Nr. 156 925z (Montgomery et al.) & J. Med. Chem. 1984, 27(5), 680-4**

(73) Patentinhaber: **F. HOFFMANN-LA ROCHE AG**
**Postfach 3255**
**CH-4002 Basel(CH)**

(72) Erfinder: **Lambert, Robert Wilson**
**33 New Road Digswell**
**Welwyn§Herts(GB)**
Erfinder: **Martin, Joseph Armstrong**
**10 The Chownes West Common**
**Harpenden Herts(GB)**
Erfinder: **Thomas, Gareth John**
**2B Woodland Way Oaklands**
**Welwyn Herts(GB)**

(74) Vertreter: **Lederer, Franz, Dr. et al**
**Patentanwalt Dr. Franz Lederer Lucile-Grahn-Strasse 22**
**W-8000 München 80(DE)**

**EP 0 257 378 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Chemical Abstracts, Band 82, Nr. 3, 20 Jänner 1975, Columbus, Ohio, USA Seite 523, Zusammenfassung-Nr. 17 034e (GAIT et al.) & J. Chem. Soc., Perkin Trans. 1, 1974, (14) 1684-6

Chemical Abstracts, Band 98, Nr. 17, 25 April 1983, Columbus, Ohio, USA, Seite 206, Zusammenfassung-Nr. 137 209t (Markham et al.) & Antiviral Res. 1982, 2(6), 319-30

Chemical Abstracts, Band 106, Nr. 19, 11 Mai 1987, Columbus, Ohio, USA, Seite 723, Zusammenfassung Nr 156 807m (ELLIOT et al.) & J. Med. Chem.1987, 30(5) 927-30

Chemical Abstracts, Band 84, Nr. 15, 12 April 1976, Columbus, Ohio, USA, Seite 18, Zusammenfassung Nr 99 155y (Cheng Yung-Chi et al.) & Ann. N.Y. Acad. Sci. 1975, 255, 332-41

Chemical Abstracts, Band 104, Nr. 23, 9. Juni 1986, Columbus, Ohio, USA, Seite 791, Zusammenfassung-Nr. 207 594h (Elliot et al.) & J. Med. Chem.1986, 29(6), 1052-6

Chemical Abstracts, Band 90, Nr. 15, 9 April 1979, Columbus, Ohio, USA. Seiten 685-686, Zusammenfassung-Nr. 121 985s (LIN TAI-SHUN et al.)

## Beschreibung

Die vorliegende Erfindung betrifft Pyrimidinderivate, ein Verfahren zu deren Herstellung und Medikamente, die diese Derivate enthalten.

Die erfindungsgemässen Pyrimidinderivate sind Verbindungen der allgemeinen Formel

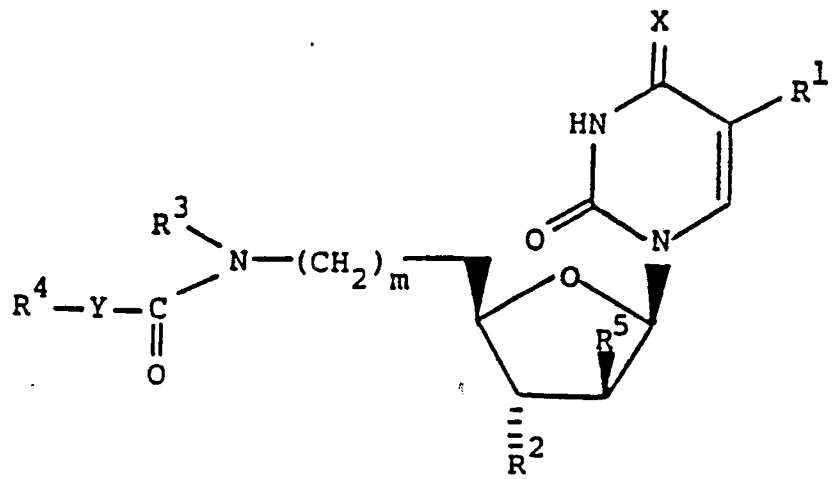

I

worin

- $R^1$ Halogen, $C_{1-4}$-Alkyl, Halo-($C_{1-4}$-alkyl) oder $C_{2-4}$-Alkanoyl,
- $R^2$ Wasserstoff, Hydroxy, $C_{1-4}$-Alkoxy, $C_{1-4}$-Alkylthio oder Phenyl-($C_{1-4}$-alkoxy), oder falls X Sauerstoff ist auch Acyloxy;
- $R^3$ Wasserstoff oder $C_{1-4}$ -Alkyl,
- $R^4$ eine carbocyclische Gruppe, nämlich eine monocyclische oder polycyclische aromatische Kohlenwasserstoffgruppe, die bis zu 14 Kohlenstoffatome in der cyclischen Struktur enthält und die einen oder mehrere Substituenten aus der Gruppe Halogen, Hydroxy, $C_{1-4}$ -Alkyl, $C_{1-4}$ -Alkoxy, Trifluormethyl, Phenyl, $C_{1-4}$-Alkylphenyl, Halophenyl, Nitro, Amino, Acylamino, Benzyloxy und O-Phosphat enthalten kann, oder eine monocyclische oder polycyclische Cycloalkylgruppe, die bis zu 13 Kohlenstoffatome in der cyclischen Struktur enthält und die, falls sie monocyclisch ist, einen oder zwei kondensierte Benzolringe tragen kann, oder eine heterocyclische Gruppe,
- $R^5$ Wasserstoff oder Fluor,
- m 0, 1 oder 2,
- X Sauerstoff oder NH,
- Y eine direkte Bindung, -CH=CH-, -C≡C- oder eine Gruppe der Formel

  $(Z)_n$ -A- (a)

  worin A eine $C_{1-8}$ -Alkylengruppe darstellt, die durch ein oder zwei Phenylgruppen substituiert sein kann,
- Z Sauerstoff, Schwefel, SO oder $SO_2$ und n 0 oder 1 bedeuten, mit der Voraussetzung, dass $R^1$ von Jod verschieden ist, wenn $R^2$ Hydroxy oder Benzoyloxy, $R^3$ Wasserstoff, $R^4$ unsubstituiertes Phenyl, $R^5$ Wasserstoff, m 0, X Sauerstoff und Y eine direkte Bindung darstellen,

und deren Tautomeren.

Die obige Voraussetzung zwischen den Bedeutungen der Symbole $R^1$ bis Y schliesst die in Antiviral Research 2, 1982, 319-330, beschriebenen 5-Jodouridine 17 und 22 aus.

Im Gegensatz zu den obigen Verbindungen der Formel I, worin eine Phenylgruppe $R^4$ nicht durch Bromacetyl substituiert sein kann, enthält die in CA 100:156925z beschriebene Verbindung III eine Bromacetophenylgruppe.

Der hier verwendete Ausdruck "$C_{1-4}$ -Alkyl" bedeutet für sich allein oder in Kombination geradkettige oder verzweigte Alkylgruppen wie Methyl, Aethyl, Propyl, Isopropyl oder t-Butyl. Beispiele von "Halo-($C_{1-4}$-alkylgruppen)" sind Trifluormethyl und 2-Chloräthyl. Beispiele von "$C_{1-4}$-Alkylthiogruppen" sind Methylthio und Aethylthio. Beispiele von "$C_{1-4}$-Alkoxygruppen" sind Methoxy und Aethoxy. Beispiele von "$C_{2-4}$-Alkanoylgruppen" sind Acetyl, Propionyl und Butyryl. Benzyloxy kann als Beispiel einer Phenyl-($C_{1-4}$-

alkoxygruppe) erwähnt werden. Die Acyloxygruppe kann sich von aliphatischen, cycloaliphatischen, araliphatischen oder aromatischen Carbonsäuren ableiten. Beispiele solcher Säuren sind Ameisensäure, Essigsäure, Propionsäure, Buttersäure, t-Butylessigsäure, Palmitinsäure, Cyclopentylpropionsäure, Phenylessigsäure, Benzoesäure und 9-Fluorencarbonsäure.

Der Ausdruck "carbocyclische Gruppe" bezeichnet monocyclische und polycyclische aromatische Kohlenwasserstoffgruppen, die bis zu 14 Kohlenstoffatome in der cyclischen Struktur enthalten und die einen oder mehrere Substituenten aus der Gruppe Halogen, Hydroxy, $C_{1-4}$-Alkyl, $C_{1-4}$-Alkoxy, Trifluormethyl, Phenyl, $C_{1-4}$-Alkylphenyl, Halophenyl, Nitro, Amino, Acylamino, Benzyloxy und O-Phosphat enthalten (z.B. Phenyl, 2-Fluorphenyl, 2-Bromphenyl, 2-Chlorphenyl, 4-Bromphenyl, 2.6-Dichlorphenyl, 2,4-Dichlorphenyl, 3,5-Dichlorphenyl- 4-Tolyl, 2,3-Dimethylphenyl, 2,6-Dimethylphenyl, 2,3,5,6-Tetramethylphenyl, 2-Methoxyphenyl, 3,5-Dimethoxyphenyl, 2-Trifluormethylphenyl, 3-Tri-fluormethylphenyl, 4-Trifluormethylphenyl, 2-Nitrophenyl, 3-Nitrophenyl, 4-Nitrophenyl, 2-Aminophenyl, 2-Acetamidophenyl, 2-Biphenylyl, 4-Biphenylyl, 2-Chlor-3-nitrophenyl, 2-Chlor-4-nitrophenyl, 4-Hydroxy-2,6-dimethylphenyl, 3-Chlor-4-biphenylyl, 1-Naphthyl und 2-Naphthyl) und monocyclische und polycyclische Cycloalkylgruppen, die bis zu 13 Kohlenstoffatome in der cyclischen Struktur enthalten und die, falls sie monocyclisch sind, einen oder zwei kondensierte Benzolringe tragen können, z.B. Cyclopentyl, Cyclohexyl, Adamantyl, Indanyl und Fluorenyl.

Der Ausdruck "heterocyclische Gruppe" beinhaltet 5- und 6-gliedrige gesättigte teilweise ungesättigte und aromatische heterocyclische Gruppen die Sauerstoff, Stickstoff oder Schwefel enthalten, die einen kondensierten Benzolring enthalten können und die einen oder mehrere der im Zusammenhang mit der carbocyclischen Gruppe $R^4$ früher erwähnten Substituenten tragen können.

Beispiele solcher heterocyclischen Gruppen sind 2-Furyl, 2-Thienyl, 3-Thienyl, 2-Pyridyl, 3-Pyridyl, 2-Benzofuranyl, 2,3-Dihydro-2-benzofuranyl, 2-Benzothienyl, 2-Chinolyl und 2-Benzopyranyl.

Beispiele von "$C_{1-8}$-Alkylengruppen" sind $-CH_2-$, $-CH_2CH_2-$, $CH(CH_3)-$, $-C(CH_3)_2-$, $-CH(C_2H_5)-$, $-CH_2CH_2CH_2-$, $-CH_2-CH(CH_3)-$und $-CH_2CH_2CH_2CH_2-$.

Je nach der Bedeutung von A in der Formel I können die erfindungsgemässen Verbindungen als Diastereomere vorliegen, die ebenfalls Gegenstand der Erfindung sind.

In der obigen Formel I ist $R^1$ vorzugsweise Fluor, Chlor, Brom, $C_{1-4}$ -Alkyl oder Halo-($C_{1-4}$-alkyl), $R^4$ eine carbocyclische oder aromatische heterocyclische Gruppe, $R^5$ Wasserstoff, und m 0.

In der obigen Formel I ist ferner $R^1$ vorzugsweise $C_{1-4}$-Alkyl, insbesondere Aethyl oder Propyl, $R^2$ ist vorzugweise Hydroxy, $R^3$ ist vorzugsweise Wasserstoff. $R^4$ ist vorzugsweise Phenyl, das durch einen oder mehrere Substituenten aus der Gruppe Halogen, $C_{1-4}$-Alkyl, $C_{1-4}$-Alkoxy, Triflurormethyl, Phenyl und Nitro substituiert sein kann.

m ist vorzugsweise 0, X vorzugsweise Sauerstoff und Y vorzugsweise eine Gruppe (a).

Besonders bevorzugte Verbindungen der vorliegenden Erfindung sind die, in denen $R^1$ Aethyl, $R^2$ Hydroxy, $R^3$ Wasserstoff, $R^4$ 2-Bromphenyl, 2,6-Dichlorphenyl oder 4-Biphenylyl, m = 0, X Sauerstoff und Y eine Gruppe der Formel (a) ist, in der A $-CH_2-$ oder $-CH(CH_3)-$ ist und n gleich 0 ist. Weitere bevorzugte erfindungsgemässe Verbindungen sind diejenigen, in den $R^1$ Aethyl oder Propyl, $R^2$ Hydroxy, $R^3$ Wasserstoff, $R^4$ 2-Biphenylyl, 2,4-Dichlorphenyl, 2,4,5-Trichlorphenyl, 4-Chlor-2-nitrophenyl oder 2,4-Dichlor-5-methoxyphenyl ist, m gleich 0 ist, X Sauerstoff und Y eine Gruppe der Formel (a) ist, worin A $-CH(CH_3)-$ oder -CH(Phenyl)-, Z Sauerstoff und n gleich 1 ist.

Besonders bevorzugte erfindungsgemässe Verbindungen sind:

5' -[2-(2-Bromphenyl)acetamido]-2' ,5' -dideoxy-5-äthyluridin
5′-[2-(2,6-Dichlorphenyl)acetamido]-2′,5′-dideoxy-5-äthyluridin,
5′-[2(RS)-(2,4-Dichlorphenoxy)propionamido]-2′,5′-dideoxy-5-äthyluridin,
5′-[2-(4-Biphenylyl)acetamido]-2′,5′-dideoxy-5-äthyluridin,
2′,5′-Dideoxy-5-äthyl-5-′-[2(RS)-(2-phenylphenoxy)propionamido]uridin,
1-[5-[2(RS)-(2,4-Dichlorphenoxy)propionamido]-2,5-dideoxy-2-fluor-β-D-arabinofuranosyl]-5-äthyluracil,
5′-[2(RS)-(2,4,5-Trichlorphenoxy)propionamido]-2′,5′-dideoxy-5-äthyluridin,
5′-[2(RS)-(4-Chlor-2-nitrophenoxy)propionamido]-2′,5′-dideoxy-5-äthyluridin,
5′-[2(RS)-(2,4-Dichlorphenoxy)-2-phenylacetamido]-2′,5′-deoxy-5-äthyluridin,
5′-[2(RS)-(2,4-Dichlor-5-methoxyphenoxy)propionamido]-2′,5′-dideoxy-5-äthyluridin und
5′-[2(RS)-(2,4-Dichlorphenoxy)propionamido]-2′,5′-dideoxy-5-propyluridin.

Beispiele anderer interessierender Verbindungen der vorliegenden Erfindung sind:

2′,5′-Dideoxy-5-äthyl-5′-(2-phenylacetamido)uridin,
5′-(4-Brombenzamido)-5′-deoxythymidin,
5′-Deoxy-5′-(4-nitrobenzamido)thymidin,
5′-Benzamido-5′-deoxythymidin,
5′-Deoxy-5′-(2-fluorbenzamido)thymidin,

5'-Deoxy-5'-(2-nitrobenzamido)thymidin,
5'-[2-(2-Bromphenyl)acetamido]-5'-deoxythymidin,
5'-Deoxy-5'-[2-(4-nitrophenyl)acetamido]thymidin,
5'-Deoxy-5'-(2-phenylacetamido)thymidin,
5'-Deoxy-5'-[2-(4-trifluormäthylphenyl)acetamido]thymidin,
5'-Benzamido-2',5'-dideoxy-5-äthyluridin,
2',5'-Dideoxy-5-äthyl-5'-(2-fluorbenzamido)uridin,
5'-(2-Brombenzamido)-2',5'-dideoxy-5-äthyluridin,
2',5'-Dideoxy-5-äthyl-5'-(4-nitrobenzamido)uridin,
2',5'-Dideoxy-5-äthyl-5'-(2-trifluormethylbenzamido)uridin,
2',5'-Dideoxy-5-äthyl-5'-[2-(2,6-dimethylphenyl)acetamido]uridin,
5'-[2(RS)-(2-Bromphenyl)propionamido]-2',5'-dideoxy-5-äthyluridin,
5'-[2-Chlor-3-nitrophenyl)acetamido]-2',5'-dideoxy-5-äthyluridin,
5'-[2(RS)-(2,6-Dichlorphenyl)propionamido]-2',5'-dideoxy-5-äthyluridin,
2',5'-Dideoxy-5-äthyl-5'-[2-(3,5-dimethylphenyl)acetamido]uridin,
2',5'-Dideoxy-5'-[2-(3,5-dimethoxyphenyl)acetamido]-5-äthyluridin,
2',5'-Dideoxy-5-äthyl-5'-[2-(2,3,5,6-tetramethylphenyl)acetamido]uridin,
2',5'-Dideoxy-5-äthyl-5'-[2-(2-methoxyphenyl)acetamido]uridin,
2',5'-Dideoxy-5-äthyl-5'-[2-(2-nitrophenyl)]acetamidouridin,
5-Brom-5'-[2-(2-bromphenyl)acetamido]-2',5'--dideoxyuridin,
3'-O-Butyryl-5'-[2-(2,6-dichlorphenyl)acetamido]-2',5'-dideoxy-5-äthyluridin,
2',5'-Dideoxy-5'-[2-(2,6-dichlorphenyl)acetamido]-3'-O-(3,3-dimethylbutyryl)-5-äthyluridin,
5'-[2-(2,6-Dichlorphenyl)acetamido]-2',5'-dideoxy-5-äthyl-3'-O-palmitoyluridin,
3'-O-Acetyl-5'-[2-(2,6-dichlorphenyl)acetamido]-2',5'-dideoxy-5-äthyluridin,
3'-O-Benzyl-5'-[2(2,6-dichlorphenyl)acetamido]-2',5'-dideoxy-5-äthyluridin,
5'-[2-(2,6-Dichlorphenyl)acetamido]-2',5'-dideoxy-3'-O-äthyl-5-äthyluridin,
2',3',5'-Trideoxy-5'-[2-(2,6-dichlorphenyl)acetamido]-5-äthyluridin,
5'-[2-(2-Aminophenyl)acetamido]-2',5'-dideoxy-5-äthyluridin,
5'-[2-(2-Acetamidophenyl)acetamido]-3'-O-acetyl-2',5'-dideoxy-5-äthyluridin,
5'-[2-(2-Acetamidophenyl)acetamido]-2',5'-dideoxy-5-äthyluridin,
2',5'-Dideoxy-5-äthyl-5'-[2-(4-hydroxy-2,6-dimethylphenyl)acetamido]uridin,
5'-(2-Chloräthyl)-5'-[2-(2,6-dichlorphenyl)acetamido]-2',5'-dideoxyuridin,
5'-[2-(2-Bromphenyl)-N-methylacetamido]-2',5'-dideoxy-5-äthyluridin,
5'-[2-(2,6-Dichlorphenyl)-N-methylacetamido]-2',5'-dideoxy-5-äthyluridin,
5'-[2(RS)-(2-Bromphenyl)-N-methylpropionamido]-2',5'-dideoxy-5-äthyluridin,
3'-O-Acetyl-5'-[2-(2-bromphenyl)-N-methylacetamido]-2',5'-dideoxy-5-äthyluridin,
2',5'-Dideoxy-5-äthyl-5'-(2-naphthalamido)uridin,
2',5'-Dideoxy-5-äthyl-5'-(2-phenylbenzamido)uridin,
2',5'-Dideoxy-5-äthyl-5'-(3-phenylpropionamido)uridin,
2',5'-Dideoxy-5-äthyl-5'-(4-phenylbutyramido)uridin,
2',5'-Dideoxy-5'-cinnamamido-5-äthyluridin,
2',5'-Dideoxy-5-äthyl-5'-(3-phenyl-2-propynamido)uridin,
2',5'-Dideoxy-5-äthyl-5'-[3-(phenylsulphonyl)propionamido]uridin,
5'-(2-Bromcinnamamido)-2',5'-dideoxy-5-äthyluridin,
2',5'-Dideoxy-5-äthyl-5'-[2-(2-thienyl)acetamido]uridin,
2',5'-Dideoxy-5-äthyl-5'-[2-(3-thienyl)acetamido]uridin,
5'-(1-Adamantylcarboxamido)-2',5'-dideoxy-5-äthyluridin,
2',5'-Dideoxy-5-äthyl-5'-(2-pyridylcarboxamido)uridin,
2',5'-Dideoxy-5-äthyl-5'-(3-pyridylcarboxamido)uridin,
2',5'-Dideoxy-5-äthyl-5'-[2-(phenylsulphonyl)acetamido]uridin,
2',5'-Dideoxy-5-äthyl-5'-(9-fluorenylcarboxamido)uridin,
3'-O-Acetyl-2',5'-dideoxy-5-äthyl-5'-(9-fluorenylcarboxamido)uridin,
3'-O-Butyryl-2',5'-Dideoxy-5-äthyl-5'-(9-fluorenylcarboxamido)uridin,
2',5'-Dideoxy-5-äthyl-3'-O-(3,3-dimethylbutyryl)-5'-(9-fluorenylcarboxamido)uridin,
2',5'-Dideoxy-5-äthyl-5'-(9-fluorenylcarboxamido)-3'-O-(hexadecanoyl)uridin,
2',5'-Dideoxy-5-äthyl-3'-O-(9-fluorenylcarbonyl)-5'-(9-fluorenylcarboxamido)uridin,
2',5'-Dideoxy-5-äthyl-5'-(2-phenylbutyramido)uridin,
2',5'-Dideoxy-5-äthyl-5'-(3-phenylbutyramido)uridin,

2',5'-Dideoxy-5-äthyl-5'-(2,2-diphenylacetamido)uridin,
5'-(2-Cyclohexyl-2-phenylacetamido)-2',5'-dideoxy-5-äthyluridin,
2',5'-Dideoxy-5'-äthyl-5'-(triphenylacetamido)uridin,
2',5'-Dideoxy-5-äthyl-5'-(2-methyl-2-phenylpropionamido)uridin,
2',5'-Dideoxy-5-äthyl-5'-[2(RS)-phenylpropionamido)]uridin,
5'-[2(RS)-(2,4-Dichlorphenoxy)propionamido]-2',5'-dideoxy-5-äthyluridin,
5'-[2(RS)-(2,6-Dichlorphenoxy)propionamido]-2',5'-dideoxy-5-äthyluridin,
5'-[2(RS)-(3,5 -Dichlorphenoxy)propionamido]-2',5'-dideoxy-5-äthyluridin,
5'-[2(RS)-(2-Chlorphenoxy)propionamido]-2',5'-dideoxy-5-äthyluridin,
5'-[2-(2,6-Dichlorphenoxy)acetamido]-2',5'-dideoxy-5-äthyluridin,
5'-[2-(2,4-Dichlorphenoxy)acetamido]-2',5'-dideoxy-5-äthyluridin,
5'-[4-(2,6-Dichlorphenoxy)butyramido]-2',5'-dideoxy-5-äthyluridin,
5'-[6-(2,6-Dichlorphenoxy)hexanamido]-2',5'-dideoxy-5-äthyluridin,
5'-[5-(2,6-Dichlorphenoxy)valeramido]-2',5'-dideoxy-5-äthyluridin,
5'-[2(RS)-(2-Chlor-4-nitrophenoxy)propionamido]-2',5'-dideoxy-5-äthyluridin,
5'-[2(RS)-(2-Chlor-4-phenylphenoxy)propionamido]-2',5'-dideoxy-5-äthyluridin,
5-Brom-5'-[2-(2-bromphenyl)acetamido]-2',5'-dideoxycytidine and
5'-[2-(2-Bromphenyl)acetamido]-2',5'-dideoxy-5-äthylcytidin.
Beispiele weiterer interessierender erfindungsgemässer Verbindungen sind:
5'-[2-(2,6-Dichlorphenyl)acetamido]-5'-deoxythymidin,
5-Brom-5'-[2(RS)-(2,4-dichlorphenoxy)propionamido]-2',5'-dideoxyuridin,
5-Brom-5'-[2-(2,6-dichlorphenyl)acetamido]-2',5'-dideoxyuridin,
5'-Benzamido-5-brom-2',5'-dideoxyuridin,
5'-[2(RS)-Dichlorphenoxy)propionamido]-2',5'-dideoxy-5-iodouridin,
5'-[2(RS)-(2,4,5-Trichlorphenoxy)propionamido]-2',5'-dideoxy-5-iodouridin,
5'-[2-(2,6-Dichlorphenyl)acetamido]-2',5'-dideoxy-5-iodouridin,
3'-O-Benzyl-5'-[2(RS)-(2,4-dichlorphenoxy)propionamido]-2',5'-dideoxy-5-äthyluridin,
5'-[2(RS)-(2,4-Dichlorphenoxy)propionamido]-2',5'-dideoxy-3'-O-äthyl-5-äthyluridin,
5'-[2(RS)-(2,4-Dichlorphenoxy)-N-methylpropionamido]-2',5'-dideoxy-5-äthyluridin,
5'-[2(R)-(2,4-Dichlorphenoxy)propionamido]-2',5'-dideoxy-5-äthyluridin,
5'-[2(S)-(2,4-Dichlorphenoxy)propionamido]-2',5'-dideoxy-5-äthyluridin,
5'-[2(RS)-(2,4-Dichlorphenoxy)butyramido]-2',5'-dideoxy-5-äthyluridin,
5'-[2(RS)-(4-Acetamido-2-chlorphenoxy)propionamido]-2',5'-dideoxy-5-äthyluridin,
5'-[2(RS)-(2-Chlor-4-methoxyphenoxy)propionamido]-2',5'-dideoxy-5-äthyluridin,
2',5'-Dideoxy-5-äthyl-5'-[2(RS)-(2-methylbiphenylyloxy)propionamido]uridin,
5'-[2(RS)-(2,4-Dichlorphenoxy)propionamido]-5'-deoxythymidin,
5'-[2-(2,4-Dichlorphenoxy)-2-methylpropionamido]-2',5'-dideoxy-5-äthyluridin,
2',5'-Dideoxy-5-äthyl-5'-[2(RS)-phenoxypropionamido]uridin,
2',5'-Dideoxy-5-äthyl-5'-[2(RS)-(2-fluorphenoxy)propionamido]uridin,
2',5'-Dideoxy-5-äthyl-5'-[2(RS)-(2-trifluormethylphenoxy)propionamido]uridin,
1-[5-[2(RS)-(2,4-Dichlorphenoxy)propionamido]-2,5-dideoxy-2-fluor-$\beta$-D-arabinofuranosyl]thymine,
5'-[2(RS)-(2,4-Dichlorphenylsulphinyl)propionamido]-2',5'-dideoxy-5-äthyluridin,
5'-[2(RS)-(2,4-Dichlorphenylsulphonyl)propionamido]-2',5'-dideoxy-5-äthyluridin,
5'-[2-(2,6-Dichlorphenylacetamido)äthyl]-2',5'-dideoxy-5-äthyluridin,
5'-[2-[2(RS)-(2,4,5-Trichlorphenoxy)propionamido]äthyl]-2',5'-dideoxy-5-äthyluridin,
5'-[2-[2(RS)-(2,4-Dichlorphenoxy)propionamido]äthyl]-2',5'-dideoxy-5-äthyluridin,
5'-[2(RS)-(2,6-Dichlorbenzyl)propionamido]-2',5'-dideoxy-5-äthyluridin,
5'-[2(RS)-(2,4-Dichlorbenzyl)propionamido]-2',5'-dideoxy-5-äthyluridin,
5'-[5-chlor-2,3-Dihydro-2(RS)-benzofuranylcarboxamido]-2',5'-dideoxy-5-äthyluridin,
5-(6-chlor-2H-1-Benzopyran-2-ylcarboxamido)-2',5'-dideoxy-5-äthyluridin,
5'-[2-(4-Benzyloxy-2,6-dimethylphenyl)acetamido]-2',5'-dideoxy-5-äthyluridin,
3'-O-Acetyl-5'-[2-(4-benzyloxy-2,6-dimethylphenyl)acetamido]-2',5'-dideoxy-5-äthyluridin,
3'-O-Acetyl-2',5'-dideoxy-5-äthyl-5'-[2-(4-hydroxy-2,6-dimethylphenyl)acetamido]uridin,
2',5'-Dideoxy-5-äthyl-5'-[2-(2,6-dimethyl-4-phosphatophenyl)acetamido]uridin,
5-(2-Chloräthyl)-2',5'-dideoxy-5'-[2-(2,4-dichlorphenoxy)propionamido]uridin,
5'-Benzamido-5-(2-chloräthyl)-2',5'-dideoxyuridin,
5-(2-Chloräthyl-5'-[2-(2,4,5-trichlorphenoxy)propionamido]-2',5'-dideoxyuridin,
5'-[2-(2,6-Dichlorphenyl)acetamido]-2',5'-dideoxy-5-propyluridin,

5'-[2(RS)-(2,4-Dichlorphenoxy)propionamido]-2',5'-dideoxy-5-propyluridin und
5-Acetyl-5'-[2-(2,6-dichlorphenyl)acetamido]-2',5'-dideoxyuridin.

Die Verbindungen der obigen Formel I und deren Tautomere können erfindungsgemäss dadurch hergestellt werden, dass man

a) eine Verbindung der allgemeinen Formel

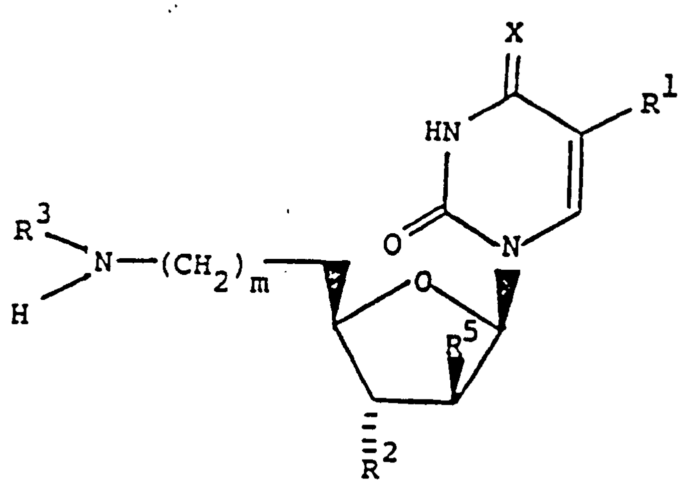

worin $R^1$, $R^2$, $R^3$ $R^5$, m und X die obige Bedeutung haben,

oder ein Tautomer davon, in der eine gegebenenfalls anwesende 4-Aminogruppe gewünschtenfalls geschützt ist, mit einem reaktionsfähigen Derivat einer Säure der allgemeinen Formel

$R^4$-Y-COOH III

worin $R^4$ und Y die obige Bedeutung haben,

umsetzt und eine im Produkt gegebenenfalls anwesende Schutzgruppe abspaltet oder

b) zur Herstellung von Verbindungen der Formel I oder einem Tautomeren davon, in denen X Sauerstoff und $R^2$ Acyloxy darstellt, eine Verbindung der Formel I oder ein Tautomer davon, in der X Sauerstoff und $R^2$ Hydroxy ist, acyliert, oder

c) zur Herstellung einer Verbindung der Formel I oder einem Tautomeren davon, in der X Sauerstoff und $R^2$ Hydroxy ist, eine Verbindung der Formel I oder ein Tautomer davon, in der X Sauerstoff und $R^2$ Acyloxy ist, deacyliert, oder

d) zur Herstellung einer Verbindung der Formel I oder einem Tautomeren davon, in der Y eine Gruppe der Formel (a), Z SO oder $SO_2$ und n 1 ist, eine Verbindung der Formel I oder ein Tautomer davon, in der Y eine Gruppe der Formel (a), Z Schwefel und n 1 ist, oxydiert, und

e) gewünschtenfalls einen in $R^4$ anwesenden reaktionsfähigen Substituenten funktionell abwandelt.

Eine 4-Aminogruppe, die in einem Ausgangsmaterial in der Verfahrensvariante (a) anwesend ist (d.h. in einem Cytidinderivat) kann durch eine leicht spaltbare Schutzgruppe, insbesondere eine Acylgruppe, vor allem eine Benzoylgruppe, geschützt werden.

Das reaktionsfähige Derivat einer Säure der Formel III kann jedes konventionelle reaktionsfähige Derivat sein, beispielsweise ein Säurehalogenid, ein Säureanhydrid oder ein reaktionsfähiges Derivat, das durch Aktivierung der Säure mit Dicyclohexylcarbodiimid oder einem ähnlichen aktivierenden Reagens gebildet wird. Vorzugsweise werden Säurehalogenide, insbesondere Säurechloride, als reaktionsfähige Derivate eingesetzt.

Die Umsetzung gemäss Verfahrensvariante a) kann in an sich bekannter Weise durchgeführt werden. Die Reaktion kann in Gegenwart oder in Abwesenheit eines inerten organischen Lösungsmittels durchgeführt werden. Wenn ein solches Lösungsmittel verwendet wird, ist dies zweckmässig ein Aether wie Diäthyläther, oder ein aromatischer Kohlenwasserstoff wie Benzol.

Die Reaktion wird zweckmässig in Gegenwart einer anorganischen Base wie einem Alkalimetallhydroxid (z.B. Natriumhydroxid oder Kaliumhydroxid) oder einer tertiären organischen Base (z.B. Pyridin) durchgeführt. Zweckmässig wird die Reaktion bei Temperaturen zwischen etwa 0 °C und Raumtemperatur vorgenommen.

Falls das Ausgangsmaterial eine geschützte 4-Aminogruppe enthält, wird die Schutzgruppe aus dem Reaktionsprodukt in üblicher Weise abgespalten. Beispielsweise kann eine Acylgruppe, wie die Benzoylgruppe, durch Behandlung mit alkoholischer Ammoniaklösung, insbesondere mit methanolischer Ammoniaklösung bei etwa Raumtemperatur, abgespalten werden.

Die Acylierung gemäss Verfahrensvariante b) kann ebenfalls in an sich bekannter Weise durchgeführt werden, beispielsweise unter Verwendung eines geeigneten reaktionsfähigen Derivats einer Säure, wie einem Säurehalid, z.B. dem Säurechlorid oder einem Säureanhydrid. Zweckmässig wird die Acylierung in Gegenwart einer Base, z.B. einer tertiären organischen Base wie Pyridin oder 4-Dimethylaminopyridin und bei Temperaturen zwischen etwa 0 °C und Raumtemperatur durchgeführt.

Die Deacylierung gemäss Verfahrensvariante c) kann ebenfalls in an sich bekannter Weise durchgeführt werden, beispielsweise unter Verwendung einer alkoholischen Ammoniaklösung, z.B. methanolischer Ammoniaklösung bei etwa Raumtemperatur.

Die Oxidation gemäss Verfahrensvariante d) kann ebenfalls in an sich bekannter Weise durchgeführt werden. Beispielsweise kann die Oxidation mittels einer organischen Persäure wie Peressigsäure, Perbenzoesäure, m-Chlorperbenzoesäure oder Perphthalsäure durchgeführt werden, zweckmässig in einem geeigneten Lösungsmittel wie einem halogenierten Kohlenwasserstoff, z.B. Chloroform oder einer Alkancarbonsäure, z.B. Essigsäure, und bei Temperaturen zwischen etwa 0 °C und Raumtemperatur. Falls Peressigsäure für die Oxidation verwendet wird, wird diese zweckmässig in situ aus Eisessig und Hydrogenperoxid hergestellt. Wenn ein Aequivalent einer organischen Persäure angewandt wird, erhält man eine Verbindung der Formel I oder ein Tautomer davon, in der Z -SO- darstellt wogegen die Anwendung von 2 Aequivalenten einer organischen Persäure zu Verbindungen der Formel I oder Tautomeren davon führt, in denen Z $-SO_2-$ ist.

Gemäss Verfahrensvariante e) kann ein in $R^4$ enthaltener reaktionsfähiger Substituent funktionell abgewandelt werden. Solche Abwandlungen können gemäss bekannten Verfahren ausgeführt werden. Falls beispielsweise $R^4$ eine Nitrogruppe enthält, kann diese katalytisch zu einer Aminogruppe reduziert werden. Falls $R^4$ eine Aminogruppe enthält, kann diese zu einer Acylaminogruppe acyliert werden. Falls $R^4$ eine Benzyloxygruppe enthält, kann diese in eine Hydroxygruppe durch Debenzylierung mittels katalytischer Hydrierung umgewandelt werden. Falls $R^4$ eine Hydroxygruppe enthält, kann diese in ein O-Phosphat durch Behandlung mit Dibenzylphosphorylchlorid und anschliessende Debenzylierung durch katalytische Hydrierung umgewandelt werden.

Die im erfindungsgemässen Verfahren eingesetzten Ausgangsmaterialien sind bekannte Verbindungen oder Analoge bekannter Verbindungen, die in ähnlicher Weise wie die bekannten Verbindungen hergestellt werden können. Weiterhin enthalten gewisse der nachfolgenden Beispiele detaillierte Informationen über die Herstellung der entsprechenden Ausgangsmaterialien.

Die Verbindungen der Formel I und deren Tautomere sind gegen Viren wirksam und können zur Kontrolle oder Verhütung viraler Infektionen, beispielsweise viraler Herpes simplex-Infektionen verwendet werden.

Die in vitro-Aktivität der Verbindung der Formel I und deren Tautomeren bei der Hemmung von Herpes simplex-Virus Typ 2 (HSV-2)-Thymidinkinase kann mittels des folgenden Testverfahrens gezeigt werden:

In diesem Test enthält ein Versuchsgemisch 50 mM Tris-HCl, pH 8, 5 mM Magnesiumchlorid, 5 mM ATP, 0,3 $\mu$M $^3$H-Thymidin (50 Ci/mMol), in geeigneter Weise verdünnten Thymidinkinaseextrakt und verschiedene Konzentrationen von Verbindungen der Formel I oder Tautomeren davon in einem Gesamtvolumen von 100 $\mu$l. Die Testlösungen werden 30 Minuten bei 37 °C inkubiert und die Reaktion durch 2 Minuten langes Eintauchen in kochendes Wasser beendet. Von jeder Testlösung werden dann 85 $\mu$l auf Cellulosepapier-Discs getrocknet und das unphosphorylierte $^3$H-Thymidin durch Waschen in 4 mM Ammoniumformiat entfernt. Die an die Discs gebunden gebliebene Radioaktivität wird dann durch Szintillationsspektrophotometrie gemessen. Der Grad der Hemmung bei jeder Konzentration der Verbindung der Formel I wird als Prozentsatz der Kontrollreaktion (100%) nach Abzug eines gemessenen Blindwertes ausgedrückt, der die Menge Radioaktivität darstellt, die an die Discs aus einem Versuch mit Hitze-inaktivierten Enzymen gebunden wird. Der $IC_{50}$-Wert, nämlich die Konzentration der Verbindung der Formel I oder dessen Tautomeren, die die Enzymaktivität zu 50% hemmt, wird dann berechnet. Die mit repräsentativen Verbindungen der Formel I erhaltenen Resultate sind in der nachfolgenden Tabelle zusammengestellt:

Tabelle

| Verbindung der Formel I | $IC_{50}$ (µM) |
|---|---|
| A | 0.02 |
| B | 0.003 |
| C | 0.0037 |
| D | 0.07 |
| E | 0.005 |
| F | 0.004 |
| G | 0.0013 |
| H | 0.0027 |
| I | 0.0047 |
| J | 0.0012 |
| K | 0.0035 |

Verbindung A: 5' -[2-(2-Bromphenyl)acetamido]-2',5'-dideoxy-5-äthyluridin.
Verbindung B: 5' -[2-(2,6-Dichlorphenyl)acetamido]-2',5'-dideoxy-5-äthyluridin.
Verbindung C: 5'-[2(RS)-(2,4-Dichlorphenoxy)propionamido]-2' ,5' -dideoxy-5-äthyluridin.
Verbindund D: 5' -[2-(4-Biphenylyl)acetamido]-2',5'-dideoxy-5-äthyluridin.
Verbindung E: 2' ,5' -Dideoxy-5-äthyl-5'-[2(RS)-(2-phenyl phenoxy)propionamido]uridin.
Verbindung F: 1-[5-[2(RS)-(2,4-Dichlorphenoxy)propionamido]-2, 5-dideoxy-2-fluoro-ß-D-arabinofuranosyl]-5-äthyluracil.
Verbindung G: 5'[2(RS)-(2,4,5-Trichlorphenoxy)propionamido]-2 ' ,5' -dideoxy-5-äthyluridin.
Verbindung H: 5'-[2(RS)-(4-Chloro-2-nitrophenoxy)propionamido]-2',5'-dideoxy-5-äthyluridin.
Verbindung I: 5'-[2(RS)-(2,4-Dichlorphenoxy)-2-phenylacetamido]-2',5'-deoxy-5-äthyluridin.
Verbindung J: 5'-[2(RS)-(2,4-Dichlor-5-methoxyphenoxy)propionamido]-2',5'-dideoxy-5-äthyluridin.
Verbindung K: 5'-[2(RS)-(2,4-Dichlorphenoxy)propionamido]-2',5'-dideoxy-5-propyluridin.

Die Verbindungen der Formel I und deren Tautomere können in Form pharmazeutischer Präparate, die diese Verbindungen zusammen mit einem verträglichen pharmazeutischen Träger enthalten, als Medikamente verwendet werden. Die Träger können organische oder anorganische Materialien sein, die für enterale, z.B. orale, oder parenterale Verabreichung geeignet sind. Beispiele solcher Träger sind Wasser, Gelatine, Gummi arabicum, Lactose, Stärke, Magnesiumstearat, Talkum, vegetabile Oele, Polyalkylenglykole und Vaseline. Die pharmazeutischen Präparate können in fester Form, z.B. als Tabletten, Dragées, Suppositorien oder Kapseln, oder in flüssiger Form, z.B. als Lösungen, Suspensionen oder Emulsionen vorliegen; sie können üblichen pharmazeutischen Operationen, z.B. Sterilisierung unterworfen werden und/oder können Hilfsstoffe, z.B. Konservierungs-, Stabilisierungs-, Befeuchtungsmittel oder Emulgatoren, Salze zur Veränderung des osmotischen Druckes oder Puffer enthalten. Sie können auch andere therapeutisch wertvolle Substanzen enthalten.

Die Verbindungen der Formel I und deren Tautomere können an Erwachsene in einer täglichen Dosierung von etwa 1-1000 mg, vorzugsweise etwa 5-500 mg verabreicht werden. Die tägliche Dosis kann in einer Einzeldosis oder in Teildosen verabreicht werden. Der obige Dosierungsbereich ist nur beispielhaft zu verstehen und kann nach oben und unten in Abhängigkeit von Faktoren wie der einzelnen zu verabreichenden Verbindung, der Verabreichungsart, der Schwere der zu behandelnden Erkrankung und dem Zustand des Patienten variiert werden.

Beispiel 1

A) Eine Lösung von 219 mg 4-Brombenzoylchlorid in 5 ml Diäthyläther wurde zu einer Lösung von 241 mg 5'-Amino-5'-deoxythymidin in 4 ml 0,25M Natriumhydroxidlösung gegeben und das Gemisch wurde 10 Minuten kräftig geschüttelt. Das Gemisch wurde filtriert, der Feststoff mit 10 ml Wasser und 4 ml Diäthyläther gewaschen und aus Aethanol umkristallisiert. Man erhielt 195 mg 5'-(4-Brombenzamido)-5'-deoxythymidin vom Schmelzpunkt 250,5-252° C.

B) In analoger Weise wurden erhalten:

a) aus 4-Nitrobenzoylchlorid und 5'-Amino-5'-deoxythymidin: 5'-Deoxy-5'-(4-nitrobenzamido)thymidin, Schmelzpunkt 230° C;

b) aus Benzoylchlorid und 5'-Amino-5'-deoxythymidin: 5'-Benzamido-5'-deoxythymidin, Schmelzpunkt 234-235° C;

c) aus 2-Fluorbenzoylchlorid und 5'-Amino-5'-deoxythymidin: 5'-Deoxy-5'-(2-fluorbenzamido)thymidin, Schmelzpunkt 228-228,5° C; und

d) aus 2-Nitrobenzoylchlorid und 5'-Amino-5'-deoxythymidin: 5'-Deoxy-5'-(2-nitrobenzamido)thymidin, Schmelzpunkt 190-191° C.

Beispiel 2

A) 140 mg Benzoylchlorid wurden zu einer Lösung von 255 mg 5'-Amino-2',5'-dideoxy-5-äthyluridin in 3 ml 0,33M Natriumhydroxidlösung gegeben. Das Gemisch wurde 5 Minuten kräftig geschüttelt, filtriert und der Feststoff mit 5 ml Wasser und 5 ml Diäthyläther gewaschen. Nach Umkristallisieren aus Aethanol wurden 140 mg 5'-Benzamido-2',5'-dideoxy-5-äthyluridin vom Schmelzpunkt 246-247° C erhalten.

B) In analoger Weise wurden erhalten:

a) aus 2-Fluorbenzoylchlorid und 5'-Amino-2',5'-dideoxy-5-äthyluridin:
2',5'-Dideoxy-5-äthyl-5'-(2-fluorbenzamido)uridin, Schmelzpunkt 216-216.5° C;

b) aus 2-Brombenzoylchlorid und 5'-Amino-2',5'-dideoxy-5-äthyluridin:
5'-(2-Brombenzamido)-2',5'-dideoxy-5-äthyluridin, Schmelzpunkt 237-238° C;

c) aus 4-Nitrobenzoylchlorid und 5'-Amino-2',5'-dideoxy-5-äthyluridin:
2',5'-Dideoxy-5-äthyl-5'-(4-nitrobenzamido)uridin, Schmelzpunkt 250-250.5° C; und

d) aus 2-Trifluormethylbenzoylchlorid und 5'-Amino-2',5'-dideoxy-5-äthyluridin:
2',5'-Dideoxy-5-äthyl-5' -(2-trifluormethylbenzamido)uridin, Schmelzpunkt 263-264° C.

Beispiel 3

A) Eine Lösung von 1,12 g (2,6-Dichlorphenyl)acetylchlorid in 15 ml Diäthyläther wurde zu einer Lösung von 1,275 g 5'-Amino-2',5'dideoxy-5-äthyluridin in 15 ml 0,33M Natriumhydroxidlösung gegeben. Das Gemisch wurde 10 Minuten kräftig geschüttelt und dann filtriert. Der Feststoff wurde mit 150 ml Wasser, 20 ml Aethanol und 40 ml Aethyläther gewaschen und aus 2,5 l Aethanol umkristallisiert. Man erhielt 1,53 g 5'-[2-(2,6-Dichlorphenyl)acetamido]-2',5'-dideoxy-5-äthyluridin als weissen Feststoff vom Schmelzpunkt 296-297° C.

B) In analoger Weise wurden erhalten:

a) aus (2,6-Dimethylphenyl)acetylchlorid und 5'-Amino-2',5'-dideoxy-5-äthyluridin:
2',5'-Dideoxy-5-äthyl-5' -[2-(2,6-dimethylphenyl)acetamido]uridin, Schmelzpunkt 282° C;

b) aus 2(RS)-(2-Bromphenyl)propionylchlorid und 5'-Amino-2',5'-dideoxy-5-äthyluridin:
5'-[2(RS)-(2-Bromphenyl)propionamido]-2',5' -dideoxy-5-äthyluridin, Schmelzpunkt 236-236.5° C;

c) aus 4-Biphenylylacetylchlorid und 5'-Amino-2',5'-dideoxy-5-äthyluridin:
5'-[2-(4-Biphenylyl)acetamido]-2',5'-dideoxy -5-äthyluridin, Schmelzpunkt 244° C;

d) aus (2-Chloro-3-nitrophenyl)acetylchlorid und 5'-Amino-2',5'-dideoxy-5-äthyluridin:
5'-[2-(2-Chloro-3-nitrophenyl)acetamido]-2',5'-dideoxy-5-äthyluridin, Schmelzpunkt 240-241.5° C;

e) aus 2(RS)-(2,6-Dichlorophenyl)propionylchlorid und 5'-Amino-2',5'-dideoxy-5-äthyluridin:
5'-[2(RS)-(2,6-Dichlorophenyl)propionamido]-2',5'-dideoxy-5-äthyluridin, Schmelzpunkt 193-194° C;

f) aus (3,5-Dimethylphenyl)acetylchlorid und 5'-Amino-2',5'-dideoxy-5-äthyluridin:
2',5'-Dideoxy-5-äthyl-5'-[2 -(3,5-dimethylphenyl)acetamido]uridin, Schmelzpunkt 234-236° C;

g) aus (3,5-Dimethoxyphenyl)acetylchlorid und 5'-Amino-2',5'-dideoxy-5-äthyluridin:
2',5'-Dideoxy-5'-[2-(3,5-dimethoxyphenyl)acetamido]-5-äthyluridin, Schmelzpunkt 219-220.5° C;

h) aus (2,3,5,6-Tetramethylphenyl)acetylchlorid und 5'-Amino-2',5'-dideoxy-5-äthyluridin:
2',5'-Dideoxy-5-äthyl-5'-[2-(2,3,5,6-tetramethylphenyl)acetamido]uridin, Schmelzpunkt 288° C;

i) aus (2-Bromphenyl)acetylchlorid und 5'-Amino-2',5'-dideoxy-5-äthyluridin:

5'-[2-(2-Bromphenyl)acetamido]-2',5'-dideoxy -5-äthyluridin, Schmelzpunkt 247-248° C;

j) aus (2-Methoxyphenyl)acetylchlorid und 5'-Amino-2',5'-dideoxy-5-äthyluridin:
2',5'-Dideoxy-5-äthyl-5'-[2 -(2-methoxyphenyl)acetamido]uridin, Schmelzpunkt 222-224° C;

k) aus (2-Nitrophenyl)acetylchlorid und 5'-Amino-2',5'-dideoxy-5-äthyluridin:
2',5'-Dideoxy-5-äthyl-5' -[2-(2-nitrophenyl)acetamido]uridin, Schmelzpunkt 240-241 ° C;

l) aus Phenylacetylchlorid und 5'-Amino-2',5'-dideoxy-5-äthyluridin:
2',5'-Dideoxy-5-äthyl-5'-(2-phenylacetamido)uridin, Schmelzpunkt 218-219° C; und

m) aus 2,6-Dichlorophenylacetylchlorid und 5'-Amino-5'-deoxythymidin:
5'-[2-(2,6-Dichlorophenyl)acetamido]-5'-deoxythymidin, Schmelzpunkt 276° C.

Beispiel 4

A) Eine Suspension von 108 mg (2-Bromphenyl)essigsäure und 1 ml Oxalylchlorid in 2,5 ml Benzol wurde unter Zusatz von einem Tropfen Dimethylformamid gerührt. Das Gemisch wurde 1,5 Stunden bei Raumtemperatur gerührt und dann eingedampft. Der Rückstand wurde in 3 ml Diäthyläther aufgenommen und mit einer Lösung von 153 mg 5'-Amino-5-brom-2',5'-dideoxyuridin in 4,5 ml 0,11M Natriumhydroxidlösung versetzt. Das Gemisch wurde 10 Minuten kräftig geschüttelt und dann filtriert. Der Feststoff wurde aus Aethanol umkristallisiert und lieferte 75 ml 5-Brom-5'-[2-(2-bromphenyl)acetamido] -2',5'-dideoxyuridin als weissen Feststoff vom Schmelzpunkt 222-223° C.

B) In analoger Weise wurden erhalten:

a) aus 2(RS)-(2,4-Dichlorophenoxy)propionsäure und 5'-Amino-5-brom-2',5'-dideoxyuridin:
5-Brom-5'-[2(RS)-(2,4-dichlorophenoxy)propionamido]-2',5'-dideoxyuridin, Schmelzpunkt 171-172° C;

b) aus 2,6-Dichlorophenylessigsäure und 5'-Amino-5-brom-2',5'-dideoxyuridin:
5-Brom-5'-[2-(2,6-dichlorophenyl)acetamido]-2',5' -dideoxyuridin, Schmelzpunkt 232-233° C;

c) aus Benzolsäure und 5'-Amino-5-brom-2',5'-dideoxyuridin:
5'-Benzamido-5-brom-2',5'-dideoxyuridin, Schmelzpunkt 221-222° C;

d) aus 2(RS)-(2,4-Dichlorophenoxy)propionsäure und 5'-Amino-2',5'-dideoxy-5-ioduridin:
5'-[2(RS)-(2,4-Dichlorophenoxy)propionamido]-2',5'-dideoxy-5-ioduridin, Schmelzpunkt 205-207° C;

e) aus 2(RS)-(2,4,5-Trichlorophenoxy)propionsäure und 5'-Amino-2',5'-dideoxy-5-ioduridin:
5'-[2(RS)-(2,4,5-Trichlorophenoxy)propionamido]-2',5'-dideoxy-5-ioduridin, Schmelzpunkt 214-215° C; und

f) aus 2,6-Dichlorophenylessigsäure und 5'-Amino-2',5'-dideoxy-5-ioduridin:
5'-[2-(2,6-Dichlorophenyl)acetamido]-2',5'-dideoxy -5-ioduridin, Schmelzpunkt 225° C.

Beispiel 5

0,84 ml 1M Natriumhydroxidlösung wurden zu einer Lösung von 256 mg 5'-Amino-5-brom-2',5'-dideoxycytidin in 3,3 ml Wasser gegeben. Danach wurde eine Lösung von (2-Bromphenyl)-acetylchlorid (hergestellt aus 185 mg Säure) in 5 ml Dichlormethan zugesetzt, das Gemisch 10 Minuten kräftig geschüttelt und dann filtriert. Der Feststoff wurde aus 30 ml Aethanol unkristallisiert und lieferte 44 mg 5-Brom-5'-[2-(2-bromphenyl)acetamido]-2',5'-dideoxycytidin als weissen Feststoff vom Schmelzpunkt 164-167° C.

Das als Ausgangsmaterial verwendete 5'-Amino-5-brom-2',5'-dideoxycytidin wurde wie folgt hergestellt:

Eine Lösung von 3,06 g 5-Brom-2'-deoxycytidin und 2,3 g p-Toluolsulfonylchlorid in 50 ml Pyridin wurde 24 Stunden bei 4° C gerührt. Danach wurden weitere 1.91 g p-Toluolsulfonylchlorid zugesetzt und es wurde weitere 22 Stunden bei 4° C gerührt. Danach wurden 20 ml Methanol zugesetzt, das Gemisch 30 Minuten bei Raumtemperatur gerührt und dann eingedampft. Das Rohprodukt wurde durch Flash Chromatographie an einer Silicagelsäule mit Aethylacetat/Methanol (9:1) gereinigt, wobei 2,26 g 5-Brom-2'-deoxy-5'-O-p-toluolsulfonylcytidin als weisser Feststoff vom Schmelzpunkt 168° C (Zersetzung) erhalten wurden.

Eine Lösung von 2,19 g der vorstehend genannten Verbindung und 354 mg Lithiumazid in 26 ml Dimethylformamid wurde 2 Stunden bei 75° C gerührt. Das Lösungsmittel wurde abgedampft und der Rückstand mit Diäthyläther verrieben,, wobei 1,351 g 5'-Azido-5-brom-2',5'-dideoxycytidin als weisser Feststoff vom Schmelzpunkt 192-193° C (Zersetzung) erhalten wurden.

Eine Lösung von 1,30 g der vorstehend genannten Verbindung und 1,648 g Triphenylphosphin in 50 ml Pyridin wurden 100 Minuten bei Raumtemperatur gerührt. Danach wurden 5 ml Ammoniumhydroxidlösung zugesetzt, das Gemisch weitere 3 Stunden gerührt und dann eingedampft. Der Rückstand wurde 4 mal mit je 40 ml Toluol gewaschen und 4 mal mit je 40 ml Diäthyläther, danach 2 mal je 125 ml extrahiert. Die

vereinigten wässrigen Extrakte wurden eingedampft, der Rückstand in 25 ml Aethanol gelöst und die Lösung mit 300 ml Diäthyläther verdünnt, wobei sich ein weisser Feststoff abschied. Nach Stehen über Nacht bei 0 °C wurde der Feststoff abfiltriert und man erhielt 0,53 g 5'-Amino-5-brom-2',5'-dideoxycytidin als weisses Pulver, das sich oberhalb 115 °C zersetzte.

Beispiel 6

A) Ein Gemisch von 400 mg 5'-[2-(2,6-Dichlorphenyl)acetamido]-2',5'-dideoxy -5-äthyluridin, 0,15 ml Butyrylchlorid und 1 ml Dimethylaminopyridin in 50 ml Pyridin wurde über Nacht bei Raumtemperatur aufbewahrt und dann eingedampft. Der Rückstand wurde durch Flash-Chromatographie an einer Silicagelsäule mit Dichlormethan/Methanol (24:1) gereinigt,, wobei 175 mg 3'-O-Butyryl-5'-[2-(2,6-dichlorphenyl)acetamido] -2',5'-dideoxy-5-äthyluridin als weisser Feststoff vom Schmelzpunkt 130-132 °C erhalten wurden.

B) In analoger Weise wurden erhalten:

a) aus 5'-[2-(2,6-Dichlorphenyl)acetamido]-2',5'-dideoxy-5-äthyluridin und 3,3-Dimethylbutyrylchlorid: 2',5'-Dideoxy-5'-[2-(2,6-dichlorphenyl)acetamido] -3'-O-(3,3-dimethylbutyryl) -5-äthyluridin, Schmelzpunkt 77-79 °C;

b) aus 5'-[2-(2,6-Dichlorphenyl)acetamido]-2',5'-dideoxy-5-äthyluridin und Palmitoylchlorid: 5'-[2-(2,6-Dichlorphenyl)acetamido]-2',5'-dideoxy-5-äthyl-3'-O-palmitoyluridin, Schmelzpunkt 150 °C; und

c) aus 5'-[2-(2,6-Dichlorphenyl)acetamido]-2',5'-dideoxy-5-äthyluridin und Acetylchlorid: 3'-O-Acetyl-5'-[2-(2,6-dichlorphenyl)acetamido]-2',5'-dideoxy-5-äthyluridin, Schmelzpunkt 193-194 °C.

Beispiel 7

A) Eine Lösung von (2,6-Dichlorphenyl)acetylchlorid (hergestellt aus 103 mg der Säure) in 2 ml Diäthyläther wurde zu einer Lösung von 173 mg 5'-Amino-3'-O-benzyl-2',5'-dideoxy-5-äthyluridin in 4,5 ml 0,11M Natriumhydroxidlösung gegeben. Das Gemisch wurde 10 Minuten kräftig geschüttelt und dann filtriert. Der Feststoff wurde aus Aethanol umkristallisiert und lieferte 120 mg 3'-O-Benzyl-5'-[2-(2,6-dichlorphenyl)acetamido]-2',5'-dideoxy -5-äthyluridin als weissen Feststoff vom Schmelzpunkt 174-177 °C.

B) Das als Ausgangsmaterial verwendete 5'-Amino-3'-O-benzyl-2',5'-dideoxy -5-äthyluridin wurde wie folgt hergestellt:

Ein Gemisch von 6,5 g 2'-Deoxy-5-äthyl-5'-O-trityluridin, 19 g gepulvertes Kaliumhydroxid und 9,5 ml Benzylchlorid in einem Gemisch von 60 ml Benzol und 21 ml Dioxan wurde 4 Stunden unter Rühren zum Rückfluss erhitzt. Nach Abkühlen wurden 65 ml Wasser und 20 ml Essigsäure zugegeben und die Phasen wurden getrennt. Die organische Phase wurde zweimal mit je 90 ml Wasser gewaschen, über Natriumsulfat getrocknet und eingedampft und lieferte 4,5 g 3'-O-Benzyl-2'-deoxy-5-äthyl-5'-O-trityluridin als klebrigen Feststoff.

Eine Lösung des vorstehend genannten Produkts in einem Gemisch von 32 ml Essigsäure und 8 ml Wasser wurde 10 Minuten unter Rühren zum Rückfluss erhitzt. Das Gemisch wurde auf 0 °C gekühlt und filtriert und das Filtrat mit 100 ml Wasser verrieben. Der so erhaltene klebrige Feststoff wurde durch Flash-Chromatographie an einer Silicagelsäule mit Aethylacetat gereinigt. Das Produkt wurde aus einem Gemisch von Aceton und Petroläther (Siedepunkt 60-80 °C) kristallisiert. Man erhielt 1,25 g 3'-O-Benzyl-2'-deoxy -5-äthyluridin als farblos glasigen Feststoff.

Ein Gemisch von 1,2 g dieses Produkts, 929 mg Triphenylphosphin, 1,131 g Natriumazid und 1,179 g Kohlenstofftetrabromid in 14 ml Dimethylformamid wurde 20 Stunden bei Raumtemperatur gerührt. Danach wurden 8 ml Methanol zugesetzt, das Gemisch 30 Minuten gerührt und dann eingedampft. Der Rückstand wurde in 80 ml Wasser suspendiert und 3 mal mit je 60 ml Aethylacetat extrahiert. Die vereinigten Extrakte wurden mit Wasse gewaschen, mit Natriumsulfat getrocknet und eingedampft. Der Rückstand wurde durch Flash-Chromatographie an einer Silicagelsäule mit Aethylacetat/Hexan (2:1) gereinigt. Man erhielt 1,20 g 5'-Azido-3'-O-benzyl-2',5'-dideoxy-5-äthyluridin in Form eines farblosen Gummis.

Eine Lösung von 1.4 g des obigen Produkts und 1,582 g Triphenylphosphin in 48 ml Pyridin wurde 100 Minuten bei Raumtemperatur gerührt. Danach wurden 5 ml Ammoniumhydroxidlösung zugesetzt, das Gemisch weitere 3 Stunden bei Raumtemperatur gerührt und dann zur Trockene eingedampft. Der Rückstand wurde aus einem Gemisch von Aethylacetat und Hexan kristallisiert, wobei ein hellgrauer Feststoff erhalten wurde. Dieser Feststoff wurde in 30 ml Diäthyläther suspendiert, die Suspension 1,5

Stunden gerührt und filtriert, wobei 0,45 g 5'-Amino-3'-O-benzyl-2',5'-dideoxy -5-äthyluridin als weisser Feststoff vom Schmelzpunkt 95° C (Zersetzung) erhalten wurden.

Beispiel 8

A) Eine Lösung von (2,6-Dichlorphenyl)acetylchlorid (hergestellt aus 103 mg der Säure) in 2 ml Diäthyläther wurde zu einer Lösung von 140 mg 5'-Amino-2',5'-dideoxy-3'-O-äthyl-5-äthyluridin in 2,5 ml 0,2M Natriumhydroxidlösung gegeben. Das Gemisch wurde 10 Minuten kräftig geschüttelt und dann filtriert. Der Feststoff wurde mit 5 ml Wasser und mit 50 ml Diäthyläther gewaschen und aus Aceton umkristallisiert, wobei 60 mg 5'-[2-(2,6-Dichlorphenyl)-acetamido] -2',5'-dideoxy-3'-O-äthyl-5-äthyluridin als weisser Feststoff vom Schmelzpunkt 249-250° C erhalten wurden.

B) In analoger Weise wurde aus 2(RS)-(2,4-Dichlorphenoxy)propionylchlorid (hergestellt aus der Säure) und 5'-Amino-2',5'-dideoxy-3'-O-äthyl-5-äthyluridin, das 5'-[2(RS)-(2,4-Dichlorphenoxy)propionamido]-2',5'-dideoxy-3'-O-äthyl-5-äthyluridin vom Schmelzpunkt 121-124° C erhalten.

Das als Ausgangsmaterial verwendete 5'-Amino-2',5'-dideoxy-3'-O-äthyl -5-äthyluridin wurde wie folgt hergestellt:

Ein Gemisch von 6 g 2'-Deoxy-5-äthyl-5'-O-trityluridin, 1,347 g gepulvertem Kaliumhydroxid und 1,95 ml Aethyljodid in einem Gemisch von 60 ml Benzol und 20 ml Dioxan wurde 14 Stunden unter Rühren zum Rückfluss erhitzt. Die Lösungsmittel wurden abgedampft, der Rückstand in 6 ml Methanol aufgenommen und die Lösung in 250 ml Wasser gegossen. Das erhaltene Gemisch wurde 4 mal mit je 150 ml Chloroform extrahiert und die vereinigten Chloroformextrakte eingedampft, wobei 2'-Deoxy-3'-O-äthyl -5-äthyl-5'-O-trityluridin erhalten wurde, das direkt im nächsten Schritt eingesetzt wurde.

Eine Lösung dieses Produkts in einem Gemisch von 52 ml Essigsäure und 13 ml Wasser wurde 1 Stunde unter Rühren zum Rückfluss erhitzt und dann zur Trockene eingedampft. Der Rückstand wurde durch Flash-Chromatographie an einer Silicagelsäule mit Aethylacetat gereinigt, wobei 1,57 g 2'-Deoxy-3'-O-äthyl -5-äthyluridin als weisser Feststoff vom Schmelzpunkt 157° C erhalten wurden.

Ein Gemisch von 1,40 g 2'-Deoxy-3'-O-äthyl -5-äthyluridin, 1,3 g Triphenylphosphin, 1,59 g Natriumazid und 1,66 g Kohlenstofftetrabromid in 19 ml Dimethylformamid wurde 20 Stunden bei Raumtemperatur gerührt. Danach wurden 11 ml Methanol zugesetzt, das Gemisch 30 Minuten gerührt und dann eingedampft. Der Rückstand wurde in 110 ml Wasser suspendiert und 3 mal mit je 70 ml Aethylacetat extrahiert. Die vereinigten Aethylacetatextrakte wurden eingedampft und der Rückstand durch Flash-Chromatographie an einer Silicagelsäule mit Aethylacetat/Hexan (2:1) gereinigt. Man erhielt 1,26 g 5'-Azido-2',5'-dideoxy-3'-O-äthyl -5-äthyluridin als farbloses Oel.

Eine Lösung von 1,26 g des oben genannten Oels in 100 ml Methanol wurde 4,5 Stunden bei Raumtemperatur und Atmosphärendruck über 10% Palladium/Kohle hydriert. Der Katalysator wurde abfiltriert und das Filtrat eingedampft. Der Rückstand wurde aus einem Gemisch von Aethylacetat und Hexan umkristallisiert und lieferte 780 mg 5'-Amino-2',5'-dideoxy-3'-O-äthyl -5-äthyluridin als weissen Feststoff vom Schmelzpunkt 122-123° C.

Beispiel 9

Eine Lösung von 210 mg (2-Bromphenyl)acetylchlorid in 3 ml Diäthyläther wurde zu einer Lösung von 324 mg 5'-Amino-4-N-benzoyl-2',5'-dideoxy -5-äthylcytidin in 4,9 ml 0,18M Natriumhydroxidlösung gegeben. Das Gemisch wurde 10 Minuten kräftig geschüttelt, der Feststoff abfiltriert, mit 2 ml Wasser und 1 ml Diäthyläther gewaschen und aus 15 ml Aethanol umkristallisiert. Man erhielt 100 mg 4-N-Benzoyl-5'-[2-(2-bromphenyl)acetamido] -2',5'-dideoxy-5-äthylcytidin als weissen Feststoff, der in dieser Form weiter umgesetzt wurde.

Eine Lösung von 100 mg dieses Feststoffs in 20 ml methanolischer Ammoniaklösung wurde über Nacht bei Raumtemperatur aufbewahrt. Danach wurde die Lösung zur Trockene eingedampft und der Rückstand mit 20 ml Diäthyläther verrieben, wobei 52 mg 5'-[2-(2-Bromphenyl)acetamido]-2',5'-dideoxy -5-äthylcytidin als weisser Feststoff vom Schmelzpunkt 236-237° C erhalten wurden.

Das als Ausgangsmaterial verwendete 5'-Amino-4-N-Benzoyl-2',5'-dideoxy -5-äthylcytidin wurde wie folgt hergestellt:

Eine Lösung von 4,08 g 2'-Deoxy-5-äthylcytidin in 200 ml Aethanol wurde unter Rühren zum Rückfluss erhitzt. Danach wurden 3,94 g Benzoesäureanhydrid und nach 1, 2 und 4 Stunden jeweils weitere 3,94 g Benzoesäureanhydrid zugesetzt. Nach dem letzten Zusatz wurde das Gemisch eine weitere Stunde zum Rückfluss erhitzt und dann zur Trockene eingedampft. Der Rückstand wurde in 250 ml Diäthyläther suspendiert und über Nacht bei Raumtemperatur aufbewahrt. Der Feststoff wurde abfiltriert und mit 75 ml

Diäthyläther gewaschen. Man erhielt 4,5 g 4-N-Benzoyl-2'-deoxy -5-äthylcytidin als weissen Feststoff vom Schmelzpunkt 172-174° C.

Ein Gemisch von 1,436 g N-Benzoyl-2'-deoxy-5-äthylcytidin, 1,072 g Triphenylphosphin, 1,304 g Natriumazid und 1,360 g Kohlenstofftetrabromid in 16 ml Dimethylformamid wurden 20 Stunden bei Raumtemperatur gerührt. Danach wurden 8 ml Methanol zugesetzt, das Gemisch 30 Minuten gerührt und dann eingedampft. Der Rückstand wurde in 80 ml Wasser suspendiert und 3 mal mit je 100 ml Aethylacetat extrahiert. Die vereinigten Aethylacetatextrakte wurden mit Wasser gewaschen und eingedampft. Der Rückstand wurde durch Flash-Chromatographie an einer Silicagelsäule mit Aethylacetat gereinigt, wobei 1 g 5'-Azido-4-N-benzoyl-2',5'-dideoxy-5-äthylcytidin als weisser kristalliner Feststoff vom Schmelzpunkt 125-126° C erhalten wurden.

Eine Lösung von 500 mg des obigen Produkts in 30 ml Methanol wurde bei Raumtemperatur und unter Atmosphärendruck 4 Stunden über 10% Palladium/Kohle hydriert. Der Katalysator wurde abfiltriert und das Filtrat eingedampft, wobei 5'-Amino-4-N-benzoyl-2',5'-dideoxy -5-äthylcytidin als farbloser Gummi erhalten wurde.

Beispiel 10

Eine Lösung von (2,6-Dichlorphenyl)acetylchlorid (hergestellt aus 146 mg der Säure) in 1 ml Diäthyläther wurde zu einer Lösung von 170 mg 5'-Amino-2',3',5'-trideoxy-5-äthyluridin in 2,72 ml 0,26M Natriumhydroxidlösung gegeben. Das Gemisch wurde 10 Minuten geschüttelt, filtriert und der Feststoff aus 20 ml Aethanol umkristallisiert. Man erhielt 150 mg 2',3',5'-Trideoxy-5'-[2-(2,6-dichlorphenyl)acetamido]-5-äthyluridin in Form eines weissen Feststoffes vom Schmelzpunkt 237-238° C.

Das als Ausgangsmaterial verwendete 5'-Amino-2',3',5'-trideoxy-5-äthyluridin wurde wie folgt hergestellt:

Eine Lösung von 16,0 g 2'-Deoxy-5-äthyl-5'-O-trityluridin und 6,8 ml Methansulfonylchlorid in 140 ml Pyridin wurde über Nacht bei 0° C aufbewahrt. Nach Zusatz von 3 g Eis wurde das Gemisch eine weitere Stunde bei 0° C gehalten und dann in 1500 ml Eiswasser gegossen. Der erhaltene Feststoff wurde abfiltriert, mit 500 ml Wasser gewaschen und getrocknet, wobei 17,35 g 2'-Deoxy-5-äthyl-3'-O-met hansulfonyl-5'-O-trityluridin als weisser Feststoff erhalten wurden, der ohne weitere Reinigung im nächsten Schritt eingesetzt wurde.

Ein Gemisch von 1,2 g des obigen Produkts und 681 mg Natriumjodid in 10 ml Aethylmethylketon wurde 7 Stunden unter Rühren zum Rückfluss erhitzt. Das Gemisch wurde abkühlen gelassen und dann filtriert. Das Filtrat wurde zu einem farblosen Gummi eingedampft der durch Flash-Chromatographie an einer Silicagelsäule mit Aethylacetat/Hexan (2:1) gereinigt wurde. Das Produkt wurde aus einem Gemisch von Aethylacetat und Hexan kristallisiert und lieferte 360 mg 2',3'-Dideoxy-5-äthyl-3'-jod-5'-O-trityluridin als weisse Kristalle vom Schmelzpunkt 93-98° C.

Eine Lösung von 720 mg 2',3'-Dideoxy-5-äthyl-3'-jod-5'-O-trityluridin in einem Gemisch von 16 ml Essigsäure und 4 ml Wasser wurde eine Stunde unter Rühren zum Rückfluss erhitzt. Das Lösungsmittel wurde abgedampft und der Rückstand aus einem Gemisch von Aethylacetat und Hexan kristallisiert, wobei 180 mg 2',3'-Dideoxy-5-äthyl-3'-joduridin in Form eines weissen Feststoffes vom Schmelzpunkt 161,5-163° C erhalten wurden.

Eine Lösung von 2,50 g 2',3'-Dideoxy-5-äthyl-3'-joduridin in einem Gemisch von 90 ml äthanolischem Ammoniak und 17 ml Wasser wurde bei Raumtemperatur und unter Atmosphärendruck 3 Stunden über 5% Palladium/Bariumsulfat hydriert. Das Gemisch wurde filtriert und das Filtrat eingedampft. Der Rückstand wurde 2 mal mit je 250 ml heissem Aethylacetat extrahiert und die vereinigten Extrakte wurden eingedampft. Der Rückstand wurde an einer kurzen Silicagelsäule mit Aethylacetat chromatographiert und das Produkt aus einem Gemisch von Aethylacetat und Hexan kristallisiert. Man erhielt 1,17 g 2',3'-Dideoxy-5-äthyluridin als weissen Feststoff vom Schmelzpunkt 109-113° C.

Ein Gemisch von 350 mg 2',3'-Dideoxy-5-äthyluridin, 391 mg Triphenylphosphin, 475 mg Natriumazid und 496 mg Kohlenstofftetrabromid wurde 20 Stunden bei Raumtemperatur gerührt. Danach wurden 3 ml Methanol zugesetzt, das Gemisch eine weitere Stunde gerührt und dann eingedampft. Der Rückstand wurde in 30 ml Wasser suspendiert und 2 mal mit je 50 ml Aethylacetat extrahiert. Die vereinigten Aethylacetatextrakte wurden mit 20 ml Wasser gewaschen und eingedampft. Der Rückstand wurde durch Flash-Chromatographie an einer Silicagelsäule mit Aethylacetat gereinigt. Das Produkt wurde aus einem Gemisch von Aethylacetat und Hexan kristallisiert und lieferte 200 mg 5'-Azido-2',3',5'-trideoxy-5-äthyluridin als weissen Feststoff, der ohne weitere Reinigung weiter umgesetzt wurde.

Eine Lösung von 190 mg des obigen Produkts in 25 ml Methanol wurde bei Raumtemperatur und Atmosphärendruck 4 Stunden über 100 mg 10% Palladium/Kohle hydriert. Der Katalysator wurde abfiltriert und das Filtrat eingedampft, wobei 180 mg 5'-Amino-2',3',5'-trideoxy-5-äthyluridin als farbloser Gummi

erhalten wurden.

Beispiel 11

Eine Lösung von 0,2 g 2',5'-Dideoxy-5-äthyl-5'-[2-(2-nitrophenyl)acetamido]uridin in 100 ml Aethanol wurde über 0,1 g 10% Palladium/Kohle bei Raumtemperatur und unter Atmosphärendruck hydriert, bis kein Wasserstoff mehr aufgenommen wurde. Das Gemisch wurde abfiltriert und das Filtrat zu einem Oel eingedampft, das aus Aethanol kristallisiert 0,1 g 5'-[2-(2-Aminophenyl)acetamido]-2',5'-dideoxy-5-äthyluridin als weissen Feststoff vom Schmelzpunkt 195-196 °C lieferte.

Beispiel 12

Eine Lösung von 0,2 g 5'-[2-(2-Aminophenyl)acetamido]-2',5'-dideoxy -5-äthyluridin in 5 ml trockenem Pyridin wurde mit 0,5 g Acetanhydrid 17 Stunden bei Raumtemperatur stehen gelassen. Das Gemisch wurde dann zu einem Oel eingedampft, das, aus Aethanol kristallisiert, 0,13 g 5'-[2-(2-Acetamidophenyl)-acetamido]-3'-O-acetyl -2',5'-dideoxy-5-äthyluridin als Feststoff vom Schmelzpunkt 143-145 °C lieferte.

Beispiel 13

In eine Lösung von 0,2 g 5'-[2-(2-Acetamidophenyl)acetamido] -3'-O-acetyl-2',5'-dideoxy-5-äthyluridin in 20 ml Methanol wurde mit 20 ml Methanol, das mit Ammoniak gesättigt war behandelt. Das Gemisch wurde 17 Stunden bei Raumtemperatur stehen gelassen. Der ausgeschiedene weisse Feststoff wurde abfiltriert, 2 mal mit je 5 ml Aethanol und 5 ml Diäthyläther gewaschen und anschliessend getrocknet. Man erhielt 5'-[2-(2-Acetamidophenyl)acetamido]-2',5'-dideoxy-5-äthyluridin vom Schmelzpunkt 266-267 °C.

Beispiel 14

In analoger Weise wie in Beispiel 1 beschrieben wurden erhalten:

a) aus 5'-Amino-5'-deoxythymidin und (2-Bromphenyl)-acetylchlorid:
5'-[2-(2-Bromphenyl)acetamido]-5'-deoxythymidin, Schmelzpunkt 232-233 °C;
b) aus 5'-Amino-5'-deoxythymidin und (4-nitrophenyl)-acetylchlorid:
5'-Deoxy-5'-[2-(4-nitrophenyl)acetamido]thymidin, Schmelzpunkt 240-241 °C;
c) aus 5'-Amino-5'-deoxythymidin und Phenylacetylchlorid:
5'-Deoxy-5'-(2-phenylacetamido)thymidin, Schmelzpunkt 221-222 °C; und
d) aus 5'-Amino-5'-deoxythymidin und (4-trifluormethylphenyl)acetylchlorid:
5'-Deoxy-5'-[2-(4-trifluormethylphenyl) acetamido]-thymidin, Schmelzpunkt 252-253 °C.

Beispiel 15

0,2 g (4-Hydroxy-2,6-dimethylphenyl)essigsäure in 10 ml Benzol wurde mit 0,15 g Oxalylchlorid und einem Tropfen Dimethylformamid versetzt und 2 Stunden gerührt. Das Lösungsmittel wurde abgedampft, der Rückstand auf -20 °C gekühlt und in 6 ml trockenem Pyridin gelöst. Danach wurden 0,26 g 5'-Amino-2',5'-dideoxy-5-äthyluridin zugesetzt und das Gemisch 4 Stunden bei 0 °C gerührt und bei 4 °C über Nacht stehen gelassen. Das Lösungsmittel wurde abgedampft und der Rückstand mit Toluol und Wasser erneut abgedampft. Der Rückstand wurde mit Wasser bei 0 °C verrieben und der erhaltene Feststoff abfiltriert und unter vermindertem Druck getrocknet. Man erhielt 0,175 g Rohprodukt vom Schmelzpunkt 232-238 °C (Zersetzung). Umkristallisation aus 6 ml Methanol lieferte 0,06 g reines 2',5'-Dideoxy-5-äthyl-5'-[2-(4--hydroxy -2,6-dimethylphenyl)acetamido]uridin vom Schmelzpunkt 240-243 °C (Zersetzung).

Beispiel 16

0,32 g 5'-Amino-5-(2-chloräthyl)-2',5'-dideoxyuridinhydrochlorid wurden in einem Gemisch von 5 ml Wasser und 2,5 ml 1M Natriumhydroxidlösung gelöst. Das Gemisch wurde mit einer benzolischen Lösung von (2,6-Dichlorphenyl)acetylchlorid (hergestellt aus 0,22 g der Säure durch Behandlung mit Oxalylchlorid) versetzt und 25 Minuten kräftig geschüttelt. Der erhaltene Fesststoff wurde abfiltriert und mit Wasser und Diäthyläther gewaschen, wobei 0,29 g Rohprodukt vom Schmelzpunkt 248-249 °C (Zersetzung) erhalten wurden. 0.1 g dieses Feststoffes wurden nacheinander jeweils 1 Stunde mit 1 ml Wasser, 2 ml Aethanol und 2 ml Methanol gerührt, wobei 0,03 g 5-(2-Chloräthyl)-5'-[2-(2,6-dichlorphenyl)acetamido] -2',5'-dideoxyu-

ridin vom Schmelzpunkt 253-254° C (Zersetzung) erhalten wurden.

Das als Ausgangsmaterial verwendete 5'-Amino-5-(2-chloräthyl)-2',5'-dideoxyuridinhydrochlorid wurde wie folgt hergestellt:

1,4 g 5-(2-Chloräthyl)-2'-deoxyuridin und 1,3 g Triphenylphosphin wurden in 20 ml Dimethylformamid gelöst und unter Rühren mit 1,2 g Lithiumazid versetzt,, wobei im Verlauf von 5 Minuten bei Raumtemperatur Lösung eintrat. Danach wurden 1,7 g Kohlenstofftetrabromid im Verlauf von 5 Minuten zugesetzt, wobei eine trüborange Lösung erhalten wurde, die bei Raumtemperatur 17 Stunden gerührt wurde. Danach wurden 5 ml Methanol zugesetzt, wobei die Lösung klar wurde. Nach 0,5 Stunden wurden die Lösungsmittel unter Oelpumpenvakuum abgedampft und der erhaltene Gummi zwischen 30 ml Aethylacetat und 20 ml Wasser verteilt. Der gebildete weisse Feststoff wurde abfiltriert und man erhielt 0,64 g Rohprodukt vom Schmelzpunkt 179-182° C (Zersetzung). Dieses Rohprodukt wurde mit Methanol gerührt und abfiltriert, wobei 0,48 g reines 5'-Azido-2',5'-dideoxy-5-(2-chloräthyl)-uridin vom Schmelzpunkt 197-199° C (Zersetzung) erhalten wurden.

0,16 g 5'-Azido-2',5'-dideoxy-5-(2-chloräthyl)-uridin wurden in 100 ml Methanol gelöst und mit einer Lösung von 1,9 ml 0,27M HCl in Methanol versetzt. Unter Stickstoffatmosphäre wurde eine Aufschlämmung von 25 mg 5% Palladium/Kohle in 10 ml Aethanol zugesetzt und das Gemisch wurde bei Raumtemperatur und Atmosphärendruck 2 Stunden hydriert. Der Katalysator wurde abfiltriert und das Filtrat zu einem Feststoff eingeengt, der aus einem Gemisch von 15 ml Methanol und 30 ml Diäthyläther umkristallisiert, 0,13 g 5'-Amino-5-(2-chloräthyl)-2',5'-dideoxyuridinhydrochlorid vom Schmelzpunkt 229-230° C (Zersetzung) lieferte.

Beispiel 17

0,72 g 2',5'-Dideoxy-5-äthyl-5'-methylaminouridin in 25 ml trockenem Pyridin wurden unter Rühren bei 0° C mit einer Lösung von (2-Bromphenyl)acetylchlorid in 7 ml Benzol (hergestellt aus 0,65 g der Säure durch Behandlung mit Thionylchlorid in Benzol unter Rückfluss) versetzt. Das Gemisch wurde eine halbe Stunde bei 0° C gerührt und dann über Nacht bei 4° C aufbewahrt. Die Lösungsmittel wurden abgedampft und der Rückstand mit Toluol abgedampft, wobei ein Gummi erhalten wurde, der auf Verreiben mit Diäthyläther einen Feststoff lieferte. Dieser Feststoff wurde in 5 ml Methylenchlorid/Methanol (9:1) aufgenommen und an einer Silicagelsäule mit Methylenchlorid/Methanol (9:1) chromatographiert. Die das Produkt enthaltenden Fraktionen wurden vereinigt und eingedampft. Der Rückstand wurde mit Aethanol abgedampft und aus Methanol umkristallisiert und lieferte 0,18 g 5'-[2-(2-Bromphenyl)-N-methylacetamido] -2',5'-dideoxy-5-äthyluridin vom Schmelzpunkt 182-185° C.

In analoger Weise wurde aus (2(RS)-(2,4-Dichlorphenoxy)propionylchlorid und 2',5'-Dideoxy-5-äthyl-5'-methylaminouridin das 5'-[2(RS)-(2,4-Dichlorphenoxy) -N-methylpropionamido]-2',5'-dideoxy-5-äthyluridin vom Schmelzpunkt 167-177° C (Zersetzung) erhalten.

Das als Ausgangsmaterial verwendete 2',5'-Dideoxy-5-äthyl-5'-methylaminouridin wurde wie folgt hergestellt:

26 g 2'-Deoxy-5-äthyluridin wurden in 400 ml trockenem Pyridin gelöst. Die Lösung wurde auf 0° C gekühlt und unter Rühren nach und nach mit 20 g Paratoluolsulfonylchlorid versetzt. Das Gemisch wurde dann noch eine Stunde bei 0° C gerührt und bei 4° C über Nacht stehen gelassen. Das Lösungsmitel wurde abgedampft und der Rückstand mit Toluol abgedampft. Der Rückstand wurde mit 200 ml Methanol geschüttelt und im Kühlschrank 2 1/2 Stunden stehen gelassen. Der erhaltene Feststoff wurde abfiltriert, mit Methanol gewaschen und unter vermindertem Druck getrocknet, wobei 18 g Rohprodukt vom Schmelzpunkt 183° C (Zersetzung) erhalten wurden. Umkristallisation aus 450 ml Aethanol lieferte 13 g reines 2'-Deoxy-5-äthyl-5'-O-(p-toluolsulfonyl)uridin vom Schmelzpunkt 189-190° C (Zersetzung).

1,8 g des vorstehenden Produkts wurden in 10 ml trockenem Dimethylformamid gelöst und die Lösung mit 1,2 ml N-Benzylmethylamin versetzt. Das Gemisch wurde unter Stickstoff gerührt und 5 Stunden auf 80° C erwärmt. Das Lösungsmittel wurde abgedampft und der Rückstand mit Toluol abgedampft. Der Rückstand wurde mit Diäthyläther verrieben und abfiltriert und lieferte 2,6 g Feststoff vom Schmelzpunkt 110-115° C. Dieser Feststoff wurde in 10 ml Methylenchlorid/Methanol (9:1) aufgenommen und an einer Silicagelsäule mit dem gleichen Lösungsmittel chromatographiert. Die das Produkt enthaltenden Fraktionen wurden vereinigt und eingedampft. Der Rückstand wurde mit Diäthyläther verrieben und lieferte 1,2 g 2',5'-Dideoxy-5-äthyl-5'-(N-methyl-N-benzylamino)uridin vom Schmelzpunkt 135-137° C.

1,1 g 2',5'-Dideoxy-5-äthyl-5'-(N-methyl-N-benzylamino)uridin wurden in 75 ml Aethanol aufgenommen und unter Stickstoff mit 1 g 5% Palladium/Kohle in 25 ml Aethanol versetzt. Das Gemisch wurde bei Raumtemperatur und Atmosphärendruck hydriert. Der Katalysator wurde abfiltriert und das Filtrat eingedampft. Man erhielt 0,84 g 2',5'-Dideoxy-5-äthyl-5'-methylaminouridin vom Schmelzpunkt 145-147° C.

Beispiel 18

In analoger Weise wie in Beispiel 17 beschrieben wurden erhalten:

a) aus (2,6-Dichlorphenyl)acetylchlorid (hergestellt aus 0,29 g der Säure durch Behandlung mit Oxalylchlorid) und 0,35 g 2',5'-Dideoxy-5-äthyl-5'-methylaminouridin nach Chromatographie und Umkristallisation aus einem Gemisch von 8 ml Aethanol und 24 ml Diäthyläther, 0,19 g 5'-[2-(2,6-Dichlorphenyl)-N-methylacetamido] -2',5'-dideoxy-5-äthyluridin vom Schmelzpunkt 209-210 °C.

b) aus 2(RS)-(2-Bromphenyl)propionylchlorid (hergestellt aus 0,32 g der Säure durch Behandlung mit Oxalylchlorid) und 0,35 g 2',5'-Dideoxy-5-äthyl-5'-methylaminouridin nach Chromatographie an Silicagel mit Methylenchlorid/Methanol (9:1), 0,19 g 5'-[2(RS)-(2-Bromphenyl)-N-methylpropionamido]-2',5'-dideoxy -5-äthyluridin vom Schmelzpunkt 80-90 °C (Zersetzung).

Beispiel 19

0,05 g 5'-[2-(2-Bromphenyl)-N-methylacetamido]-2',5'-dideoxy -5-äthyluridin wurden in 2 ml trockenem Pyridin gelöst und die Lösung mit 0,12 ml Acetanhydrid versetzt. Das Gemisch wurde 5 Stunden bei Raumtemperatur gerührt und dann eingedampft. Der Rückstand wurde mit Toluol abgedampft und lieferte einen Feststoff, der mit Diäthyläther verrieben und abfiltriert wurde. Man erhielt 0,03 g 3'-O-Acetyl-5'-[2-(2-bromphenyl)-N-methylacetamido] -2',5'-dideoxy-5-äthyluridin vom Schmelzpunkt von etwa 170 °C.

Beispiel 20

A) 1,2 ml einer 1M Natriumhydroxidlösung wurden zu 0,255 g 5'-Amino-2',5'-dideoxy-5-äthyluridin in 5 ml Wasser gegeben und die Lösung dann mit 0,21 g Naphthaloylchlorid versetzt. Das Gemisch wurde 10 Minuten kräftig geschüttelt, wobei sich ein weisser Feststoff abschied. Dieser Feststoff wurde abfiltriert, 3 mal mit je 5 ml Wasser gewaschen und dann unter vermindertem Druck getrocknet. Umkristallisation aus Aethanol lieferte 0,2 g 2',5'-Dideoxy-5-äthyl-5'-(2-naphthalamido)uridin als weissen Feststoff vom Schmelzpunkt 242-244 °C.

B) In analoger Weise wurden erhalten:

a) aus 5'-Amino-2',5'-dideoxy-5-äthyluridin und 2-Phenylbenzoylchlorid:
2',5'-Dideoxy-5-äthyl-5'-(2-phenylbenzamido)uridin, Schmelzpunkt 246-247 °C;

b) aus 5'-Amino-2',5'-dideoxy-5-äthyluridin und 3-Phenylpropionylchlorid:
2',5'-Dideoxy-5-äthyl-5'-(3-phenylpropionamido)uridin, Schmelzpunkt 225-226 °C;

c) aus 5'-Amino-2',5'-dideoxy-5-äthyluridin und 4-Phenylbutyrylchlorid:
2',5'-Dideoxy-5-äthyl-5'-(4-phenylbutyramido)uridin, Schmelzpunkt 215 °C;

d) aus 5'-Amino-2',5'-dideoxy-5-äthyluridin und Cinnamoylchlorid:
2',5'-Dideoxy-5'-cinnamamido-5-äthyluridin, Schmelzpunkt 252-254 °C;

e) aus 5'-Amino-2',5'-dideoxy-5-äthyluridin und Phenylpropiolylchlorid:
2',5'-Dideoxy-5--äthyl-5'-(3-phenyl -2-propynamido)uridin, Schmelzpunkt 234-236 °C;

f) aus 5'-Amino-2',5'-dideoxy-5-äthyluridin und 3-(Phenylsulphonyl)propionylchlorid:
2',5'-Dideoxy-5-äthyl-5' -[3(phenylsulphonyl)propionamido]uridin, Schmelzpunkt 190-192 °C; und

g) aus 5'-Amino-2',5'-dideoxy-5-äthyluridin und 2-Bromcinnamoylchlorid:
5'-(2-Bromcinnamamido)-2',5'-dideoxy-5-äthyluridin, Schmelzpunkt 202-204 °C.

Beispiel 21

A) 5 g Oxalylchlorid um zu einem Gemisch von 5 g 2-Thiophenessigsäure und einem Tropfen Dimethylformamid in 40 ml trockenem Benzol gegeben. Nach 2 Stunden Rühren wurde das Benzol abgedampft und das rohe 2-Thiophenacetylchlorid durch Destillation gereinigt.

0,18 g 2-Thiophenacetylchlorid wurde zu einer Lösung von 0,255 g 5'-Amino-2',5'-dideoxy-5-äthyluridin in 5 ml Wasser, die 1,2 ml 1M Natriumhydroxid enthielt gegeben und das erhaltene Gemisch wurde 10 Minuten kräftig geschüttelt. Der erhaltene weisse Feststoff wurde abfiltriert, mit Wasser gewaschen, bei 50 °C über Phosphorpentoxid unter vermindertem Druck getrocknet und aus Aethanol umkristallisiert. Man erhielt 0,2 g 2',5'-Dideoxy-5-äthyl-5'-[-2-(2-thienyl)acetamido]uridin vom Schmelzpunkt 215-217 °C.

B) In analoger Weise wurden erhalten:

a) aus 5'-Amino-2',5'-dideoxy-5-äthyluridin und 3-Thiophenessigsäure:
2',5'-Dideoxy-5-äthyl-5'-[2-(3-thienyl)acetamido]uridin, Schmelzpunkt 220-222 °C; und

b) aus 5'-Amino-2',5'-dideoxy-5-äthyluridin und 1-Adamantancarbonylchlorid: 5'-(1-Adamantylcarboxamido)-2',5'-dideoxy-5-äthyluridin, Schmelzpunkt 160-162° C.

Beispiel 22

A) Eine Lösung von 0,255 g 5'-Amino-2',5'-dideoxy-5-äthyluridin in einem Gemisch von 5 ml Wasser und 1,1 ml 1M Natriumhydroxidlösung wurden mit 0,14 g Picolinoylchlorid versetzt. Das Reaktionsgemisch wurde 15 Minuten kräftig geschüttelt und die erhaltene dunkelbraune Lösung 2 mal mit je 10 ml n-Butanol extrahiert. Die Extrakte wurden mit gesättigter Natriumchloridlösung gewaschen, über Natriumsulfat getrocknet und eingedampft. Der Feststoff wurde durch Flash-Chromatographie an einer Silicagelsäule mit Diäthylacetat gereinigt. Die das Produkt enthaltenden Fraktionen wurden vereinigt und eingedampft und der erhaltene hellbraune Feststoff aus Aethanol/Diäthyläther umkristallisiert. Man erhielt 0,12 g 2',5'-Dideoxy-5-äthyl-5'-(2-pyridylcarboxamido)uridin als weissen Feststoff vom Schmelzpunkt 196-198° C.

B) In analoger Weise wurde aus 5'-Amino-2',5'-dideoxy-5-äthyluridin und Nicotinoylchlorid das 2',5'-Dideoxy-5-äthyl-5'-(3-pyridylcarboxamido)uridin vom Schmelzpunkt 223-225° C erhalten.

Beispiel 23

Ein Gemisch von 0,26 g 5'-Amino-2',5'-dideoxy-5-äthyluridin, 0,2 g Phenylsulfonylessigsäure und 1,2 g Dicyclohexylcarbodiimid in 10 ml trockenem Pyridin wurde bei Raumtemperatur über Nacht gerührt, das Lösungsmittel abgedampft und der Rückstand 3 mal mit Toluol und 2 mal mit Diäthyläther abgedampft. Der Rückstand wurde mit Diäthyläther verrieben und bei Raumtemperatur stehen gelassen. Der erhaltene Feststoff wurde abfiltriert, mit Diäthyläther gewaschen und unter vermindertem Druck getrocknet. Nach Umkristallisation des Produkts (0,66 g) aus 15 ml Aethanol wurden 0,40 g Produkt vom Schmelzpunkt 213-217° C erhalten. Weitere Umkristallisationen von 0,2 g des Produktes aus 15 ml Aethanol lieferten 0,09 g 2',5'-Dideoxy-5-äthyl-5'-[2-(phenylsulfonyl)acetamido]uridin als weissen Feststoff vom Schmelzpunkt 218-219° C.

Beispiel 24

0,23 g 9-Fluorencarbonsäure wurde in 10 ml Benzol bei 25° C mit 0,15 g Oxalylchlorid und einem Tropfen Dimethylformamid versetzt. Das Gemisch wurde 1,5 Stunden stehen gelassen und dann eingedampft. Der Rückstand wurde in 1,5 ml Benzol gelöst und zu einer Lösung von 0,26 g 5'-Amino-2',5'-dideoxy-5-äthyluridin in 5 ml Wasser und 1,5 ml 1M Natriumhydroxid gegeben. Das Gemisch wurde 15 Minuten kräftig geschüttelt und dann teilweise eingedampft. Der erhaltene Feststoff wurde abfiltriert und nacheinander mit Wasser, Aethanol und Diäthyläther gewaschen, wobei 0,22 g 2',5'-Dideoxy-5-äthyl-5'-(9-fluorenylcarboxamido)uridin vom Schmelzpunkt 275-278° C (Zersetzung) erhalten wurden.

Beispiel 25

Eine Aufschlämmung von 0,45 g 2',5'-Dideoxy-5-äthyl-5'-(9-fluorenylcarboxamido)uridin in 9 ml trockenem Pyridin wurde unter Rühren mit 0,6 ml Acetanhydrid versetzt. Das Gemisch wurde 4 Stunden bei Raumtemperatur gerührt und dann über Nacht stehen gelassen. Das Lösungsmittel wurde abgedampft und der Rückstand mit Toluol abgedampft. Der Rückstand wurde mit Diäthyläther verrieben und lieferte 0,4 g Rohprodukt vom Schmelzpunkt 240-255° C (Zersetzung). Umkristallisation des Rohprodukts aus einem Gemisch von 10 ml Chloroform und 50 ml Diäthyläther lieferte 0,21 g reines 3'-O-Acetyl-2',5'-dideoxy-5-äthyl -5'-(9-fluorenylcarboxamido)uridin vom Schmelzpunkt 263-265° C (Zersetzung).

Beispiel 26

In analoger Weise wie in Beispiel 25 wurden erhalten:

A) aus 2,75 g 2',5'-Dideoxy-5-äthyl-5'-(9-fluorenylcarboxamido)uridin und 1 ml Butyrylchlorid nach Chromatographie an Silicagel mit Methylenchlorid/Methanol (19:1) 0,98 g 3'-O-Butyryl-2',5'-dideoxy-5-äthyl -5'-(9-fluorenylcarboxamido)uridin vom Schmelzpunkt 208-210° C (Zersetzung).

B) aus 0,45 g 2',5'-Dideoxy-5-äthyl-(9-fluorenylcarboxamido)uridin und 0,22 g tert.Butylacetylchlorid nach Chromatographie an Silicagel mit Methylenchlorid und Verreiben mit Petroläther (Siedepunkt 40-60° C) 0,14 g 2',5'-Dideoxy-5-äthyl -3'-O-(3,3-dimethylbutyryl)-5'-(9-fluorenylcarboxamido)uridin vom Schmelzpunkt 100-110° C (Zersetzung).

C) aus 0,45 g 2',5'-Dideoxy-5-äthyl-5'-(9-fluorenylcarboxamido)uridin und 0,3 g Palmitoylchlorid nach Umkristallisation aus einem Gemisch von 12 ml Aethylacetat und 48 ml Petroläther (Siedepunkt 40-60° C), 0,14 g 2',5'-Dideoxy-5-äthyl -5'-(9-fluorenylcarboxamido)-3'-O-(hexadecanoyl)uridin vom Schmelzpunkt 154-157° C (Zersetzung).

Beispiel 27

Eine Aufschlämmung von 5,5 g 9-Fluorencarbonsäure in 100 ml Benzol wurde unter Rühren bei 25° C mit 3,6 g Oxalylchlorid und dann mit 0,1 ml Dimethylformamid versetzt. Das Gemisch wurde 1,5 Stunden gerührt und dann abgedampft, wobei das Säurechlorid in Form eines Gummis erhalten wurde. Dieser Gummi wurde auf -15° C gekühlt und mit 90 ml kaltem (-10° C) Pyridin, 3,3 g 5'-Amino-2',5'-dideoxy -5-äthyluridin versetzt und das Gemisch 2,5 Stunden bei einer Temperatur unterhalb 0° C gerührt. Die hellgelbe Lösung wurde bei 4° C über Nacht stehen gelassen. Das Lösungsmittel wurde abgedampft und der Rückstand mit Toluol abgedampft. Der Rückstand wurd mit 70 ml Wasser und danach mit 70 ml Aethanol verrieben, wobei 3,4 g fast reines Produkt vom Schmelzpunkt 240-245° C (Zersetzung) erhalten wurden. Umkristallisation aus einem Gemisch von 110 ml Methylenchlorid und 110 ml Diäthyläther lieferte 2,3 g reines 2',5'-Dideoxy-5-äthyl-3'-O-(9-fluorenylcarbonyl) -5'-(9-fluorenylcarboxamido)uridin vom Schmelzpunkt 240-245° C (Zersetzung).

Beispiel 28

A) 1,25 ml 1M Natriumhydroxidlösung wurden zu 0,255 g 5'-Amino-2',5'-dideoxy-5-äthyluridin in 5 ml Wasser gegeben. Danach wurden 0,2 g 2-Phenylbutyrylchlorid zugesetzt und das erhaltene Gemisch 10 Minuten geschüttelt, wobei sich ein weisser Feststoff abschied. Dieser Feststoff wurde abfiltriert, 3 mal mit je 5 ml Wasser gewaschen, bei 50° C unter vermindertem Druck über Phosphorpentoxid getrocknet und aus Aethanol umkristallisiert. Man erhielt 0,28 g 2',5'-Dideoxy-5-äthyl-5'-(2-phenylbutyramido)uridin vom Schmelzpunkt 235-236° C.

B) In analoger Weise wurden erhalten:

a) aus 5'-Amino-2',5'-dideoxy-5-äthyluridin und 3-Phenylbutyrylchlorid:
2',5'-Dideoxy-5-äthyl-5'-(3-phenylbutyramido)uridin, Schmelzpunkt 222-223° C;

b) aus 5'-Amino-2',5'-dideoxy-5-äthyluridin und diphenylacetylchlorid:
2',5'-Dideoxy-5-äthyl-5'-(2,2-diphenylacetamido)uridin, Schmelzpunkt 208-210° C; und

c) aus 5'-Amino-2',5'-dideoxy-5-äthyluridin und Cyclohexylphenylacetylchlorid:
5'-(2-Cyclohexyl-2-phenylacetamido)-2',5'-dideoxy-5-äthyluridin vom Schmelzpunkt 130-132° C.

Beispiel 29

Eine Aufschlämmung von 0,4 g Triphenylessigsäure in 10 ml Benzol wurde unter Rühren bei 25° C mit 0,2 g Oxalylchlorid und einem Tropfen Dimethylformamid versetzt. Nach 30 Minuten hörte die Gasentwicklung auf und eine Lösung wurde erhalten. Diese Lösung wurde bei Raumtemperatur 1,5 Stunden stehen gelassen und dann eingedampft, wobei das Triphenylacetylchlorid als Oel erhalten wurde. Das Säurechlorid wurde auf -10° C gekühlt und mit 10 ml trockenem Pyridin und 0,36 g 5'-Amino-2',5'-dideoxy -5-äthyluridin versetzt. Dieses Gemisch wurde 15 Minuten geschüttelt, wobei sich das Säurechlorid löste und die Temperatur auf 0° C stieg. Die Lösung wurde dann 4 Stunden bei 0° C gerührt und eingedampft. Der Rückstand wurde 3 mal mit Toluol abgedampft, wobei ein Gummi erhalten wurde, der mit 20 ml Wasser verrieben wurde und 0,66 g eines Feststoffs vom Schmelzpunkt 110-114° C lieferte. Umkristallisation aus 12 ml Aethanol und anschliessende Chromatographie an einer Silicagelsäule mit Methylenchlorid/Methanol (9:1) und Umkristallisation aus einem Gemisch aus 5 ml Toluol, 10 ml Cyclohexan und 0,1 ml Aethylacetat lieferte 0,25 g 2',5'-Dideoxy-5-äthyl-5'-(triphenylacetamido)uridin vom Schmelzpunkt von etwa 110-120° C (Zersetzung).

Beispiel 30

In analoger Weise wie in Beispiel 29 wurde aus 0,66 g 2-Phenylisobuttersäure und 0,89 g 5'-amino-2',5'-dideoxy-5-äthyluridin nach Chromatographie und Verreiben mit Diäthyläther 0,23 g 2',5'-Dideoxy-5-äthyl -5'-(2-methyl-2-phenylpropionamido)uridin vom Schmelzpunkt 156-158° C erhalten.

Beispiel 31

In analoger Weise wie in Beispiel 29 wurde unter Verwendung von Thionylchlorid zur Herstellung des Säurechlorids aus 0,55 ml 2(RS)-Phenylpropionsäure und 0,89 g 5'-Amino-2',5'-dideoxy -5-äthyluridin nach Umkristallisation aus Aethanol 0,59 g 2',5'-Dideoxy-5-äthyl -5'-[2(RS)-phenylpropionamido)]uridin vom Schmelzpunkt 237-239 °C erhalten.

Beispiel 32

A) Eine Suspension von 2,35 g 2(RS)-(2,4-Dichlorphenoxy)propionsäure in einem Gemisch von 50 ml Benzol, 20 ml Oxalylchlorid und 0,1 ml Dimethylformamid wurde bei Raumtemperatur 1,5 Stunden gerührt. Das Gemisch wurde zur Trockene eingedampft und der Rückstand in 50 ml Diäthyläther gelöst und zu einer Lösung von 2,55 g 5'-Amino-2',5'-dideoxy-5-äthyluridin in 40 ml 0,25M Natriumhydroxidlösung gegeben. Das Gemisch wurde 10 Minuten kräftig geschüttelt und dann filtriert. Der Feststoff wurde mit 100 ml Wasser und 50 ml Diäthyläther gewaschen und aus 1 L Aethanol umkristallisiert und lieferte 2,31 g 5'-[2(RS)-(2,4-Dichlorphenoxy)propionamid] -2',5'-dideoxy-5-äthyluridin als weissen Feststoff vom Schmelzpunkt 240-243 °C.

B) In analoger Weise wurden erhalten:

1) aus 2(RS)-(2,6-dichlorphenoxy)propionsäure und 5'-Amino-2',5'-dideoxy-5-äthyluridin:
das 5'-[2(RS)-(2,6 -Dichlorphenoxy)propionamido]-2',5'-dideoxy-5-äthyluridin, Schmelzpunkt 206 °C;

2) aus 2(RS)-(3,5-Dichlorphenoxy)propionsäure und 5'-Amino-2',5'-dideoxy-5-äthyluridin:
das 5'-[2(RS)-(3,5 -Dichlorphenoxy)propionamido]-2',5'-dideoxy-5-äthyluridin, Schmelzpunkt 224-226 °C;

3) aus 2(RS)-(2-Chlorphenoxy)propionsäure und 5'-Amino-2',5'-dideoxy-5-äthyluridin:
das 5'-[2(RS)-(2-Chlorphenoxy)propionamido]-2',5'-dideoxy-5-äthyluridin, Schmelzpunkt 204-206 °C;

4) aus 2-(2,6-Dichlorphenoxy)essigsäure und 5'-Amino-2',5'-dideoxy-5-äthyluridin:
das 5'-[2-(2,6-Dichlorphenoxy)acetamido]-2',5'-dideoxy-5-äthyluridin, Schmelzpunkt 193,5-194 °C;

5) aus 2-(2,4-Dichlorphenoxy)essigsäure und 5'-Amino-2',5'-dideoxy-5-äthyluridin:
das 5'-[2-(2,4-Dichlorphenoxy)acetamido]-2',5'-dideoxy-5-äthyluridin, Schmelzpunkt 191-193 °C;

6) aus 4-(2,6-Dichlorphenoxy)buttersäure und 5'-Amino-2',5'-dideoxy-5-äthyluridin:
das 5'-[4-(2,6-Dichlorphenoxy)butyramido]-2',5'-dideoxy-5-äthyluridin, Schmelzpunkt 218-220 °C;

7) aus 6-(2,6-Dichlorphenoxy)hexansäure und 5'-Amino-2',5'-dideoxy-5-äthyluridin:
das 5'-[6-(2,6-Dichlorphenoxy)hexanamido]-2',5'-dideoxy-5-äthyluridin, Schmelzpunkt 219-221 °C;

8) aus 5-(2,6-Dichlorphenoxy)pentansäure und 5'-Amino-2',5'-dideoxy-5-äthyluridin:
das 5'-[5-(2,6-Dichlorphenoxy)valeramido]-2',5'-dideoxy-5-äthyluridin, Schmelzpunkt 215-217 °C;

9) aus 3-(2,6-Dichlorphenoxy)propionsäure und 5'-Amino-2',5'-dideoxy-5-äthyluridin:
das 5'-[3-(2,6-Dichlorphenoxy)propionamido]-2',5'-dideoxy-5-äthyluridin, Schmelzpunkt 217-218 °C;

10) aus [2(RS)-(2-Chlor-4-nitrophenoxy)]propionsäure und 5'-Amino-2',5'-dideoxy-5-äthyluridin:
das 5'-[2(RS)-(2-Chlor-4-nitrophenoxy)propionamido]-2',5'-dideoxy-5-äthyluridin, Schmelzpunkt 214-216 °C;

11) aus [2(RS)-(2-Chlor-4-phenylphenoxy)]propionsäure und 5'-Amino-2',5'-dideoxy-5-äthyluridin:
das 5'-[2(RS)-(2-Chlor-4-phenylphenoxy)propionamido]-2',5'-dideoxy-5-äthyluridin, Schmelzpunkt 219-228 °C;

12) aus 2(R)-(2,4-Dichlorphenoxy)propionsäure und 5'-Amino-2',5'-dideoxy-5-äthyluridin:
das 5'-[2(R)-(2,4-Dichlorphenoxy)propionamido]-2',5'-dideoxy-5-äthyluridin, Schmelzpunkt 230-231 °C;

13) aus 2(S)-(2,4-Dichlorphenoxy)propionsäure und 5'-Amino-2',5'-dideoxy-5-äthyluridin:
das 5'-[2(S)-(2,4-Dichlorphenoxy)propionamido]-2',5'-dideoxy-5-äthyluridin, Schmelzpunkt 260.5-261.5 °C;

14) aus (2(RS)-(2,4,5-Trichlorphenoxy)propionsäure und 5'-Amino-2',5'-dideoxy-5-äthyluridin:
das 5'-[2(RS)-(2,4,5-Trichlorphenoxy)propionamido]-2',5'-dideoxy-5-äthyluridin, Schmelzpunkt 255-257 °C;

15) aus 2(RS)-(2,4-Dichlorphenoxy)buttersäure und 5'-Amino-2',5'-dideoxy-5-äthyluridin:
das 5'-[2(RS)-(2,4-Dichlorphenoxy)butyramido]-2',5'-dideoxy-5-äthyluridin, Schmelzpunkt 223-227 °C;

16) aus 2(RS)-(4-Chlor-2-nitrophenoxy)propionsäure und 5'-Amino-2',5'-dideoxy-5-äthyluridin:
das 5'-[2(RS)-(4-Chlor-2-nitrophenoxy)propionamido]-2',5'-dideoxy-5-äthyluridin, Schmelzpunkt 199-2O1 °C;

17) aus 2(RS)-(4-Acetamido-2-chlorphenoxy)propionsäure und 5'-Amino-2',5'-dideoxy-5-äthyluridin:
das 5'-[2(RS)-(4-Acetamido-2-chlorphenoxy)propionamido]-2',5'-dideoxy-5-äthyluridin, Schmelzpunkt

234-236 ° C;
18) aus 2(RS)-(2,4-Dichlorphenoxy)-2-phenylessigsäure und 5'-Amino-2',5'-dideoxy-5-äthyluridin: das 5'-[2(RS)-(2,4-Dichlorphenoxy)-2-phenylacetamido]-2',5'-dideoxy-5-äthyluridin, Schmelzpunkt 120-125 ° C;
19) aus 2(RS)-(2,4-Dichlor-5-methoxyphenoxy)propionsäure und 5'-Amino-2',5'-dideoxy-5-äthyluridin: das 5'-[2(RS)-(2,4-Dichlor-5-methoxyphenoxy)propionamido]-2',5'-dideoxy-5-äthyluridin, Schmelzpunkt 191-194 ° C;
20) aus 2(RS)-(2-Chlor-4-methoxyphenoxy)propionsäure und 5'-Amino-2',5'-dideoxy-5-äthyluridin: das 5'-[2(RS)-(2-Chlor-4-methoxyphenoxy)propionamido]-2',5'-dideoxy-5-äthyluridin, Schmelzpunkt 215-221 ° C;
21) aus 2(RS)-(2-Methylbiphenylyloxy)propionsäure und 5'-Amino-2',5'-dideoxy-5-äthyluridin: das 2',5'-Dideoxy-5-äthyl-5'-[2(RS)-(2-methylbiphenylyloxy)propionamido]uridin, Schmelzpunkt 216-217 ° C; 22) aus 2(RS)-(2,4-Dichlorphenoxy)propionsäure und 5'-Amino-5'-deoxythymidin: das 5'-[2(RS)-(2,4-Dichlorphenoxy)propionamido]-5'-deoxythymidin, Schmelzpunkt 208-210 ° C;
23) aus 2-(2,4-Dichlorphenoxy)-2-methylpropionsäure und 5'-Amino-2',5'-dideoxy-5-äthyluridin: das 5'-[2-(2,4-Dichlorphenoxy)-2-methylpropionamido]-2',5'-dideoxy-5-äthyluridin, Schmelzpunkt 188-190 ° C;
24) aus 2(RS)-Phenoxypropionsäure und 5'-amino-2',5'-dideoxy-5-äthyluridin: das 2',5'-Dideoxy-5-äthyl-5'-[2(RS)-phenoxypropionamido]uridin, Schmelzpunkt 214-216 ° C;
25) aus 2(RS)-(2-Fluorphenoxy)propionsäure und 5'-Amino-2',5'-dideoxy-5-äthyluridin: das 2',5'-Dideoxy-5-äthyl-5'-[2(RS)-fluorphenoxy)propionamido]uridin, Schmelzpunkt 205-207 ° C;
26) aus 2(RS)-(2-Trifluormethylphenoxy)propionsäure und 5'-Amino-2',5'-dideoxy-5-äthyluridin: das 2',5'-Dideoxy-5-äthyl-5'-[2(RS)-(2-trifluoromethylphenoxy)propionamido]uridin, Schmelzpunkt 221-225 ° C; und
27) aus 2(RS)-(2-Phenylphenoxy)propionsäure und 5'-Amino-2',5'-dideoxy-5-äthyluridin: das 2',5-Dideoxy-5-äthyl-5'-[2(RS)-(2-phenylphenoxy)propionamido]uridin, Schmelzpunkt 206-207 ° C.

Die 4-(2,6-Dichlorphenoxy)buttersäure wurde wie folgt hergestellt:

353 mg Natrium wurde in kleinen Stücken zu einer gerührten Lösung von 2,5 g 2,6-Dichlorphenol in 10 ml Aethanol gegeben. Das Gemisch wurde bei Raumtemperatur gerührt, bis die Gasentwicklung aufhörte. Danach wurden 2,715 g 4-Brombuttersäuremethylester gegeben und das Gemisch 4 Stunden unter Rühren zum Rückfluss erhitzt. Nach Kühlen wurde das Gemisch abfiltriert und das Filtrat zur Trockene eingedampft. der Rückstand wurde in 50 ml Dichlormethan gelöst und die Lösung mit 10% Natriumcarbonatlösung gewaschen, über Natriumsulfat getrocknet und eingedampft. Man erhielt 3,62 g eines farblosen Oels.

Dieses Oel wurde in 20 ml Aethanol gelöst und die Lösung mit einer Lösung von 0,5 g Kaliumhydroxid in 10 ml Wasser versetzt. Das Gemisch wurde 3 Stunden unter Rühren zum Rückfluss erhitzt und dann zur Trockene eingedampft. Der Rückstand wurde in 50 ml gesättigter Natriumhydrogencarbonatlösung aufgenommen und die Lösung 2 mal mit je 50 ml Dichlormethan gewaschen, mit 2M Salzsäure angesäuert und mit 50 ml Dichlormethan extrahiert. Der Dichlormethanextrakt wurde über Natriumsulfat getrocknet und eingedampft und lieferte 1,3 g 4-(2,6-Dichlorphenoxy)buttersäure in Form eines weissen kristallinen Feststoffs vom Schmelzpunkt 66-67 ° C.

In analoger Weise wurde aus 2,6-Dichlorphenol und 6-Bromhexansäuremethylester die 6-(2,6-Dichlorphenoxy)hexansäure und aus 2,6-Dichlorphenol und 5-Brompentansäuremethylester die 5-(2,6-Dichlorphenoxy)pentansäure erhalten.

## Beispiel 33

In analoger Weise wie in Beispiel 32 wurde aus 2(RS)-(2,4-Dichlorphenoxy)propionsäure und 1-(5-Amino-2,5-dideoxy-2-fluor-ß-D-arabinofuranosyl) -5-äthyluracil das 1-[5-[2(RS)-(2,4-Dichlorphenoxy)-propionamido] -2,5-dideoxy-2-fluor-ß-D-arabinofuranosyl] -5-äthyluracil vom Schmelzpunkt 194-214 ° C erhalten.

Das als Ausgangsmaterial verwendete 1-(5-Amino-2,5-dideoxy -2-fluor-ß-D-arabinofuranosyl)-5-äthyluracil wurde wie folgt hergestellt:

Ein Gemisch von 0,4 g 1-(2-Deoxy-2-fluor-ß-D-arabinofuranosyl) -5-äthyluracil, 0,44 g Triphenylphosphin, 0,48 g Natriumazid und 6 ml trockenes Dimethylformamid wurden unter Stickstoff bei Raumtemperatur gerührt, wobei nach und nach 0,48 g Kohlenstofftetrabromid zugegeben wurde. Das Gemisch wurde 20 Stunden unter Stickstoff bei Raumtemperatur gerührt. Danach wurde 3 ml Methanol zugesetzt, das Gemisch 0,5 Stunden gerührt und dann eingedampft. Der Rückstand wurde eine halbe Stunde mit 8 ml 0,5M Natronlauge gerührt. Die erhaltene Suspension wurde abfiltriert und das unlösliche Triphenylphosphinoxid

mit Wasser gewaschen. Das Filtrat wurde auf pH 5 mit Salzsäure angesäuert und 3 mal mit 20 ml Methylacetat extrahiert. Die Extrakte wurden über Magnesiumsulfat getrocknet, filtriert und eingedampft. Der als Rückstand erhaltene Feststoff wurde auf dem Filter mit 10 ml Diäthyläther/Petroläther (1:1) gewaschen und dann unter vermindertem Druck getrocknet. Man erhielt 0,4 g 1-(5-Azido-2,5-dideoxy-2-fluor-ß-D-arabinofuranosyl) -5-äthyluracil als weissen Feststoff vom Schmelzpunkt 185-188° C.

Eine Lösung von 0,4 g 1-(5-Azido-2,5-dideoxy -2-fluor-ß-D-arabinofuranosyl)-5-äthyluracil in 20 ml Aethanol wurde in Gegenwart von 0,1 g Palladium/Kohle 24 Stunden unter Atmosphärendruck bei Raumtemperatur hydriert. Der Katalysator wurde abfiltriert und das Filtrat eingedampft. Der Rückstand wurde mit Diäthyläther verrieben und der Feststoff abfiltriert, mit Aethylacetat gewaschen und unter vermindertem Druck getrocknet. Man erhielt 0,3 g 1-(5-Amino-2,5-dideoxy-2-fluor -ß-D-arabinofluoranosyl)-5-äthyluracil als weissen Feststoff.

Beispiel 34

In analoger Weise wie in Beispiel 32 wurde aus 2(RS)-(2,4-Dichlorphenoxy)propionsäure und 1-(5-Amino-2,5-dideoxy-2-fluor-ß-D-arabinofuranosyl)thymin das 1-[5-[2(RS)-(2,4-Dichlorphenoxy)propionamido]-2,5-dideoxy-2-fluor-ß-D-arabinofuranosyl]thymidin vom Schmelzpunkt 147-155° C erhalten.

Das als Ausgangsmaterial verwendete 1-(5-Amino-2,5-dideoxy -2-fluor-ß-D-arabinofuranosyl)thymin wurde aus 1-(2-Deoxy-2-fluor-ß-D-arabinofuranosyl)thymin in Analogie zu der in Beispiel 33 beschriebenen Herstellung von 1-(5-Amino-2,5-dideoxy-2-fluor-ß-D-arabinofuranosyl)-5-äthyluracil hergestellt.

Beispiel 35

Eine Suspension von 3,0 g 2(RS)-(2,4-Dichlorphenylthio)propionsäure in einem Gemisch von 50 ml Toluol, 5 ml Oxalylchlorid und 0,1 ml Dimethylformamid wurde 2,5 Stunden bei Raumtemperatur gerührt. Das Gemisch wurde zur Trockene eingedampft und eine Lösung des Rückstandes in 20 ml Diäthyläther zu einer Lösung von 3,05 g 5'-Amino-2',5'-dideoxy-5-äthyluridin in 32 ml 0,375M Natriumhydroxidlösung gegeben. Das Gemisch wurde 5 Minuten kräftig geschüttelt und dann filtriert. Der Feststoff wurde mit Wasser gewaschen und aus 600 ml Aethanol umkristallisiert, wobei 3,0 g 5'-[2(RS)-(2,4-Dichlorphenylthio)-priopionamido]-2',5'-dideoxy-5-äthyluridin als hellgelben Feststoff vom Schmelzpunkt 218-220° C erhalten wurden.

Die als Ausgangsmaterial verwendete 2(RS)-(2,4-Dichlorphenylthio)propionsäure wurde wie folgt hergestellt:

Ein Gemisch von 11,78 g 2,4-Dichlorthiophenol und 12,0 g Kaliumhydroxid in 50 ml Aceton wurde unter Rühren zum Rückfluss erhitzt. Nach und nach wurden 11,77 g 2-Brompropionsäureäthylester zugesetzt und das Gemisch wurde 22 Stunden zum Rückfluss erhitzt. Nach Kühlen auf Raumtemperatur wurde das Gemisch filtriert und das Filtrat zur Trockene eingedampft. Der Rückstand wurde in 400 ml Diäthyläther aufgenommen und die Lösung mit 400 ml Wasser und 400 ml 10% Natriumcarbonatlösung gewaschen, über Natriumsulfat getrocknet und zur Trockene eingedampft. Eine Lösung des Rückstandes in einem Gemisch von 30 ml Aethanol und 17 ml 10% Kaliumhydroxidlösung wurde 21 Stunden zum Rückfluss erhitzt. Die Lösung wurde zur Trockene eingedampft und der Rückstand in 50 ml Wasser gelöst. Die erhaltene Lösung wurde 2 mal mit je 50 ml Diäthyläther gewaschen und dann mit konzentrierter Salzsäure angesäuert und 2 mal mit je 150 ml Aethylacetat extrahiert. Die vereinigten organischen Extrakte wurden 2 mal mit je 200 ml Wasser gewaschen, über Natriumsulfat getrocknet und zur Trockene eingedampft. Der Rückstand wurde aus Diäthyläther kristallisiert und lieferte 4,08 g 2(RS)-(2,4-Dichlorphenylthio)propionsäure in Form eines gelben Feststoffs vom Schmelzpunkt 95-98° C.

Beispiel 36

Eine Suspension von 250 mg 5'-[2(RS)-(2,4-Dichlorphenylthio)propionamido] -2',5'-dideoxy-5-äthyluridin in 10 ml Essigsäure wurde auf 0° C gekühlt und mit 60 mg 30% Wasserstoffperoxid versetzt. Das Gemisch wurde eine halbe Stunde bei 0° C und über Nacht bei Raumtemperatur gerührt. Danach wurden weitere 60 mg 30% Wasserstoffperoxid zugesetzt und das Gemisch 24 Stunden gerührt. Dann wurden weitere 30 mg 30% Wasserstoffperoxid zugesetzt und das Gemisch 4 Stunden gerührt und zur Trockene eingedampft. Der Rückstand wurde mit Methanol verrieben und der erhaltene Feststoff abfiltriert und aus Methanol umkristallisiert. Man erhielt 90 mg 5'-[2(RS)-(2,4-Dichlorphenylsulphinyl)propionamido] -2',5'-dideoxy-5-äthyluridin in Form eines weissen Feststoffs vom Schmelzpunkt 208-209° C.

Beispiel 37

Eine Suspension von 250 mg 5'-[2(RS)-(2,4-Dichlorphenylthio)propionamido] -2',5'-dideoxy-5-äthyluridin in 10 ml Essigsäure wurde auf 0° C gekühlt und mit 800 mg 30% Wasserstoffperoxid versetzt. Das Gemisch wurde eine halbe Stunde bei 0° C und über Nacht bei Raumtemperatur gerührt. Das Gemisch wurde zur Trockene eingedampft und der Rückstand aus Methanol kristallisiert. Man erhielt 140 mg 5'-[2-(RS)-(2,4-Dichlorphenylsulfonyl)propionamido] -2',5'-dideoxy-5-äthyluridin als weissen Feststoff vom Schmelzpunkt 183-184° C.

Beispiel 38

A) Eine Suspension von 51 mg 2,6-Dichlorphenylessigsäure in einem Gemisch von 10 ml Toluol, 100 mg Oxalylchlorid und einem Tropfen Dimethylformamid wurde eine halbe Stunde bei Raumtemperatur gerührt. Das Gemisch wurde zur Trockene eingedampft und eine Lösung des Rückstandes in 2 ml Diäthyläther zu einer Lösung von 70 mg 5'-(2-Aminoäthyl)-2',5'-dideoxy -5-äthyluridin in 5 ml 0,1M Natronlauge gegeben. Das Gemisch wurde 5 Minuten kräftig geschüttelt und dann filtriert. Der Feststoff wurde nacheinander mit Wasser, Aethanol und Diäthyläther gewaschen und über Natriumsulfat getrocknet. Man erhielt 90 mg 5'-[2-(2,6-Dichlorphenylacetamido)äthyl] -2',5'-dideoxy-5-äthyluridin als weissen Feststoff vom Schmelzpunkt 227-229° C.

B) In analoger Weise wurden erhalten:

a) aus 5'-[2-(Aminoäthyl)]-2',5'-dideoxy-5-äthyluridin und 2(RS)-(2,4,5-Trichlorphenoxy)propionsäure: das 5'-[2-[2(RS)-(2,4,5-Trichlorphenoxy)propionamido]äthyl]-2',5'-dideoxy-5-äthyluridin, Schmelzpunkt 184-185° C; und

b) aus 5'-[2-(Aminoäthyl)]-2',5'-dideoxy-5-äthyluridin und 2(RS)-(2,4-Dichlorphenoxy)propionsäure: das 5'-[2-[2(RS)-(2,4-Dichlorphenoxy)propionamido]äthyl]-2',5'-dideoxy-5-äthyluridin vom Schmelzpunkt 170-172° C.

Das als Ausgangsmaterial verwendete 5'-(2-Aminoäthyl)-2',5'-dideoxy -5-äthyluridin wurde wie folgt hergestellt:

1,9 g Dichloressigsäure wurden zu einem Gemisch von 8,95 g 3'-O-Acetyl-2',5'-dideoxy -5-äthyluridin und 18,6 g Dicyclohexylcarbodiimid in 75 ml Dimethylsulfoxid gegeben. Das Gemisch wurde 24 Stunden bei Raumtemperatur gerührt, danach wurden 1,2 ml Pyridin und 10,5 g Carbäthoxymethylentriphenylphosphoran zugesetzt und das Gemisch weitere 24 Stunden bei Raumtemperatur gerührt. Das Lösungsmittel wurde unter vermindertem Druck entfernt und der Rückstand in 300 ml Aethylacetat gelöst. Die Lösung wurde mit 300 ml Wasser gewaschen, über Natriumsulfat getrocknet und zu einem gelben Oel eingedampft. Dieses Oel wurde durch Flash-Chromatographie an einer Silicagelsäule mit 5% Methanol/Dichlormethan gereinigt. Das Produkt wurde aus Aethanol umkristallisiert und lieferte 7,3 g trans-3'-O-Acetyl-2',5'-dideoxy-5'-(äthoxycarbonylmethylen) -5-äthyluridin als weissen Feststoff vom Schmelzpunkt 132-133° C.

Eine Lösung von 7,3 g trans-3'-O-Acetyl-2',5'-dideoxy-5'-(äthoxycarbonylmethylen)-5-äthyluridin in 150 ml Aethanol wurde 6 Stunden über 0,5 g 10% Palladium/Kohle hydriert. Der Katalysator wurde abfiltriert und das Filtrat zur Trockene eingedampft. Der Rückstand wurde aus Aethanol umkristallisiert und lieferte 5,8 g 3'-O-Acetyl-2',5'-dideoxy-5'-(äthoxycarbonylmethyl) -5-äthyluridin als weissen Feststoff vom Schmelzpunkt 137-138° C.

0,4 g Lithiumborhydrid wurden zu einer Lösung von 5,6 g 3'-O-Acetyl-2',5'-dideoxy -5'-(äthoxycarbonylmethyl)-5-äthyluridin in 100 ml Tetrahydrofuran gegeben. Das Gemisch wurde 4 Stunden bei Raumtemperatur gerührt, mit weiteren 0,2 g Lithiumborhydrid versetzt, über Nacht bei Raumtemperatur gerührt und dann 1 Stunde zum Rückfluss erhitzt. Das Lösungsmittel wurde unter vermindertem Druck abgedampft und der Rückstand in Methanol gelöst. Nach einigen Minuten stehender Raumtemperatur wurde das Lösungsmittel abgedampft und der Rückstand in 40 ml Pyridin aufgenommen. Danach wurden 5 g Acetanhydrid zugesetzt und das Gemisch über Nacht bei Raumtemperatur gerührt. Das Pyridin wurde abgedampft und der Rückstand zwischen Wasser und Aethylacetat verteilt. Die wässrige Lösung wurde zur Trockene eingedampft und der Rückstand durch Flash-Chromatographie an einer Silicagelsäule mit 10% Methanol/Dichlormethan gereinigt. Man erhielt 1,3 g 5'-(2-Acetoxyäthyl)-3'-O-acetyl -2',5'-dideoxy-5-äthyluridin als farbloses Oel, das beim Stehen kristallisierte.

0,10 g 5'-(2-Acetoxyäthyl)-3'-O-acetyl-2',5'-dideoxy-5-äthyluridin wurden mit 5 ml einer verdünnten Lösung von Natriummethoxid in Methanol versetzt. Nach 2 Stunden Stehen bei Raumtemperatur wurde die Lösung durch Zusatz eines quer-vernetzten Polystyrol-Divinylbenzol-Kationenaustauscherharzes mit Sulfonsäuregruppen ($H^+$-Form) neutralisiert und dann filtriert. Das Filtrat wurde zur Trockene eingedampft und lieferte 0,045 g 2',5'-Dideoxy-5-äthyl-5'-(2-hydroxyäthyl)uridin als weissen Feststoff vom Schmelzpunkt 113-115° C.

Ein Gemisch von 1,25 g 2',5'-Dideoxy-5-äthyl-5'-(2-hydroxyäthyl)uridin, 1,18 g Triphenylphosphin, 1,49 g Natriumazid und 1,55 g Kohlenstofftetrabromid in 30 ml Dimethylformamid wurde über Nacht bei Raumtemperatur gerührt. Das Gemisch wurde zur Trockene verdampft und der Rückstand zwischen Aethylacetat und Wasser verteilt. Die Aethylacetatlösung wurde zur Trockene eingedampft und der Rückstand durch Flash-Chromatographie an einer Silicagelsäule mit 1% Methanol/Dichlormethan gereinigt. Man erhielt 0,28 g 5'-(2-Azidoäthyl)-2',5'-dideoxy -5-äthyluridin als farbloses Oel, das beim Stehen kristallisierte.

Eine Lösung von 0,28 g 5'-(2-Azidoäthyl)-2',5'-dideoxy-5-äthyluridin in 50 ml Aethanol wurde 2 Stunden über 50 mg 10% Palladium/Kohle hydriert. Der Katalysator wurde abfiltriert und das Filtrat zur Trockene eingedampft. Man erhielt 0,23 g 5'-(2-Aminoäthyl)-2',5'-dideoxy -5-äthyluridin als farbloses Oel, das beim Stehen kristallisierte.

Beispiel 39

0,175 g 2(RS)-(2,6-Dichlorbenzyl)propionsäure wurden in 5 ml trockenem Benzol, das einen Tropfen Dimethylformamid enthielt gelöst. Die Lösung wurde mit 96 mg Oxalylchlorid versetzt und nach 1 Stunde wurde das Lösungsmittel abgedampft. Der Rückstand wurde mit einer Lösung von 5'-Amino-2',5'-dideoxy -5-äthyluridin in 5 ml einer wässrigen Lösung die 3 ml 2M Natriumhydroxidlösung enthielt versetzt. Das Gemisch wurde 15 Minuten geschüttelt, der Niederschlag abfiltriert, über Natriumsulfat getrocknet und aus einem Gemisch von Aethanol und Diäthyläther umkristallisiert. Man erhielt 0,11 g 5'-[2(RS)-(2,6-Dichlorbenzyl)propionamido]-2',5'-dideoxy-5-äthyluridin vom Schmelzpunkt 194-197° C.

Die als Ausgangsmaterial verwendete 2(RS)-(2,6-Dichlorbenzyl)propionsäure wurde wie folgt hergestellt:

2,4 g Triäthyl-2-phosphinopropionat und 1,75 g 2,6-Dichlorbenzaldehyd wurden in 15 ml trockenem Dimethylformamid gelöst. Die Lösung wurde nach und nach mit 0,29 g einer 80% Dispersion von Natriumhydrid in Mineralöl versetzt und das Gemisch wurde 2 Stunden gerührt. Danach wurden 100 ml Wasser zugesetzt und das Gemisch 3 mal mit je 40 ml Methylenchlorid extrahiert. Die vereinigten Methylenchloridextrakte wurden 2 mal mit je 50 ml Natriumchloridlösung zurückgewaschen und das Lösungsmittel abgedampft. Man erhielt 2,4 g 2,6-Dichlor-2-methylzimtsäureäthylester als Oel. 0,26 g 2,6-Dichlor-2-methylzimtsäureäthylester wurden in 10 ml Methanol gelöst und die Lösung mit 0,96 g Magnesiumspänen versetzt und gerührt. Das Gemisch wurde auf -5° C gekühlt und 1 Stunde bei dieser Temperatur und 4 Stunden bei Raumtemperatur gehalten. Danach wurden während 20 Minuten 25 ml 6M Salzsäure zugetropft, wobei eine klare Lösung erhalten wurde. Die Lösung wurde 3 mal mit je 15 ml Diäthyläther extrahiert, in die vereinigten Extrakte wurden über Natriumsulfat getrocknet und eingedampft. Man erhielt 0,21 g eines farblosen Oels, das gemäss NMR-Spektroskopie ein 2:1-Gemisch des Methyl- und Aethylesters von 2(RS)-(2,6-Dichlorbenzyl)propionsäure war.

0,20 g des obigen Gemisches wurden in einem Gemisch von 5 ml 10% Kalilauge und 5 ml Aethanol gelöst. Die Lösung wurde 2 Stunden zum Rückfluss erhitzt, das Aethanol abgedampft und der Rückstand gekühlt und mit konzentrierter Salzsäure angesäuert. Nach Extraktion mit 3 mal 10 ml Aethylacetat, Waschen mit Natriumchlorid, Trocknen über Natriumsulfat und Abdampfen des Lösungsmittels wurden 0,18 g 2(RS)-(2,6-Dichlorbenzyl)propionsäure als Oel erhalten.

Beispiel 40

In analoger Weise wie in Beispiel 39 wurden erhalten:

A) aus 0,21 g 2(RS)-(2,4-Dichlorbenzyl)propionsäure und 0,20 g 5'-Amino-2',5'-dideoxy-5-äthyluridin:

0,35 g 5'-[2(RS)-(2,4-Dichlorbenzyl)propionamido]-2',5'-dideoxy -5-äthyluridin, Schmelzpunkt 243-245° C,

B) aus 0,20 g 5-chloro-2,3-Dihydro-2(RS)-benzofurancarbonsäure und 0,26 g 5'-Amino-2',5'-dideoxy-5--äthyluridin:

0,10 g 5'-[5-Chlor-2,3-dihydro-2(RS)-benzofuranylcarboxamido] -2',5'-dideoxy-5-äthyluridin, Schmelzpunkt 204-206° C,

C) aus 0,10 g 6-Chlorchroman-2-carbonsäure und 0,12 g 5'-amino-2',5'-dideoxy-5-äthyluridin:

20 mg 5-(6-Chlor-2H-1-benzopyran-2-ylcarboxamido)-2',5' -dideoxy-5-äthyluridin, Schmelzpunkt 171-176° C.

Beispiel 41

In Analogie zu Beispiel 15 wurden aus 0,335 g 2-(4-Benzyloxy-2,6-dimethylphenyl)essigsäure und 0,28 g 5'-amino-2',5'-dideoxy -5-äthyluridin nach Umkristallisation aus einem Gemisch von 2-Methoxyäthanol und Wasser 0,33 g 5'-[2-(4-Benzyloxy-2,6-dimethylphenyl)acetamido]-2',5'-dideoxy-5-äthyluridin vom Schmelzpunkt 259-260 °C erhalten.

Die als Ausgangsmaterial verwendete 2-(4-Benzyloxy-2,6-dimethylphenyl)essigsäure wurde wie folgt hergestellt:

0,36 g 2-(4-Hydroxy-2,6-dimethylphenyl)essigsäure wurde mit 184 mg einer 60%-igen Dispersion von Natriumhydrid in Mineralöl und 0,72 g Benzylbromid in 10 ml trockenem Dimethylformamid versetzt. Das Lösungsmittel wurde abgedampft und der Rückstand zwischen Wasser und Methylenchlorid verteilt. Die organische Phase wurde eingedampft und lieferte 0,63 g Rohprodukt vom Schmelzpunkt 54-57 °C. Umkristallisation aus wässrigem Methanol lieferte 0,58 g reines Benzyl-2-(4-benzyloxy-2,6-dimethylphenyl)acetat vom Schmelzpunkt 62-64 °C.

0,35 g Benzyl-2-(4-benzyloxy-2,6-dimethylphenyl)acetat wurde mit einem Gemisch von 7 ml Methanol, 0,7 ml Wasser und 0,28 g festem Natriumhydroxid über Nacht geschüttelt. Die erhaltene Lösung wurde eingedampft und der Rückstand zwischen 50 ml Methylenchlorid und 25 ml 2M Salzsäure verteilt. Die organische Phase wurde über Natriumsulfat getrocknet und eingedampft. Der feste Rückstand wurde aus einem Gemisch von 3 ml Methanol und 3 ml Wasser umkristallisiert und lieferte 0,21 g 2-(4-Benzyloxy-2,6-dimethylphenyl)essigsäure vom Schmelzpunkt 149-150 °C.

## Beispiel 42

0,25 g 5'-[2-(4-Benzyloxy-2,6-dimethylphenyl)acetamido]-2',5'-dideoxy-5-äthyluridin wurden in einer Aufschlämmung mit 5 ml trockenem Pyridin und 0,4 ml Acetanhydrid 72 Stunden gerührt. Die erhaltene Lösung wurde unter vermindertem Druck eingedampft und der Rückstand 3 mal mit Toluol abgedampft. Nach Verreiben mit Diäthyläther und Filtrieren wurden 0,22 g Rohprodukt vom Schmelzpunkt 188-191 °C erhalten. Umkristallisation aus einem Gemisch von 4 ml Methylenchlorid und 4 ml Petroläther lieferte 0,20 g reines 3'-O-Acetyl-5'-[2-(4-benzyloxy-2,6-dimethylphenyl)acetamido] -2',5'-dideoxy-5-äthyluridin vom Schmelzpunkt 194-196 °C (Zersetzung).

## Beispiel 43

0,165 g 3'-O-Acetyl-5'-[2-(4-benzyloxy -2,6-dimethylphenyl)acetamido]-2',5'-dideoxy -5-äthyluridin wurden in 25 ml Methanol gelöst. Die Lösung wurde mit einer Aufschlämmung von 0,1 g 5% Palladium/Kohle in Aethanol versetzt und das Gemisch 2 Stunden bei Raumtemperatur unter Atmosphärendruck hydriert. Der Katalysator wurde abfiltriert und das Filtrat eingedampft, wobei 0,125 g eines Feststoffs vom Schmelzpunkt 234-237 °C erhalten wurden. Umkristallisation aus einem Gemisch von 10 ml Aceton und 20 ml Petroläther lieferte 80 mg 3'-O-Acetyl-2',5'-dideoxy -5-äthyl-5'-[2-(4-hydroxy-2,6-dimethylphenyl)acetamido] uridin vom Schmelzpunkt 240-241 °C (Zersetzung).

## Beispiel 44

24 mg 5'-[2-(4-Benzyloxy-2,6-dimethylphenyl)acetamido]-2',5'-dideoxy-5-äthyluridin wurden in 50 ml Methanol gelöst und die Lösung wurde 72 Stunden über 15 mg 5% Palladium/Kohle bei Raumtemperatur unter Atmosphärendruck hydriert. Der Katalysator wurde abfiltriert und das Filtrat eingedampft, wobei 15 mg 2',5'-Dideoxy-5-äthyl-5'-[2-(4-hydroxy-2,6-dimethylphenyl) acetamido]uridin vom Schmelzpunkt 221-225 °C (Zersetzung) erhalten wurden.

## Beispiel 45

95 mg 3'-O-Acetyl-2',5'-dideoxy -5-äthyl-5'-[2-(4-hydroxy-2,6-dimethylphenyl) acetamido]uridin wurden in 5 ml trockenem Dimethylformamid unter Rühren bei Raumtemperatur gelöst. Die Lösung wurde mit 69 mg einer 60%-igen Dispersion von Natriumhydrid in Mineralöl versetzt und das Gemisch 1 Stunde gerührt. Dann wurden 68 mg frisch hergestelltes Dibenzylphosphorylchlorid in 2 ml trockenem Benzol zugesetzt und das Gemisch bei Raumtemperatur 18 Stunden gerührt. Das Gemisch wurde in 30 ml gesättigte wässrige Natriumbicarbonatlösung gegossen und mit Aethylacetat extrahiert. Der Extrakt wurde eingedampft und das erhaltene Oel durch Flash-Chromatographie an Silicagel mit 5% Methanol/Dichlormethan gereinigt. Das Lösungsmittel wurde abgedampft und man erhielt 65 mg eines Produktes, das nach Verreiben mit Petroläther (Siedepunkt 60-80 °C) 3'-O-Acetyl-2',5'-dideoxy-5-äthyl-5'-[2-(2,6-dimethyl-4-(dibenzyloxyphosphinyloxy) phenyl]acetamido]uridin als weissen kristallinen Feststoff lieferte.

Der obige Feststoff wurde in 10 ml einer gesättigten Lösung von Ammoniak in Methanol bei Raumtemperatur aufgenommen und über Nacht stehen gelassen. Das Lösungsmittel wurde abgedampft und man erhielt 2',5'-Dideoxy-5-äthyl-5'-[2-[2,6-dimethyl-4-(dibenzyloxyphosphinyloxy)phenyl] acetamido]uridin als Feststoff.

60 mg des obigen Feststoffes wurden in 10 ml Aethanol suspendiert und nach Zusatz von 10 mg 10% Palladium/Kohle 4 Stunden bei Raumtemperatur und Atmosphärendruck hydriert. Der Katalysator wurde abfiltriert und mit mehreren Portionen warmem Aethanol gewaschen. Die vereinigten Filtrate wurden eingedampft und lieferten 40 mg 2',5'-Dideoxy-5-äthyl-5'-[2-(2,6-dimethyl-4-phosphatophenyl)acetamido]-uridin vom Schmelzpunkt 214-216° C.

Beispiel 46

A) In analoger Weise wie in Beispiel 16 wurden aus 0,22 g (RS)-(2,4-Dichlorphenoxy)propionsäure und 0,27 g 5'-Amino-5-(2-chloräthyl)-2',5'-dideoxyuridinhydrochlorid nach Umkristallisation aus einem Gemisch von 2-Methoxyäthanol und Wasser 0,13 g 5-(2-Chloräthyl)-2',5'-dideoxy-5'-[2-(2,4-dichlorphenoxy) propionamido]uridin vom Schmelzpunkt 214-216° C erhalten.

B) Aus 55 mg Benzoylchlorid und 115 mg 5'-Amino-5-(2-chloräthyl)-2',5'-dideoxyuridinhydrochlorid nach Umkristallisation aus einem Gemisch von 2-Methoxyäthanol und Wasser, 70 mg 5'-Benzamido-5-(2-chloräthyl) -2',5'-dideoxyuridin vom Schmelzpunkt 249-250° C (Zersetzung).

C) aus 104 mg 2-(2,4,5-Trichlorphenoxy)propionsäure und 115 mg 5'-Amino-5-(2-chloräthyl)-2',5'-dideoxyuridinhydrochlorid nach Umkristallisation aus einem Gemisch von 2-Methoxyäthanol und Wasser, 95 mg 5-(2-Chloräthyl)-5-[2-(2,4,5-trichlorphenoxy)propionamido] -2',5'-dideoxyuridin vom Schmelzpunkt 223-225° C

D) aus 136 mg 2,6-Dichlorphenylessigsäure und 0,16 g 5'Amino-2',5'-dideoxy-5-n-propyluridin nach Umkristallisation aus einem Gemisch von 2-Methoxyäthanol und Wasser 0,21 g 5'-[2-(2,6-Dichlorphenyl)-acetamido]-2',5'-dideoxy-5-propyluridin vom Schmelzpunkt 297-298° C (Zersetzung).

Das als Ausgangsmaterial verwendete 5'-Amino-2',5'-dideoxy-5-n-propyluridin wurde wie folgt hergestellt:

0,57 g 2'-Deoxy-5-n-propyluridin, 0,6 g Triphenylphosphin und 0,69 g Natriumazid wurden in 20 ml trockenem Trimethylformamid gerührt und im Verlauf von 5 Minuten mit 0,77 g Kohlenstofftetrabromid versetzt. Das Gemisch wurde 72 Stunden bei Raumtemperatur gerührt, danach wurden 20 ml Methanol zugesetzt und das Gemisch eine weitere halbe Stunde gerührt. Das Gemisch wurde eingedampft und der Rückstand mit einem Gemisch von 25 ml Dichlormethan/Methanol (8:2) gerührt. Ein unlöslicher Feststoff wurde abfiltriert und das Filtrat an 200 g Silicagel mit dem gleichen Lösungsmittel chromatographiert. Man erhielt 0,41 g 5'-Azido-2',5'-dideoxy-5-n-propyluridin vom Schmelzpunkt 177-179° C (Zersetzung).

0,38 g 5'-Azido-2',5'-dideoxy-5-n-propyluridin wurden in 100 ml Aethanol gelöst und in Gegenwart von 0,1 g 10% Palladium/Kohle 2 Stunden bei Raumtemperatur und Atmosphärendruck hydriert. Der Katalysator wurde abfiltriert und das Filtrat eingedampft. Man erhielt 5'-Amino-2',5'-dideoxy-5-n-propyluridin als Feststoff, der ohne weitere Reinigung verwendet wurde.

Beispiel 47

In analoger Weise wie in Beispiel 46D, wurde aus 155 mg 2(RS)-(2,4-Dichlorphenoxy)propionsäure und 0,16 g 5'-Amino-2',5'-dideoxy-5-n-propyluridin, 0,20 g 5-[2(RS)-(2,4-Dichlorphenoxy)propionamido]-2',5'-dideoxy -5-propyluridin vom Schmelzpunkt 232-243° C (Zersetzung) erhalten.

Beispiel 48

In analoger Weise wie in Beispiel 16 wurden aus 0,23 g 2,6-Dichlorphenylessigsäure und 0,31 g 5-Acetyl-5'-amino-2',5'-dideoxyuridinhydrochlorid nach Umkristallisation aus einem Gemisch von Aethanol und Petroläther 60 mg 5-Acetyl-5'-[2-(2,6-dichlorphenyl)acetamido] -2',5'-dideoxyuridin vom Schmelzpunkt 210-212° C (Zersetzung) erhalten.

Das als Ausgangsmaterial verwendete 5-Acetyl-5'-amino-2',5'-dideoxyuridinhydrochlorid wurde wie folgt hergestellt:

1,26 g 5-Acetyl-2'-deoxyuridin, 1,35 g Triphenylphosphin, 1,52 g Natriumazid und 1,7 g Kohlenstofftetrabromid wurden in 40 ml Dimethylformamid wie in Beispiel 54 umgesetzt. Das Produkt wurde durch Chromatographie an 400 g Silicagel mit Dichlormethan/Methanol (9:1) gereinigt und lieferte 0,80 g 5-Acetyl-

5′-azido-2′,5′-dideoxyuridin vom Schmelzpunkt 159-162° C (Zersetzung).

0,80 g 5-Acetyl-5′-azido-2′,5′-dideoxyuridin wurden in 100 ml Methanol gelöst und mit 10 ml 0,27M HCl in Methanol versetzt. Danach wurden 0,1 g 5% Palladium/Kohle als Aufschlämmung in Aethanol zugesetzt und das Gemisch 3 Stunden bei Raumptemperatur unter Atmosphärendruck hydriert. Der Katalysator wurde abfiltriert und das Filtrat eingedampft. Man erhielt 0,91 g hygroskopisches 5-Acetyl-5′-amino-2′,5′-dideoxyuridinhydrochlorid vom Schmelzpunkt 73-85° C (Zersetzung).

Beispiel 49

Das folgende Beispiel illustriert eine pharmazeutische Zubereitung mit einer Verbindung der Formel I: Tabletten können die folgenden Inhaltsstoffe enthalten:

| Inhaltsstoff | pro Tablette |
|---|---|
| Verbindung der Formel I | 100 mg |
| Lactose | 70 mg |
| Maisstärke | 70 mg |
| Polyvinylpyrrolidon | 5 mg |
| Magnesiumstearat | 5 mg |
| Tablettengewicht | 250 mg |

**Ansprüche**

**Patentansprüche für folgende Vertragsstaaten: BE, CH, DE, FR, GB, IT, LI, LU, NL, SE**

1. Verbindungen der allgemeinen Formel

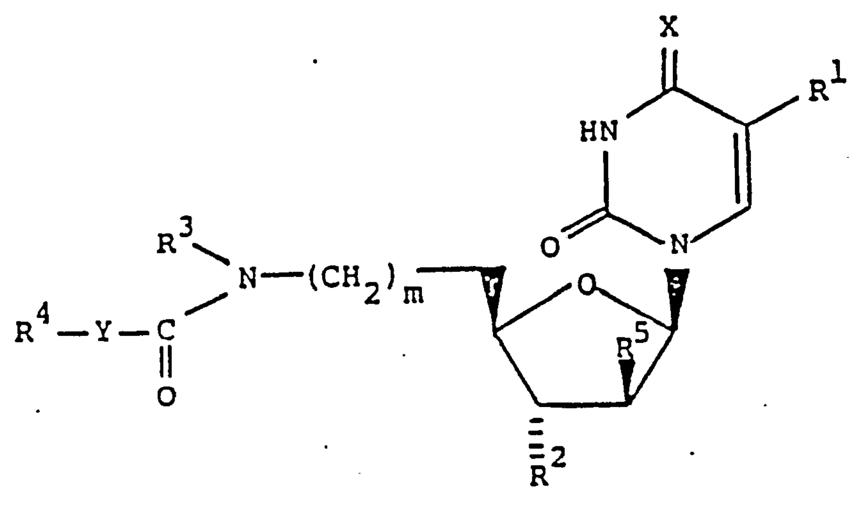

I

worin

- $R^1$ Halogen, $C_{1-4}$-Alkyl, Halo-($C_{1-4}$-alkyl) oder $C_{2-4}$-Alkanoyl,
- $R^2$ Wasserstoff, Hydroxy, $C_{1-4}$-Alkoxy, $C_{1-4}$-Alkylthio oder Phenyl-($C_{1-4}$-alkoxy), oder, falls X Sauerstoff ist, auch Acyloxy;
- $R^3$ Wasserstoff oder $C_{1-4}$-Alkyl,
- $R^4$ eine carbocyclische Gruppe, nämlich eine monocyclische oder polycyclische aromatische Kohlenwasserstoffgruppe, die bis zu 14 Kohlenstoffatome in der cyclischen Struktur enthält und die einen oder mehrere Substituenten aus der Gruppe Halogen, Hydroxy, $C_{1-4}$-Alkyl,

$C_{1-4}$-Alkoxy, Trifluormethyl, Phenyl, $C_{1-4}$-Alkylphenyl, Halophenyl, Nitro, Amino, Acylamino, Benzyloxy und O-Phosphat enthalten kann, oder eine monocyclische oder polycyclische Cycloalkylgruppe, die bis zu 13 Kohlenstoffatome in der cyclischen Struktur enthält und die, falls sie monocyclisch ist, einen oder zwei kondensierte Benzolringe tragen kann, oder eine heterocyclische Gruppe,

$R^5$ Wasserstoff oder Fluor

m 0, 1 oder 2,

X Sauerstoff oder NH,

Y eine direkte Bindung, -CH=CH-, -C≡C- oder eine Gruppe der Formel

$(Z)_n$-A- (a)

worin A eine $C_{1-8}$-Alkylengruppe darstellt, die durch ein oder zwei Phenylgruppen substituiert sein kann,

Z Sauerstoff, Schwefel, SO oder $SO_2$ und n 0 oder 1 bedeuten, mit der Voraussetzung, dass $R^1$ von Jod verschieden ist, wenn $R^2$ Hydroxy oder Benzoyloxy, $R^3$ Wasserstoff, $R^4$ unsubstituiertes Phenyl, $R^5$ Wasserstoff, m 0, X Sauerstoff und Y eine direkte Bindung darstellen,

und deren Tautomeren.

**2.** Verbindungen gemäss Anspruch 1, worin $R^1$ Fluor, Chlor, Brom, $C_{1-4}$-Alkyl oder Halo-($C_{1-4}$-alkyl), $R^4$ eine carbocyclische oder aromatische heterocyclische Gruppe, $R^5$ Wasserstoff, und m O ist.

**3.** Verbindungen gemäss Anspruch 1 oder 2, worin $R^1$ $C_{1-4}$-Alkyl ist.

**4.** Verbindungen gemäss den Ansprüchen 1-3, worin $R^2$ Hydroxy ist.

**5.** Verbindungen gemäss den Ansprüchen 1-4, worin $R^3$ Wasserstoff ist.

**6.** Verbindungen gemäss den Ansprüchen 1-5, worin $R^4$ Phenyl ist, das durch einen oder mehrere Substituenten aus der Gruppe Halogen, $C_{1-4}$-Alkyl, $C_{1-4}$-Alkoxy, Trifluormethyl, Phenyl und Nitro substituiert sein kann.

**7.** Verbindungen gemäss den Ansprüchen 1 oder 3-6, worin m = O ist.

**8.** Verbindungen gemäss den Ansprüchen 1-7, worin X Sauerstoff ist.

**9.** Verbindungen gemäss den Ansprüchen 1-8, worin Y eine Gruppe der Formel (a) ist.

**10.** Verbindungen gemäss den Ansprüchen 1-7, in denen $R^1$ Aethyl, $R^2$ Hydroxy, $R^3$ Wasserstoff, $R^4$ 2-Bromphenyl, 2,6-Dichlorphenyl oder 4-Biphenylyl, m = 0, X Sauerstoff und Y eine Gruppe der Formel (a) ist, in der A-$CH_2$- oder -$CH(CH_3)$- ist und n gleich 0 ist.

**11.** Verbindungen gemäss den Ansprüchen 1-7, in denen $R^1$ Aethyl oder Propyl, $R^2$ Hydroxy, $R^3$ Wasserstoff, $R^4$ 2-Biphenylyl, 2,4-Dichlorphenyl, 2,4,5-Trichlorphenyl, 4-Chlor-2-nitrophenyl oder 2,4-Dichlor-5-methoxyphenyl ist, m gleich 0 ist, X Sauerstoff und Y eine Gruppe der Formel (a) ist, worin A -$CH(CH_3)$- oder -CH(Phenyl)-, Z Sauerstoff und n gleich 1 ist.

**12.** Die Verbindungen gemäss Anspruch 1,
5'-[2-(2-Bromphenyl)acetamido]-2',5'-dideoxy-5-äthyluridin
5'-[2-(2,6-Dichlorphenyl)acetamido]-2',5'-dideoxy-5-äthyluridin,
5'-[2(RS)-(2,4-Dichlorphenoxy)propionamido]-2',5'-dideoxy-5-äthyluridin,
5'-[2-(4-Biphenylyl)acetamido]-2',5'-dideoxy-5-äthyluridin.
2',5'-Dideoxy-5-äthyl-5'-[2(RS)-(2-phenylphenoxy)propionamido]uridin,
1-[5-[2(RS)-(2,4-Dichlorphenoxy)propionamido]-2,5-dideoxy-2-fluor-ß-D-arabinofuranosyl]-5-äthyluracil,
5'-[2(RS)-(2,4,5-Trichlorphenoxy)propionamido]-2',5'-dideoxy-5-äthyluridin,
5'-[2(RS)-(4-Chlor-2-nitrophenoxy)propionamido]-2',5'-dideoxy-5-äthyluridin,
5'-[2(RS)-(2,4-Dichlorphenoxy)-2-phenylacetamido]-2',5'-deoxy-5-äthyluridin,

5'-[2(RS)-(2,4-Dichlor-5-methoxyphenoxy)propionamido]-2',5'-dideoxy-5-äthyluridin und
5'-[2(RS)-(2,4-Dichlorphenoxy)propionamido]-2',5'-dideoxy-5-propyluridin.

13. Die Verbindungen gemäss Anspruch 2,

2',5'-Dideoxy-5-äthyl-5'-(2-phenylacetamido)uridin,
5'-(4-Brombenzamido)-5'-deoxythymidin,
5'-deoxy-5'-(4-nitrobenzamido)thymidin,
5'-Benzamido-5'-deoxythymidin,
5'-Deoxy-5'-(2-fluorbenzamido)thymidin,
5'-Deoxy-5'-(2-nitrobenzamido)thymidin,
5'-[2-(2-Bromphenyl)acetamido)-5'-deoxythymidin,
5'-Deoxy-5'-[2-(4-nitrophenyl)acetamido]thymidin,
5'-Deoxy-5'-(2-phenylacetamido)thymidin,
5'-Deoxy-5'-[2-(4-trifluormäthylphenyl)acetamido]thymidin,
5'-Benzamido-2',5'-dideoxy-5-äthyluridin,
2',5'-Dideoxy-5-äthyl-5'-(2-fluorbenzamido)uridin,
5'-(2-Brombenzamido)-2',5'-dideoxy-5-äthyluridin,
2',5'-Dideoxy-5-äthyl-5'-(4-nitrobenzamido)uridin,
2',5'-Dideoxy-5-äthyl-5'-(2-trifluormethylbenzamido)uridin,
2',5'-Dideoxy-5-äthyl-5'-[2-(2,6-dimethylphenyl)acetamido]uridin,
5'-[2(RS)-(2-Bromphenyl)propionamido]-2',5'-dideoxy-5-äthyluridin,
5'-[2-(2-Chlor-3-nitrophenyl)acetamido]-2',5'-dideoxy-5-äthyluridin,
5'-[2(RS)-(2,6-Dichlorphenyl)propionamido]-2',5'-dideoxy-5-äthyluridin,
2',5'-Dideoxy-5-äthyl-5'-[2-(3,5-dimethylphenyl)acetamido]uridin,
2',5'-Dideoxy-5'-[2-(3,5-dimethoxyphenyl)acetamido]-5-äthyluridin,
2',5'-Dideoxy-5-äthyl-5'-[2-(2,3,5,6-tetramethylphenyl)acetamido]uridin,
2',5'-Dideoxy-5-äthyl-5'-[2-(2-methoxyphenyl)acetamido]uridin,
2',5'-Dideoxy-5-äthyl-5'-[2-(2-nitrophenyl)]acetamidouridin,
5-Brom-5'-[2-(2-bromphenyl)acetamido]-2',5'--dideoxyuridin,
3'-O-Butyryl-5'-[2-(2,6-dichlorphenyl)acetamido]-2',5'-dideoxy-5-äthyluridin,
2',5'-Dideoxy-5'-[2-(2,6-dichlorphenyl)acetamido]-3'-O-(3,3-dimethylbutyryl)-5-äthyluridin,
5'-[2-(2,6-Dichlorphenyl)acetamido]-2',5'-dideoxy-5-äthyl-3'-O-palmitoyluridin,
3'-O-Acetyl-5'-[2-(2,6-dichlorphenyl)acetamido]-2',5'-dideoxy-5-äthyluridin,
3'-O-Benzyl-5'-[2-(2,6-dichlorphenyl)acetamido]-2',5'-dideoxy-5-äthyluridin,
5'-[2-(2,6-Dichlorphenyl)acetamido]-2',5'-dideoxy-3'-O-äthyl-5-äthyluridin,
2',3',5'-Trideoxy-5'-[2-(2,6-dichlorphenyl)acetamido]-5-äthyluridin,
5'-[2-(2-Aminophenyl)acetamido]-2',5'-dideoxy-5-äthyluridin,
5'-[2-(2-Acetamidophenyl)acetamido]-3'-O-acetyl-2',5'-dideoxy-5-äthyluridin,
5'-[2-(2-Acetamidophenyl)acetamido]-2',5'-dideoxy-5-äthyluridin,
2',5'-Dideoxy-5-äthyl-5'-[2-(4-hydroxy-2,6-dimethylphenyl)acetamido]uridin,
5-(2-Chloräthyl)-5'-[2-(2,6-dichlorphenyl)acetamido]-2',5'-dideoxyuridin,
5'-[2-(2-Bromphenyl)-N-methylacetamido]-2',5'-dideoxy-5-äthyluridin,
5'-[2-(2,6-Dichlorphenyl)-N-methylacetamido]-2',5'-dideoxy-5-äthyluridin,
5'-[2(RS)-(2-Bromphenyl)-N-methylpropionamido]-2',5'-dideoxy-5-äthyluridin,
3'-O-Acetyl-5'-[2-(2-bromphenyl)-N-methylacetamido]-2',5'-dideoxy-5-äthyluridin,
2',5'-Dideoxy-5-äthyl-5'-(2-naphthalamido)uridin,
2',5'-Dideoxy-5-äthyl-5'-(2-phenylbenzamido)uridin,
2'-5'-Dideoxy-5-äthyl-5'-(3-phenylpropionamido)uridin,
2',5'-Dideoxy-5-äthyl-5'-(4-phenylbutyramido)uridin,
2',5'-Dideoxy-5'-cinnamamido-5-äthyluridin, 2',5'-Dideoxy-5-äthyl-5'-(3-phenyl-2-propynamido)-uridin,
2',5'-Dideoxy-5-äthyl-5'-[3-(phenylsulphonyl)propion-amido]uridin,
5'-(2-Bromcinnamamido)-2',5'-dideoxy-5-äthyluridin,
2',5'-Dideoxy-5-äthyl-5'-[2-(2-thienyl)acetamido]uridin,
2',5'-Dideoxy-5-äthyl-5'-[2-(3-thienyl)acetamido]uridin,
5'-(1-Adamantylcarboxamido)-2',5'-dideoxy-5-äthyluridin,
2',5'-Dideoxy-5-äthyl-5'-(2-pyridylcarboxamido)uridin,

2',5'-Dideoxy-5-äthyl-5'-(3-pyridylcarboxamido)uridin,
2',5'-Dideoxy-5-äthyl-5'-[2-(phenylsulphonyl)acetamido]uridin,
2',5'-Dideoxy-5-äthyl-5'-(9-fluorenylcarboxamido)uridin,
3'-O-Acetyl-2',5'-dideoxy-5-äthyl-5'-(9-fluorenylcarboxamido)uridin,
3'-O-Butyryl-2',5'-dideoxy-5-äthyl-5'-(9-fluorenylcarboxamido)uridin,
2',5'-Dideoxy-5-äthyl-3'-O-(3,3-dimethylbutyryl)-5'-(9-fluorenylcarboxamido)uridin,
2',5'-Dideoxy-5-äthyl-5'-(9-fluorenylcarboxamido)-3'-O-(hexadecanoyl)uridin,
2',5'-Dideoxy-5-äthyl-3'-O-(9-fluorenylcarbonyl)-5'-(9-fluorenylcarboxamido)uridin,
2',5'-Dideoxy-5-äthyl-5'-(2-phenylbutyramido)uridin,
2',5'-Dideoxy-5-äthyl-5'-(3-phenylbutyramido)uridin,
2',5'-Dideoxy-5-äthyl-5'-(2,2-diphenylacetamido)uridin,
5'-(2-Cyclohexyl-2-phenylacetamido)-2',5'-dideoxy-5-äthyluridin,
2',5'-Dideoxy-5'-äthyl-5'-(triphenylacetamido)uridin,
2',5'-Dideoxy-5-äthyl-5'-(2-methyl-2-phenylpropionamido)uridin,
2',5'-Dideoxy-5-äthyl-5'-[2(RS)-phenylpropionamido)]uridin,
5'-[2(RS)-2,4-Dichlorphenoxy)propionamido]-2',5'-dideoxy-5-äthyluridin,
5'-[2(RS)-2,6-Dichlorphenoxy)propionamido]-2',5'-dideoxy-5-äthyluridin,
5'-[2(RS)-3,5 -Dichlorphenoxy)propionamido]-2',5'-dideoxy-5-äthyluridin,
5'-[2(RS)-2-Chlorphenoxy)propionamido]-2',5'-dideoxy-5-äthyluridin,
5'-[2-(2,6-Dichlorphenoxy)acetamido]-2',5'-dideoxy-5-äthyluridin,
5'-[2-(2,4-Dichlorphenoxy)acetamido]-2',5'-dideoxy-5-äthyluridin,
5'-[4-(2,6-Dichlorphenoxy)butyramido]-2',5'-dideoxy-5-äthyluridin,
5'-[6-(2,6-Dichlorphenoxy)hexanamido]-2',5'-dideoxy-5-äthyluridin,
5'-[5-(2,6-Dichlorphenoxy)valeramido]-2',5'-dideoxy-5-äthyluridin,
5'-[2(RS)-(2-Chlor-4-nitrophenoxy)propionamido]-2',5'-dideoxy-5-äthyluridin,
5'-[2(RS)-(2-Chlor-4-phenylphenoxy)propionamido]-2',5'-dideoxy-5-äthyluridin,
5'-Brom-5'-[2-(2-bromphenyl)acetamido]-2',5'-dideoxycytidine and
5'-[2-(2-Bromphenyl)acetamido]-2',5'-dideoxy-5-äthyluridin.

**14.** Die Verbindungen gemäss Anspruch 1,

5'-[2-(2,6-Dichlorphenyl)acetamido]-5'-deoxythymidin,
5-Brom-5'-[2(RS)-(2,4-dichlorphenoxy)propionamido]-2',5'-dideoxyuridin,
5-Brom-5'-[2-(2,6-dichlorphenyl)acetamido]-2',5'-dideoxyuridin,
5'-Benzamido-5-brom-2',5'-dideoxyuridin,
5'-[2(RS)-(2,4-Dichlorphenoxy)propionamido]-2',5'-dideoxy-5-iodouridin,
5'-[2(RS)-(2,4,5-Trichlorphenoxy)propionamido]-2',5'-dideoxy-5-iodouridin,
5'-[2-(2,6-Dichlorphenyl)acetamido]-2',5'-dideoxy-5-iodouridin,
3'-O-Benzyl-5'-[2(RS)-(2,4-dichlorphenoxy)propionamido]-2',5'-dideoxy-5-äthyluridin,
5'-[2(RS)-(2,4-Dichlorphenoxy)propionamido]-2',5'-dideoxy-3'-O-äthyl-5-äthyluridin,
5'-[2(RS)-(2,4-Dichlorphenoxy)-N-methylpropionamido]-2',5'-dideoxy-5-äthyluridin,
5'-[2(R)-(2,4-Dichlorphenoxy)propionamido]-2',5'-dideoxy-5-äthyluridin,
5'-[2(S)-(2,4-Dichlorphenoxy)propionamido]-2',5'-dideoxy-5-äthyluridin,
5'-[2(RS)-(2,4-Dichlorphenoxy)butyramido]-2',5'-dideoxy-5-äthyluridin,
5'-[2(RS)-(4-Acetamido-2-chlorphenoxy)propionamido]-2',5'-dideoxy-5-äthyluridin,
5'-[2(RS)-(2-Chlor-4-methoxyphenoxy)propionamido]-2',5'-dideoxy-5-äthyluridin,
2',5'-Dideoxy-5-äthyl-5'-[2(RS)-(2-methylbiphenylyloxy)propionamido]uridin,
5'-[2(RS)-(2,4-Dichlorphenoxy)propionamido]-5'-deoxythymidin,
5'-[2-(2,4-Dichlorphenoxy)-2-methylpropionamido]-2',5'-dideoxy-5-äthyluridin,
2',5'-Dideoxy-5-äthyl-5'-[2(RS)-phenoxypropionamido]uridin,
2',5'-Dideoxy-5-äthyl-5'-[2(RS)-(2-fluorphenoxy)propionamido]uridin,
2',5'-Dideoxy-5-äthyl-5'-[2(RS)-(2-trifluormethylphenoxy)propionamido]uridin,
1-[5-[2(RS)-(2,4-Dichlorphenoxy)propionamido]-2,5-dideoxy-2-fluor-ß-D-arabinofuranosyl]thymine,
5'-[2(RS)-(2,4-Dichlorphenylsulphinyl)propionamido]-2',5'-dideoxy-5-äthyluridin,
5'-[2(RS)-(2,4-Dichlorphenylsulphonyl)propionamido]-2',5'-dideoxy-5-äthyluridin,
5'-[2-(2,6-Dichlorphenylacetamido)äthyl]-2',5'-dideoxy-5-äthyluridin, 5'-[2-[2(RS)-(2,4,5-Trichlorphenoxy)propionamido]äthyl]-2',5'-dideoxy-5-äthyluridin,
5'-[2-[2(RS)-(2,4-Dichlorphenoxy)propionamido]äthyl]-2',5'-dideoxy-5-äthyluridin,

5'-[2(RS)-(2,6-Dichlorbenzyl)propionamido]-2',5'-dideoxy-5-äthyluridin,
5'-[2(RS)-(2,4-Dichlorbenzyl)propionamido]-2',5'-dideoxy-5-äthyluridin,
5'-[5-chlor-2,3-Dihydro-2(RS)-benzofuranylcarboxamido]-2',5'-dideoxy-5-äthyluridin,
5-(6-chlor-2H-1-Benzopyran-2-ylcarboxamido)-2',5'-dideoxy-5-äthyluridin
5'-[2-(4-Benzyloxy-2,6-dimethylphenyl)acetamido]-2',5'-dideoxy-5-äthyluridin,
3'-O-Acetyl-5'-[2-(4-benzyloxy-2,6-dimethylphenyl)acetamido]-2',5'-dideoxy-5-äthyluridin,
3'-O-Acetyl-2',5'-dideoxy-5-äthyl-5'-[2-(4-hydroxy-2,6-dimethylphenyl)acetamido]uridin,
2',5'-Dideoxy-5-äthyl-5'-[2-(2,6-dimethyl-4-phosphatophenyl)acetamido]uridin,
5-(2-Chloräthyl)-2',5'-dideoxy-5'-[2-(2,4-dichlorphenoxy)propionamido]uridin,
5'-Benzamido-5-(2-chloräthyl)-2',5'-dideoxyuridin,
5-(2-Chloräthyl)-5'-[2-(2,4,5-trichlorphenoxy)propionamido]-2',5'-dideoxyuridin,
5'-[2-(2,6-dichlorphenyl)acetamido]-2',5'-dideoxy-5-propyluridin,
5'-[2(RS)-(2,4-Dichlorphenoxy)propionamido]-2',5'-dideoxy-5-propyluridin und
5-Acetyl-5'-[2-(2,6-dichlorphenyl)acetamido]-2',5'-dideoxyuridin.

**15.** Die Verbindungen gemäss den Ansprüchen 1-14 zur Verwendung als Heilmittel, insbesondere als Mittel gegen Viren.

**16.** Verfahren zur Herstellung der Verbindungen der allgemeinen Formel I nach Anspruch 1 und deren Tautomeren, dadurch gekennzeichnet, dass man

a) eine Verbindung der allgemeinen Formel

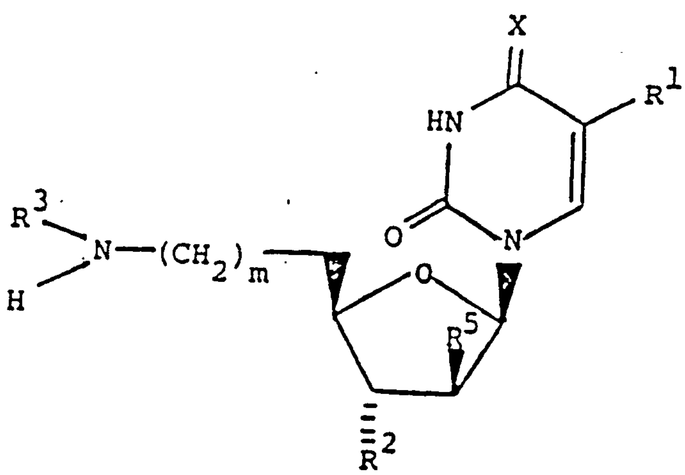

II

worin $R^1$, $R^2$, $R^3$ $R^5$, m und X die obige Bedeutung haben,
oder ein Tautomer davon, in der eine gegebenenfalls anwesende 4-Aminogruppe gewünschtenfalls geschützt ist, mit einem reaktionsfähigen Derivat einer Säure der allgemeinen Formel

$R^4$-Y-COOH III

worin $R^4$ und Y die obige Bedeutung haben,
umsetzt und eine im Produkt gegebenenfalls anwesende Schutzgruppe abspaltet oder

b) zur Herstellung von Verbindungen der Formel I oder einem Tautomeren davon, in denen X Sauerstoff und $R^2$ Acyloxy darstellt. eine Verbindung der Formel I oder ein Tautomer davon, in der X Sauerstoff und $R^2$ Hydroxy ist. acyliert, oder

c) zur Herstellung einer Verbindung der Formel 1 oder einem Tautomeren davon, in der X Sauerstoff und $R^2$ Hydroxy ist, eine Verbindung der Formel I oder ein Tautomer davon, in der X Sauerstoff und $R^2$ Acyloxy ist, deacyliert, oder

d) zur Herstellung einer Verbindung der Formel I oder einem Tautomeren davon, in der Y eine Gruppe der Formel (a), Z SO oder $SO_2$ und n 1 ist, eine Verbindung der Formel I oder ein Tautomer davon, in der Y eine Gruppe der Formel (a), Z Schwefel und n 1 ist, oxydiert, und

e) gewünschtenfalls einen in $R^4$ anwesenden reaktionsfähigen Substituenten funktionell abwandelt.

**17.** Pharmazeutische, insbesondere anti-viral wirksame Präparate, enthaltend eine Verbindung gemäss den

Ansprüchen 1-14 und ein pharmazeutisches Trägermaterial.

18. Verwendung einer Verbindung gemäss den Ansprüchen 1-14 zur Herstellung von Arzneimitteln zur Kontrolle oder Prophylaxe viraler Infektionen.

**Patentansprüche für folgende Vertragsstaaten: AT, ES, GR**

1. Verfahren zur Herstellung von Verbindungen der allgemeinen Formel

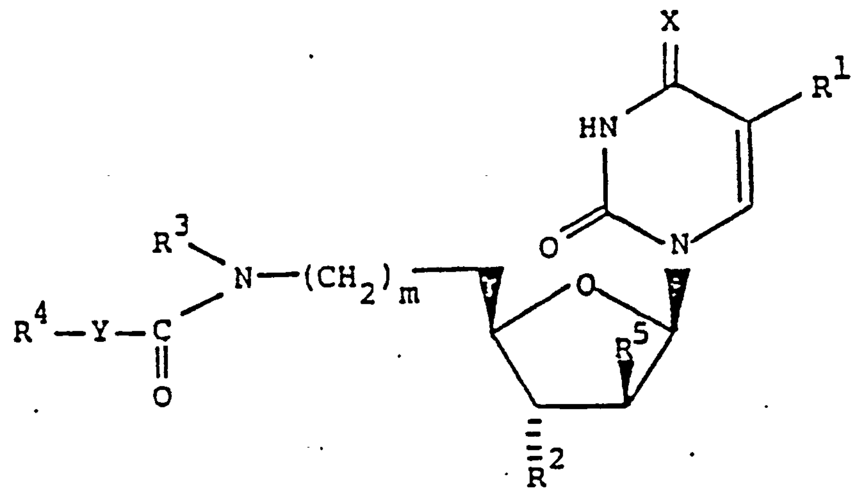

I

worin
- $R^1$ Halogen, $C_{1-4}$-Alkyl, Halo-($C_{1-4}$-alkyl) oder $C_{2-4}$-Alkanoyl,
- $R^2$ Wasserstoff, Hydroxy, $C_{1-4}$-Alkoxy, $C_{1-4}$-Alkylthio oder Phenyl-($C_{1-4}$-alkoxy), oder, falls X Sauerstoff ist, auch Acyloxy;
- $R^3$ Wasserstoff oder $C_{1-4}$-Alkyl,
- $R^4$ eine carbocyclische Gruppe, nämlich eine monocyclische oder polycyclische aromatische Kohlenwasserstoffgruppe, die bis zu 14 Kohlenstoffatome in der cyclischen Struktur enthält und die einen oder mehrere Substituenten aus der Gruppe Halogen, Hydroxy, $C_{1-4}$-Alkyl, $C_{1-4}$-Alkoxy, Trifluormethyl, Phenyl, $C_{1-4}$-Alkylphenyl, Halophenyl, Nitro, Amino, Acylamino, Benzyloxy und O-Phosphat enthalten kann, oder eine monocyclische oder polycyclische Cycloalkylgruppe, die bis zu 13 Kohlenstoffatome in der cyclischen Struktur enthält und die, falls sie monocyclisch ist, einen oder zwei kondensierte Benzolringe tragen kann, oder eine heterocyclische Gruppe,
- $R^5$ Wasserstoff oder Fluor
- m 0, 1 oder 2,
- X Sauerstoff oder NH,
- Y eine direkte Bindung, -CH = CH-, -C≡C- oder eine Gruppe der Formel

  $(Z)_n$-A- (a)

  worin A eine $C_{1-8}$-Alkylengruppe darstellt, die durch ein oder zwei Phenylgruppen substituiert sein kann,
- Z Sauerstoff, Schwefel, SO oder $SO_2$ und n 0 oder 1 bedeuten, mit der Voraussetzung, dass $R^1$ von Jod verschieden ist, wenn $R^2$ Hydroxy oder Benzoyloxy, $R^3$ Wasserstoff, $R^4$ unsubstituiertes Phenyl, $R^5$ Wasserstoff, m 0, X Sauerstoff und Y eine direkte Bindung darstellen,

und deren Tautomeren, dadurch gekennzeichnet, dass man

a) eine Verbindung der allgemeinen Formel

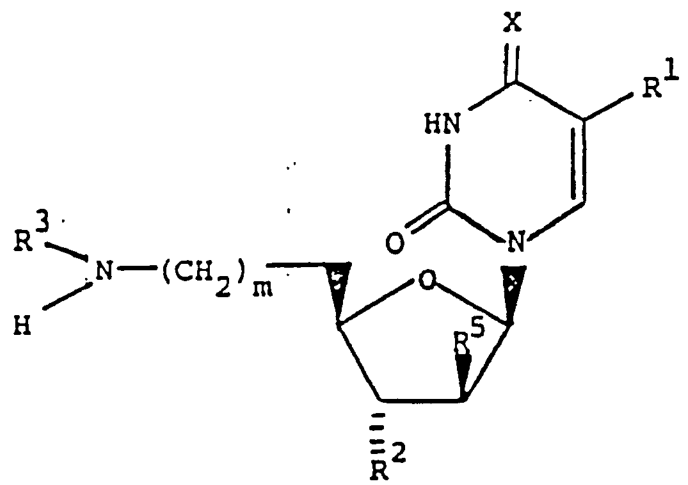

II

worin $R^1$, $R^2$, $R^3$ $R^5$, m und X die obige Bedeutung haben,

oder ein Tautomer davon, in der eine gegebenenfalls anwesende 4-Aminogruppe gewünschtenfalls geschützt ist, mit einem reaktionsfähigen Derivat einer Säure der allgemeinen Formel

$R^4$-Y-COOH III

worin $R^4$ und Y die obige Bedeutung haben,

umsetzt und eine im Produkt gegebenenfalls anwesende Schutzgruppe abspaltet oder

b) zur Herstellung von Verbindungen der Formel I oder einem Tautomeren davon, in denen X Sauerstoff und $R^2$ Acyloxy darstellt, eine Verbindung der Formel I oder ein Tautomer davon, in der X Sauerstoff und $R^2$ Hydroxy ist, acyliert, oder

c) zur Herstellung einer Verbindung der Formel I oder einem Tautomeren davon, in der X Sauerstoff und $R^2$ Hydroxy ist, eine Verbindung der Formel I oder ein Tautomer davon, in der X Sauerstoff und $R^2$ Acyloxy ist, deacyliert, oder

d) zur Herstellung einer Verbindung der Formel I oder einem Tautomeren davon, in der Y eine Gruppe der Formel (a), Z SO oder $SO_2$ und n 1 ist, eine Verbindung der Formel I oder ein Tautomer davon, in der Y eine Gruppe der Formel (a), Z Schwefel und n 1 ist, oxydiert, und

e) gewünschtenfalls einen in $R^4$ anwesenden reaktionsfähigen Substituenten funktionell abwandelt.

2. Verfahren gemäss Anspruch 1, worin $R^1$ Fluor, Chlor, Brom, $C_{1-4}$-Alkyl oder Halo-($C_{1-4}$-alkyl), $R^4$ eine carbocyclische oder aromatische heterocyclische Gruppe, $R^5$ Wasserstoff, und m O ist.

3. Verfahren gemäss Anspruch 1 oder 2, worin $R^1$ $C_{1-4}$-Alkyl ist.

4. Verfahren gemäss den Ansprüchen 1-3, worin $R^2$ Hydroxy ist.

5. Verfahren gemäss den Ansprüchen 1-4, worin $R^3$ Wasserstoff ist.

6. Verfahren gemäss den Ansprüchen 1-5, worin $R^4$ Phenyl ist, das durch einen oder mehrere Substituenten aus der Gruppe Halogen, $C_{1-4}$-Alkyl, $C_{1-4}$-Alkoxy, Trifluormethyl, Phenyl und Nitro substituiert sein kann.

7. Verfahren gemäss den Ansprüchen 1 oder 3-6, worin m = O ist.

8. Verfahren gemäss den Ansprüchen 1-7, worin X Sauerstoff ist.

9. Verfahren gemäss den Ansprüchen 1-8, worin Y eine Gruppe der Formel (a) ist.

10. Verfahren gemäss den Ansprüchen 1-7, in denen $R^1$ Aethyl, $R^2$ Hydroxy, $R^3$ Wasserstoff, $R^4$ 2-Bromphenyl, 2,6-Dichlorphenyl oder 4-Biphenylyl, m = 0, X Sauerstoff und Y eine Gruppe der Formel (a) ist, in der A $-CH_2-$ oder$-CH(CH_3)-$ ist und n gleich 0 ist.

11. Verfahren gemäss den Ansprüchen 1-7, in denen $R^1$ Aethyl oder Propyl, $R^2$ Hydroxy, $R^3$ Wasserstoff, $R^4$ 2-Biphenylyl, 2,4-Dichlorphenyl, 2,4,5-Trichlorphenyl, 4-Chlor-2-nitrophenyl oder 2,4-Dichlor-5-methoxyphenyl ist, m gleich 0 ist, X Sauerstoff und Y eine Gruppe der Formel (a) ist, worin A $-CH(CH_3)-$ oder -CH(Phenyl)-, Z Sauerstoff und n gleich 1 ist.

12. Verfahren gemäss Anspruch 1 zur Herstellung der Verbindungen,

5'-[2-(2-Bromphenyl)acetamido]-2',5'-dideoxy-5-äthyluridin
5'-[2-(2,6-Dichlorphenyl)acetamido]-2',5'-dideoxy-5-äthyluridin
5'-[2(RS)-(2,4-Dichlorphenoxy)propionamido]-2',5'-dideoxy-5-äthyluridin
5'-[2-(4-Biphenylyl)acetamido]-2',5'-dideoxy-5-äthyluridin
2',5'-Dideoxy-5-äthyl-5'-[2(RS)-(2-phenylphenoxy)propionamido]uridin
1-[5-[2(RS)-(2,4-Dichlorphenoxy)propionamido]-2,5-dideoxy-2-fluor-ß-D-arabinofuranosyl]-5-äthyluracil,
5'-[2(RS)-(2,4,5-Trichlorphenoxy)propionamido]-2',5'-dideoxy-5-äthyluridin,
5'-[2(RS)-(4-Chlor-2-nitrophenoxy)propionamido]-2',5'-dideoxy-5-äthyluridin,
5'-[2-(RS)-(2,4-Dichlorphenoxy)-2-phenylacetamido]-2',5'-deoxy-5-äthyluridin,
5'-[2(RS)-(2,4-Dichlor-5-methoxyphenoxy)propionamido]-2',5'-dideoxy-5-äthyluridin und
5'-[2(RS)-(2,4-Dichlorphenoxy)propionamido]-2',5'-dideoxy-5-propyluridin.

13. Verfahren gemäss Anspruch 2 zur Herstellung der Verbindungen

2',5'-Dideoxy-5-äthyl-5'-(2-phenylacetamido)uridin,
5'-(4-Brombenzamido)-5'-deoxythymidin,
5'-Deoxy-5'-(4-nitrobenzamido)thymidin,
5'-Benzamido-5'-deoxythymidin,
5'-Deoxy-5'-(2-fluorbenzamido)thymidin,
5'-Deoxy-5'-(2-nitrobenzamido)thymidin,
5'-[2-(2-Bromphenyl)acetamido]-5'-deoxythymidin,
5'-Deoxy-5'-(2-(4-nitrophenyl)acetamido]thymidin,
5'-Deoxy-5'-(2-phenylacetamido)thymidin,
5'-Deoxy-5'-[2-(4-trifluormäthylphenyl)acetamido]thymidin,
5'-Benzamido-2',5'-dideoxy-5-äthyluridin,
2',5'-Dideoxy-5-äthyl-5'-(2-fluorbenzamido)uridin,
5'-(2-Brombenzamido)-2',5'-dideoxy-5-äthyluridin,
2',5'-Dideoxy-5-äthyl-5'-(4-nitrobenzamido)uridin,
2',5'-Dideoxy-5-äthyl-5'-(2-trifluormethylbenzamido)uridin,
2',5'-Dideoxy-5-äthyl-5'-[2-(2,6-dimethylphenyl)acetamido]uridin,
5'-[2(RS)-(2-Bromphenyl)propionamido]-2',5'-dideoxy-5-äthyluridin,
5'-[2-(2-Chlor-3-nitrophenyl)acetamido]-2',5'-dideoxy-5-äthyluridin,
5'-[2(RS)-(2,6-Dichlorphenyl)propionamido]-2',5'-dideoxy-5-äthyluridin,
2',5'-Dideoxy-5-äthyl-5'-[2-(3,5-dimethylphenyl)acetamido]uridin,
2',5'-Dideoxy-5'-[2-(3,5-dimethoxyphenyl)acetamido]-5-äthyluridin,
2',5'-Dideoxy-5-äthyl-5'-[2-(2,3,5,6-tetramethylphenyl)acetamido]uridin,
2',5'-Dideoxy-5-äthyl-5'-[2-(2-methoxyphenyl)acetamido]uridin,
2',5'-Dideoxy-5-äthyl-5'-[2-(2-nitrophenyl)]acetamidouridin,
5-Brom-5'-[2-(2-bromphenyl)acetamido]-2',5'--dideoxyuridin,
3'-O-Butyryl-5'-[2-(2,6-dichlorphenyl)acetamido]-2',5'-dideoxy-5-äthyluridin,
2',5'-Dideoxy-5'-[2-(2,6-dichlorphenyl)acetamido]-3'-O-(3,3-dimethylbutyryl)-5-äthyluridin,
5'-[2-(2,6-Dichlorphenyl)acetamido]-2',5'-dideoxy-5-äthyl-3'-O-palmitoyluridin,
3'-O-Acetyl-5'-[2-(2,6-dichlorphenyl)acetamido]-2',5'-dideoxy-5-äthyluridin,
3'-O-Benzyl-5'-[2-(2,6-dichlorphenyl)acetamido]-2',5'-dideoxy-5-äthyluridin,
5'-[2-(2,6-Dichlorphenyl)acetamido]-2',5'-dideoxy-3'-O-äthyl-5-äthyluridin,
2',3',5'-Trideoxy-5'-[2-(2,6-dichlorphenyl)acetamido]-5-äthyluridin,
5'-[2-(2-Aminophenyl)acetamido]-2',5'-dideoxy-5-äthyluridin,
5'-[2-(2-Acetamidophenyl)acetamido]-3'-O-acetyl-2',5'-dideoxy-5-äthyluridin,
5'-[2-(2-Acetamidophenyl)acetamido]-2',5'-dideoxy-5-äthyluridin,
2',5'-Dideoxy-5-äthyl-5'-[2-(4-hydroxy-2,6-dimethylphenyl)acetamido]uridin,

5-(2-Chloräthyl)-5′-[2-(2,6-dichlorphenyl)acetamido]-2′,5′-dideoxyuridin,
5′-[2-(2-Bromphenyl)-N-methylacetamido]-2′,5′-dideoxy-5-äthyluridin,
5′-[2-(2,6-Dichlorphenyl)-N-methylacetamido]-2′,5′-dideoxy-5-äthyluridin,
5′-[2-(RS)-(2-Bromphenyl)-N-methylpropionamido]-2′,5′-dideoxy-5-äthyluridin,
3′-O-Acetyl-5′-[2-(2-bromphenyl)-N-methylacetamido]-2′,5′-dideoxy-5-äthyluridin,
2′,5′-Dideoxy-5-äthyl-5′-(2-naphthalamido)uridin,
2′,5′-Dideoxy-5-äthyl-5′-(2-phenylbenzamido)uridin
2′-5′-Dideoxy-5-äthyl-5′-(3-phenylpropionamido)uridin,
2′,5′-Dideoxy-5-äthyl-5′-(4-phenylbutyramido)uridin,
2′,5′-Dideoxy-5′-cinnamamido-5-äthyluridin,
2′,5′-Dideoxy-5-äthyl-5′-(3-phenyl-2-propynamido)uridin,
2′,5′-Dideoxy-5-äthyl-5′-[3-(phenylsulphonyl)propionamido]uridin,
5′-(2-Bromcinnamamido)-2′,5′-dideoxy-5-äthyluridin,
2′,5′-Dideoxy-5-äthyl-5′-[2-(2-thienyl)acetamido]uridin,
2′,5′-Dideoxy-5-äthyl-5′-[2-(3-thienyl)acetamido]uridin,
5′-(1-Adamantylcarboxamido)-2′,5′-dideoxy-5-äthyluridin,
2′,5′-Dideoxy-5-äthyl-5′-(2-pyridylcarboxamido)uridin,
2′,5′-Dideoxy-5-äthyl-5′-(3-pyridylcarboxamido)uridin,
2′,5′-Dideoxy-5-äthyl-5′-[2-phenylsulphonyl)acetamido]uridin,
2′,5′-Dideoxy-5-äthyl-5′-(9-fluorenylcarboxamido)uridin,
3′-O-Acetyl-2′,5′-dideoxy-5-äthyl-5′-(9-fluorenylcarboxamido)uridin,
3′-O-Butyryl-2′,5′-dideoxy-5-äthyl-5′-(9-fluorenylcarboxamido)uridin,
2′,5′-Dideoxy-5-äthyl-3′-O-(3,3-dimethylbutyryl)-5′-(9-fluorenylcarboxamido)uridin,
2′,5′-Dideoxy-5-äthyl-5′-(9-fluorenylcarboxamido)-3′-O-(hexadecanoyl)uridin,
2′,5′-Dideoxy-5-äthyl-3′-O-(9-fluorenylcarbonyl)-5′-(9-fluorenylcarboxamido)uridin,
2′,5′-Dideoxy-5-äthyl-5′-(2-phenylbutyramido)uridin,
2′,5′-Dideoxy-5-äthyl-5′-(3-phenylbutyramido)uridin,
2′,5′-Dideoxy-5-äthyl-5′-(2,2-diphenylacetamido)uridin,
5′-(2-Cyclohexyl-2-phenylacetamido)-2′,5′-dideoxy-5-äthyluridin,
2′,5′-Dideoxy-5′-äthyl-5′-(triphenylacetamido)uridin,
2′,5′-Dideoxy-5-äthyl-5′-(2-methyl-2-phenylpropionamido)uridin,
2′,5′-Dideoxy-5-äthyl-5′-[2(RS)-phenylpropionamido)]uridin,
5′-[2(RS)-(2,4-Dichlorphenoxy)propionamido]-2′,5′-dideoxy-5-äthyluridin,
5′-[2(RS)-(2,6-Dichlorphenoxy)propionamido]-2′,5′-dideoxy-5-äthyluridin,
5′-[2(RS)-(3,5-Dichlorphenoxy)propionamido]-2′,5′-dideoxy-5-äthyluridin,
5′-[2(RS)-(2-Chlorphenoxy)propionamido]-2′,5′-dideoxy-5-äthyluridin,
5′-[2-(2,6-Dichlorphenoxy)acetamido]-2′,5′-dideoxy-5-äthyluridin,
5′-[2-(2,4-Dichlorphenoxy)acetamido]-2′,5′-dideoxy-5-äthyluridin,
5′-[4-(2,6-Dichlorphenoxy)butyramido]-2′,5′-dideoxy-5-äthyluridin,
5′-[6-(2,6-Dichlorphenoxy)hexanamido]-2′,5′-dideoxy-5-äthyluridin,
5′-[5-(2,6-Dichlorphenoxy)valeramido]-2′,5′-dideoxy-5-äthyluridin,
5′-[2(RS)-(2-Chlor-4-nitrophenoxy)propionamido]-2′,5′-dideoxy-5-äthyluridin,
5′-[2(RS)-(2-Chlor-4-phenylphenoxy)propionamido]-2′,5′-dideoxy-5-äthyluridin,
5′-Brom-5′-[2-(2-bromphenyl)acetamido]-2′,5′-dideoxycytidine and
5′-[2-(2-Bromphenyl)acetamido]-2′,5′-dideoxy-5-äthylcytidin.

**14.** Verfahren gemäss Anspruch 1 zur Herstellung der Verbindungen

5′-[2-(2,6-Dichlorphenyl)acetamido]-5′-deoxythymidin,
5-Brom-5′-[2(RS)-(2,4-dichlorphenoxy)propionamido]-2′,5′-dideoxyuridin,
5-Brom-5′-[2-(2,6-dichlorphenyl)acetamido]-2′,5′-dideoxyuridin,
5′-Benzamido-5-brom-2′,5′-dideoxyuridin,
5′-[2(RS)-(2,4-Dichlorphenoxy)propionamido]-2′,5′-dideoxy-5-iodouridin,
5′-[2(RS)-(2,4,5-Trichlorphenoxy)propionamido]-2′,5′-didoexy-5-iodouridin
5′-[2-(2,6-Dichlorphenyl)acetamido]-2′,5′-dideoxy-5-iodouridin,
3′-O-Benzyl-5′-[2(RS)-(2,4-dichlorphenoxy)propionamido]-2′,5′-dideoxy-5-äthyluridin,
5′-[2(RS)-(2,4-Dichlorphenoxy)propionamido]-2′,5′-dideoxy-3′-O-äthyl-5-äthyluridin,
5′-[2(RS)-(2,4-Dichlorphenoxy)-N-methylpropionamido]-2′,5′-dideoxy-5-äthyluridin,

5'-[2(R)-(2,4-Dichlorphenoxy)propionamido]-2',5'-dideoxy-5-äthyluridin,
5'-[2(S)-(2,4-Dichlorphenoxy)propionamido]-2',5'-dideoxy-5-äthyluridin,
5'-[2(RS)-(2,4-Dichlorphenoxy)butyramido]-2',5'-dideoxy-5-äthyluridin,
5'-[2(RS)-(4-Acetamido-2-chlorphenoxy)propionamido]-2',5'-dideoxy-5-äthyluridin,
5'-[2(RS)-(2-Chlor-4-methoxyphenoxy)propionamido]-2',5'-dideoxy-5-äthyluridin,
2',5'-Dideoxy-5-äthyl-5'-[2(RS)-(2-methylbiphenylyloxy)propionamido]uridin,
5'-[2(RS)-(2,4-Dichlorphenoxy)propionamido]-5'-deoxythymidin,
5'-[2-(2,4-Dichlorphenoxy)-2-methylpropionamido]-2',5'-dideoxy-5-äthyluridin,
2',5'-Dideoxy-5-äthyl-5'-[2(RS)-phenoxypropionamido]uridin,
2',5'-Dideoxy-5-äthyl-5'-[2(RS)-(2-fluorphenoxy)propionamido]uridin,
2',5'-Dideoxy-5-äthyl-5'-[2(RS)-(2-trifluormethylphenoxy)propionamido]uridin,
1-[5-[2(RS)-(2,4-Dichlorphenoxy)propionamido]-2,5-dideoxy-2-fluor-ß-D-arabinofuranosyl]thymine,
5'-[2(RS)-(2,4-Dichlorphenylsulphinyl)propionamido]-2',5'-dideoxy-5-äthyluridin,
5'-[2(RS)-(2,4-Dichlorphenylsulphonyl)propionamido]-2',5'-dideoxy-5-äthyluridin,
5'-[2-(2,6-Dichlorphenylacetamido)äthyl]-2',5'-dideoxy-5-äthyluridin,
5'-[2-[2(RS)-(2,4,5-Trichlorphenoxy)propionamido]äthyl]-2',5'-dideoxy-5-äthyluridin,
5'-[2-[2(RS)-(2,4-Dichlorphenoxy)propionamido]äthyl]-2',5'-dideoxy-5-äthyluridin,
5'-[2(RS)-(2,6-Dichlorbenzyl)propionamido]-2',5'-dideoxy-5-äthyluridin,
5'-[2(RS)-(2,4-Dichlorbenzyl)propionamido]-2',5'-dideoxy-5-äthyluridin,
5'-[5-chlor-2,3-Dihydro-2(RS)-benzofuranylcarboxamido]-2',5'-dideoxy-5-äthyluridin,
5-(6-chlor-2H-1-Benzopyran-2-ylcarboxamido)-2',5'-dideoxy-5-äthyluridin
5'-[2-(4-Benzyloxy-2,6-dimethylphenyl)acetamido]-2',5'-dideoxy-5-äthyluridin,
3'-O-Acetyl-5'-[2-(4-benzyloxy-2,6-dimethylphenyl)acetamido]-2',5'-dideoxy-5-äthyluridin,
3'-O-Acetyl-2',5'-dideoxy-5-äthyl-5'-[2-(4-hydroxy-2,6-dimethylphenyl)acetamido]uridin,
2',5'-Dideoxy-5-äthyl-5'-[2-(2,6-dimethyl-4-phosphatophenyl)acetamido]uridin,
5-(2-Chloräthyl)-2',5'-dideoxy-5'-[2-(2,4-dichlorphenoxy)propionamido]uridin,
5'-Benzamido-5-(2-chloräthyl)-2',5'-dideoxyuridin,
5-(2-Chloräthyl)-5'-[2-(2,4,5-trichlorphenoxy)propionamido]-2',5'-dideoxyuridin,
5'-[2-(2,6-Dichlorphenyl)acetamido]-2',5'-dideoxy-5-propyluridin,
5'-[2(RS)-(2,4-dichlorphenoxy)propionamido]-2',5'-dideoxy-5-propyluridin und
5-Acetyl-5'-[2-(2,6-dichlorphenyl)acetamido]-2',5'-dideoxyuridin.

**15.** Verfahren zur Herstellung pharmazeutischer Präparate, insbesondere solcher mit anti-viraler Wirksamkeit, dadurch gekennzeichnet, dass man eine Verbindung der Formel I oder ein Tautomer davon in eine galenische Verabreichungsform bringt.

**16.** Verwendung einer Verbindung der Formel I oder eines Tautomeren davon zur Herstellung von Arzneimitteln zur Kontrolle oder Prophylaxe viraler Infektionen.

## Claims

**Claims for the following Contracting States: BE, CH, DE, FR, GB, IT, LI, LU, NL, SE**

**1.** Compounds of the general formula

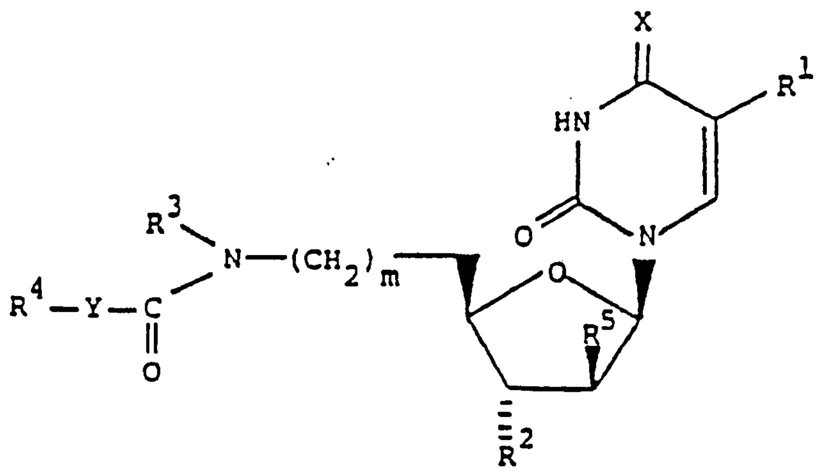

I

wherein

- $R^1$ signifies halogen, $C_{1-4}$-alkyl, halo-($C_{1-4}$-alkyl) or $C_{1-4}$-alkanoyl,
- $R^2$ signifies hydrogen, hydroxy. $C_{1-4}$ -alkoxy, $C_{1-4}$-alkylthio or phenyl-($C_{1-4}$-alkoxy), or. when X is oxygen, also acyloxy;
- $R^3$ signifies hydrogen or $C_{1-4}$-alkyl.
- $R^4$ signifies a carbocyclic group, namely a monocyclic or polycyclic aromatic hydrocarbon group, which contains up to 14 carbon atoms in the cyclic structure and which can contain one or more substituents from the group halogen. hydroxy, $C_{1-4}$ -alkyl, $C_{1-4}$-alkoxy, trifluoromethyl, phenyl, $C_{1-4}$-alkylphenyl, halophenyl, nitro, amino, acylamino, benzyloxy and O-phosphate, or a monocyclic or polycyclic cycloalkyl group, which contains up to 13 carbon atoms in the cyclic structure and which, when it is monocyclic, can carry one or two condensed benzene rings, or a heterocyclic group,
- $R^5$ signifies hydrogen or fluorine,
- m signifies 0, 1 or 2,
- X signifies oxygen or NH,
- Y signifies a direct bond, -CH = CH-, -C≡C- or a group of the formula

  $-(Z)_n-A-$ (a)

  in which A represents a $C_{1-8}$-alkylene group which can be substituted by one or two phenyl groups.
- Z signifies oxygen, sulphur, SO or $SO_2$ and n signifies O or 1, with the proviso that $R^1$ is different from iodine when $R^2$ represents hydroxy or benzoyloxy, $R^3$ represents hydrogen, $R^4$ represents unsubstituted phenyl, $R^5$ represents hydrogen, m represents O, X represents oxygen and Y represents a direct bond, and tautomers thereof.

2. Compounds according to claim 1, wherein $R^1$ is fluorine, chlorine, bromine, $C_{1-4}$-alkyl or halo-($C_{1-4}$-alkyl), $R^4$ is a carbocyclic or aromatic heterocyclic group, $R^5$ is hydrogen and m is O.

3. Compounds according to claim 1 or 2, wherein $R^1$ is $C_{1-4}$-alkyl.

4. Compounds according to claims 1-3, wherein $R^2$ is hydroxy.

5. Compounds according to claims 1-4, wherein $R^3$ is hydrogen.

6. Compounds according to claims 1-5. wherein $R^4$ is phenyl which can be substituted by one or more substituents from the group halogen, $C_{1-4}$-alkyl, $C_{1-4}$-alkoxy, trifluoromethyl, phenyl and nitro.

7. Compounds according to claims 1 or 3-6, wherein m = O.

8. Compounds according to claims 1-7. wherein X is oxygen.

9. Compounds according to claims 1-8, wherein Y is a group of formula (a).

10. Compounds according to claims 1-7, in which $R^1$ is ethyl, $R^2$ is hydroxy, $R^3$ is a hydrogen, $R^4$ is 2-bromophenyl, 2,6-dichlorophenyl or 4-biphenylyl, m = O, X is oxygen and Y is a group of formula (a) in which A is-$CH_2$ or -$CH(CH_3)$- and n is equal to O.

11. Compounds according to claims 1-7, in which $R^1$ is ethyl or propyl, $R^2$ is hydroxy, $R^3$ is hydrogen, $R^4$ is 2-biphenylyl, 2,4-dichlorophenyl, 2.4,5-trichlorophenyl, 4-chloro-2-nitrophenyl or 2,4-dichloro-5-methoxyphenyl, m is equal to O. X is oxygen and Y is a group of formula (a) in which A is -$CH(CH_3)$- or -CH(phenyl)-, Z is oxygen and n is equal to 1.

12. The compounds according to claim 1,

5'-[2-(2-bromophenyl)acetamido]-2',5'-dideoxy-5-ethyl-uridine,
5'-[2-(2,6-dichlorophenyl)acetamido]-2',5'-dideoxy-5-ethyluridine,
5'-[2(RS)-(2,4-dichlorophenoxy)propionamido]-2',5'-dideoxy-5-ethyluridine,
5'-[2-(4-biphenylyl)acetamido]-2',5'-dideoxy-5-ethyl-uridine,
2',5'-dideoxy-5-ethyl-5'-[2(RS)-(2-phenylphenoxy)-propionamido]uridine,
1-[5-[2(RS)-(2,4-dichlorophenoxy)propionamido]-2,5-dideoxy-2-fluoro-ß-D-arabinofuranosyl]-5-ethyluracil,
5'-[2(RS)-(2.4,5-trichlorophenoxy)propionamido]-2',5'-dideoxy-5-ethyluridine,
5'[2(RS)-(4-chloro-2-nitrophenoxy)propionamido]-2',5'-dideoxy-5-ethyluridine,
5'-[2(RS)(2,4-dichlorophenoxy)-2-phenylacetamido]-2',5'-deoxy-5-ethyluridine,
5'-[2(RS)-(2,4-dichloro-5-methoxyphenoxy)propionamido]-2',5'-dideoxy-5-ethyluridine,
5'-[2(RS)-(2,4-dichlorophenoxy)propionamido]-2',5'-dideoxy-5-propyluridine.

13. The compounds according to claim 2,

2', 5'-Dideoxy-5-ethyl-5'-(2-phenylacetamido]uridine,
5'-(4-bromobenzamido)-5'-deoxythymidine,
5'-deoxy-5'-(4-nitrobenzamido)thymidine,
5'-benzamido-5'-deoxythymidine,
5'-deoxy-5'-(2-fluorobenzamido)thymidine,
5'-deoxy-5'-(2-nitrobenzamido)thymidine,
5'-[2-(2-bromophenyl)acetamido]-5'-deoxythymidine,
5'-deoxy-5'-[2-(4-nitrophenyl)acetamido]thymidine,
5'-deoxy-5'-(2-phenylacetamido)thymidine,
5'-deoxy-5'-[2-(4-trifluoromethylphenyl)acetamido]-thymidine,
5'-benzamido-2',5'-dideoxy-5-ethyluridine,
2' ,5'-dideoxy-5-ethyl-5'-(2-fluorobenzamido)uridine,
5'-(2-bromobenzamido)-2' ,5'-dideoxy-5-ethyluridine,
2' ,5'-dideoxy-5-ethyl-5'-(4-nitrobenzamido)uridine,
2',5'-dideoxy-5-ethyl-5'-(2-trifluoromethylbenzamido)uridine,
2',5'-dideoxy-5-ethyl-5'-[2-(2,6-dimethylphenyl)-acetamido]uridine.
5'-[2(RS)-(2-bromophenyl)propionamido]-2',5'-dideoxy-5-ethyluridine,
5'-[2-(2-chloro-3-nitrophenyl)acetamido]-2',5'-dideoxy-5-ethyluridine,
5'-[2(RS)-(2,6-dichlorophenyl)propionamido]-2',5'-dideoxy-5-ethyluridine,
2',5'-dideoxy-5-ethyl-5'-[2-(3,5-dimethylphenyl)acetamido]uridine,
2',5'-dideoxy-5'-[2-(3,5-dimethoxyphenyl)acetamido]-5-ethyluridine,
2',5'-dideoxy-5-ethyl-5'-[2-(2,3,5,6-tetramethylphenyl)acetamido]uridine,
2',5'-dideoxy-5-ethyl-5'-[2-(2-methoxyphenyl)acetamido]uridine,
2',5'-dideoxy-5-ethyl-5'-[2-(2-nitrophenyl)acetamido]-uridine,
5-bromo-5'-[2-(2-bromophenyl)acetamido]-2',5'-dideoxyuridine,
3'-O-butyryl-5'-[2-(2,6-dichlorophenyl)acetamido]-2',5'-dideoxy-5-ethyluridine,
2',5'-dideoxy-5'-[2-(2,6-dichlorophenyl)acetamido]-3'-O-(3,3-dimethylbutyryl)-5-ethyluridine,
5'-[2-(2,6-dichlorophenyl)acetamido]-2',5'-dideoxy-5-ethyl-3'-O-palmitoyluridine,

3'-O-acetyl-5'-[2-(2,6-dichlorophenyl)acetamido]-2',5'-dideoxy-5-ethyluridine,
3'-O-benzyl-5'-[2-(2,6-dichlorophenyl)acetamido]-2',5'-dideoxy-5-ethyluridine,
5'-[2-(2,6-dichlorophenyl)acetamido]-2',5'-dideoxy-3'-O-ethyl-5-ethyluridine,
2',3',5'-trideoxy-5'-[2-(2,6-dichlorophenyl)acetamido]-5-ethyluridine,
5'-[2-(2-aminophenyl)acetamido]-2',5'-dideoxy-5-ethyluridine,
5'-[2-(2-acetamidophenyl)acetamido]-3'-O-acetyl-2',5'-dideoxy-5-ethyluridine,
5'-[2-(2-acetamidophenyl)acetamido]-2',5'-dideoxy-5-ethyluridine,
2',5'-dideoxy-5-ethyl-5'-[2-(4-hydroxy-2,6-dimethylphenyl)acetamido]uridine,
5-(2-chloroethyl)-5'-[2-(2,6-dichlorophenyl)acetamido]-2',5'-dideoxyuridine,
5'-[2-(2-bromophenyl)-N-methylacetamido]-2',5'-dideoxy-5-ethyluridine,
5'-[2-(2,6-dichlorophenyl)-N-methylacetamido]-2',5'-dideoxy-5-ethyluridine,
5'-[2(RS)-(2-bromophenyl)-N-methylpropionamido]-2',5'-dideoxy-5-ethyluridine,
3'-O-acetyl-5'-[2-(2-bromophenyl)-N-methylacetamido]-2',5'-dideoxy-5-ethyluridine,
2',5'-dideoxy-5-ethyl-5'-(2-naphthalamido)uridine,
2',5'-dideoxy-5-ethyl-5'-(2-phenylbenzamido)uridine,
2',5'-dideoxy-5-ethyl-5 ` -(3-phenylpropionamido)uridine,
2',5'-dideoxy-5-ethyl-5'-(4-phenylbutyramido)uridine,
2',5'-dideoxy-5'-cinnamamido-5-ethyluridine,
2',5'-dideoxy-5-ethyl-5'-(3-phenyl-2-propynamido)-uridine,
2',5'-dideoxy-5-ethyl-5'-[3-(phenylsulphonyl)propionamido]uridine,
5'-(2-bromocinnamamido)-2',5'-dideoxy-5-ethyluridine,
2',5'-dideoxy-5-ethyl-5'-[2-(2-thienyl)acetamido]-uridine,
2',5'-dideoxy-5-ethyl-5'-[2-(3-thienyl)acetamido]-uridine,
5'-(1-adamantylcarboxamido)-2',5'-dideoxy-5-ethyluridine,
2',5'-dideoxy-5-ethyl-5'-(2-pyridylcarboxamido)-uridine,
2',5'-dideoxy-5-ethyl-5'-(3-pyridylcarboxamido)uridine,
2',5'-dideoxy-5-ethyl-5'-(2-(phenylsulphonyl)acetamido]uridine,
2',5'-dideoxy-5-ethyl-5'-(9-fluorenylcarboxamido)-uridine,
3'-O-acetyl-2',5'-dideoxy-5-ethyl-5'-(9-fluorenylcarboxamido)uridine,
3'-O-butyryl-2',5'-dideoxy-5-ethyl-5'-(9-fluorenylcarboxamido)uridine,
2',5'-dideoxy-5-ethyl-3'-O-(3,3-dimethylbutyryl)-5'-(9-fluorenylcarboxamido)uridine,
2',5'-dideoxy-5-ethyl-5'-(9-fluorenylcarboxamido)-3'-O-(hexadecanoyl)uridine.
2',5'-dideoxy-5-ethyl-3'-O-(9-fluorenylcarbonyl)-5'-(9-fluorenylcarboxamido)uridine,
2',5'-dideoxy-5-ethyl-5'-(2-phenylbutyramido)uridine,
2',5'-dideoxy-5-ethyl-5'-(3-phenylbutyramido)uridine,
2',5'-dideoxy-5-ethyl-5'-(2,2-diphenylacetamido)uridine,
5'-(2-cyclohexyl-2-phenylacetamido)-2',5'-dideoxy-5-ethyluridine,
2',5'-dideoxy-5-ethyl-5'-(triphenylacetamido)uridine,
2',5'-dideoxy-5-ethyl-5'-(2-methyl-2-phenylpropionamido)uridine,
2',5'-dideoxy-5-ethyl-5'-[2(RS)-phenylpropionamido)]-uridine,
5'-[2(RS)-(2,4-dichlorophenoxy)propionamido]-2',5'-dideoxy-5-ethyluridine,
5'-[2(RS)-(2,6-dichlorophenoxy)propionamido]-2',5'-dideoxy-5-ethyluridine,
5'-[2(RS)-(3,5-dichlorophenoxy)propionamido]-2',5'-dideoxy-5-ethyluridine,
5'-[2(RS)-(2-chlorophenoxy)propionamido]-2',5'-dideoxy-5-ethyluridine,
5'-[2-(2,6-dichlorophenoxy)acetamido]-2',5'-dideoxy-5-ethyluridine,
5'-[2-(2,4-dichlorophenoxy)acetamido]-2',5'-dideoxy-5-ethyluridine,
5'-[4-(2,6-dichlorophenoxy)butyramido]-2',5'-dideoxy-5-ethyluridine,
5'-[6-(2,6-dichlorophenoxy)hexanamido]-2',5'-dideoxy-5-ethyluridine,
5'-[5-(2,6-dichlorophenoxy)valeramido]-2',5'-dideoxy-5-ethyluridine,
5'-[2(RS)-(2-chloro-4-nitrophenoxy)propionamido]-2',5'-dideoxy-5-ethyluridine,
5'-[2(RS)-(2-chloro-4-phenylphenoxy)propionamido]-2',5'-dideoxy-5-ethyluridine,
5-bromo-5'-[2-(2-bromophenyl)acetamido]-2',5'-dideoxycytidine and
5'-[2-(2-bromophenyl)acetamido]-2',5'-dideoxy-5-ethylcytidine.

**14.** The compounds according to claim 1,

5'-[2-(2,6-Dichlorophenyl)acetamido]-5'-deoxythymidine,
5-bromo-5'-[2(RS)-(2,4-dichlorophenoxy)propionamido]-2',5'-dideoxyuridine,

5-bromo-5'-[2-(2,6-dichlorophenyl)acetamido]-2',5'-dideoxyuridine,
5'-benzamido-5-bromo-2',5'-dideoxyuridine,
5'-[2(RS)-(2,4-dichlorophenoxy)propionamido]-2',5'-dideoxy-5-iodouridine,
5'-[2(RS)-(2,4,5-trichlorophenoxy)propionamido]-2',5'-dideoxy-5-iodouridine,
5'-[2-(2,6-dichlorophenyl)acetamido]-2',5'-dideoxy-5-iodouridine,
3'-O-benzyl-5'-[2(RS)-(2,4-dichlorophenoxy)propionamido]-2',5'-dideoxy-5-ethyluridine,
5'-[2(RS)-(2,4-dichlorophenoxy)propionamido]-2',5'-dideoxy-3'-O-ethyl-5-ethyluridine,
5'-[2(RS)-(2,4-dichlorophenoxy)-N-methylpropionamido]-2',5'-dideoxy-5-ethyluridine,
5'-[2(R)-(2,4-dichlorophenoxy)propionamido]-2',5'-dideoxy-5-ethyluridine,
5'-[2(S)-(2,4-dichlorophenoxy)propionamido]-2',5'-dideoxy-5-ethyluridine,
5'-[2(RS)-(2,4-dichlorophenoxy)butyramido]-2',5'-dideoxy-5-ethyluridine,
5'-[2(RS)-(4-acetamido-2-chlorophenoxy)propionamido]-2',5'-dideoxy-5-ethyluridine,
5'-[2(RS)-(2-chloro-4-methoxyphenoxy)propionamido]-2',5'-dideoxy-5-ethyluridine,
2',5'-dideoxy-5-ethyl-5'-[2(RS)-(2-methylbiphenylyloxy)-propionamido]uridine,
5'-[2(RS)-(2,4-dichlorophenoxy)propionamido]-5'-deoxythymidine,
5'-[2-(2,4-dichlorophenoxy)-2-methylpropionamido]-2',5'-dideoxy-5-ethyluridine,
2',5'-dideoxy-5-ethyl-5'-[2(RS)-phenoxypropionamido]-uridine,
2',5'-dideoxy-5-ethyl-5'-[2(RS)-(2-fluorophenoxy)propionamido]uridine,
2',5'-dideoxy-5-ethyl-5'-[2(RS)-(2-trifluoromethylphenoxy)propionamido]uridine,
1-[5-[2(RS)-(2,4-dichlorophenoxy)propionamido]-2,5 -dideoxy-2-fluoro-β-D-arabinofuranosyl]thymine,
5'-[2(RS)-(2,4-dichlorophenylsulphinyl)propionamido]-2',5'-dideoxy-5-ethyluridine,
5'-[2(RS)-(2,4-dichlorophenylsulphonyl)propionamido]-2',5'-dideoxy-5-ethyluridine,
5'-[2-(2,6-dichlorophenylacetamido)ethyl]-2',5'-dideoxy-5-ethyluridine,
5'-[2-[2(RS)-(2,4,5-trichlorophenoxy)propionamido]-ethyl]-2',5'-dideoxy-5-ethyluridine,
5'-[2-[2(RS)-(2,4-dichlorophenoxy)propionamido]ethyl]-2',5'-dideoxy-5-ethyluridine,
5'-[2(RS)-(2,6-dichlorobenzyl)propionamido]-2',5'-dideoxy-5-ethyluridine,
5'-[2(RS)-(2,4-dichlorobenzyl)propionamido]-2',5'-dideoxy-5-ethyluridine,
5'-[5-chloro-2,3-dihydro-2(RS)-benzofuranylcarboxamido]-2',5'-dideoxy-5-ethyluridine,
5-(6-chloro-2H-1-benzopyran-2-ylcarboxamido)-2',5'-dideoxy-5-ethyluridine,
5'-[2-(4-benzyloxy-2,6-dimethylphenyl)acetamido]-2',5'-dideoxy-5-ethyluridine,
3'-O-acetyl-5'-[2-(4-benzyloxy-2,6-dimethylphenyl)acetamido]-2',5'-dideoxy-5-ethyluridine,
3'-O-acetyl-2',5'-dideoxy-5-ethyl-5'-[2-(4-hydroxy-2,6-dimethylphenyl)acetamido]uridine,
2',5'-dideoxy-5-ethyl-5'-[2-(2,6-dimethyl-4-phosphatophenyl)acetamido]uridine,
5-(2-chloroethyl)-2',5'-dideoxy-5'-[2-(2,4-dichlorophenoxy)propionamido]uridine,
5'-benzamido-5-(2-chloroethyl)-2',5'-dideoxyuridine,
5-(2-chloroethyl)-5'-[2-(2,4,5-trichlorophenoxy)propionamido]-2',5'-dideoxyuridine,
5'-[2-(2,6-dichlorophenyl)acetamido]-2',5'-dideoxy-5-propyluridine,
5'-[2(RS)-(2,4-dichlorophenoxy)propionamido]-2',5'-dideoxy-5-propyluridine and
5-acetyl-5'-[2-(2,6-dichlorophenyl)acetamido]-2',5'-dideoxyuridine.

**15.** The compounds according to claims 1-14 for use as medicaments, especially as agents against viruses.

**16.** A process for the manufacture of the compounds of general formula I according to claim 1 and of their tautomers, characterized by

(a) reacting a compound of the general formula

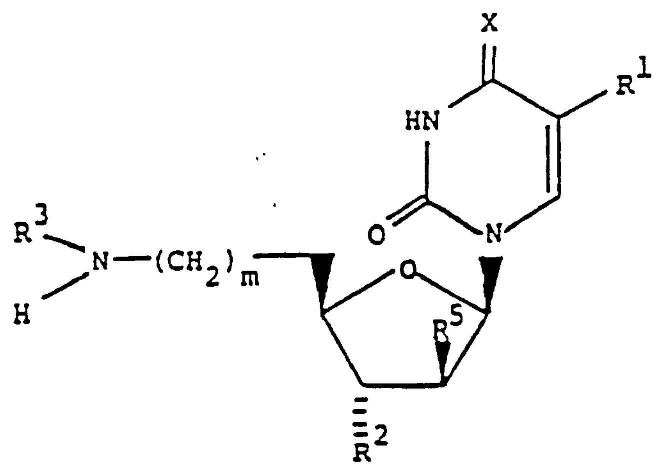

II

wherein $R^1$, $R^2$, $R^3$, $R^5$ m and X have the above significance,
or a tautomer thereof, in which a 4-amino group which may be present is optionally protected, with a reactive derivative of an acid of the general formula

$R^4$-Y-COOH   III

wherein $R^4$ and Y have the above significance,
and cleaving off a protecting group which may be present in the product, or
(b) for the manufacture of compounds of formula I or a tautomer thereof in which X represents oxygen and $R^2$ represents acyloxy, acylating a compound of formula I or a tautomer thereof in which X is oxygen and $R^2$ is hydroxy, or
(c) for the manufacture of a compound of formula I or a tautomer thereof in which X is oxygen and $R^2$ is hydroxy, deacylating a compound of formula I or a tautomer thereof in which X is oxygen and $R^2$ is acyloxy, or
(d) for the manufacture of a compound of formula I or a tautomer thereof in which Y is a group of formula (a), Z is SO or $SO_2$ and n is 1, oxidizing a compound of formula I or a tautomer thereof in which Y is a group of formula (a), Z is sulphur and n is 1, and
(e) if desired, functionally modifying a reactive substituent present in $R^4$.

**17.** Pharmaceutical preparations, especially anti-virally active preparations, containing a compound according to claims 1-14 and a pharmaceutical carrier material.

**18.** The use of a compound according to claims 1-14 for the manufacture of medicaments for the control or prophylaxis of viral infections.

**Claims for the following Contracting States: AT,ES,GR**

**1.** A process for the manufacture of compounds of the general formula

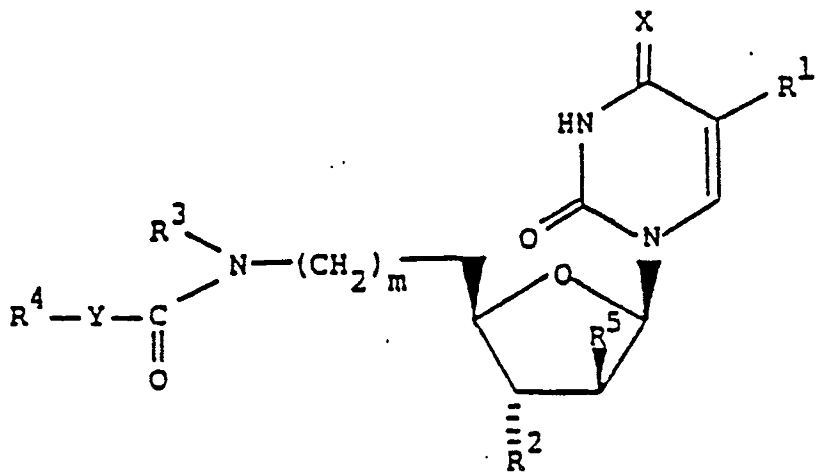

I

wherein

$R^1$ signifies halogen, $C_{1-4}$-alkyl, halo-($C_{1-4}$-alkyl) or $C_{2-4}$-alkanoyl,

$R^2$ signifies hydrogen, hydroxy, $C_{1-4}$-alkoxy, $C_{1-4}$-alkylthio or phenyl-($C_{1-4}$-alkoxy), or, when X is oxygen, also acyloxy:

$R^3$ signifies hydrogen or $C_{1-4}$-alkyl,

$R^4$ signifies a carbocyclic group, namely a monocyclic or polycyclic aromatic hydrocarbon group, which contains up to 14 carbon atoms in the cyclic structure and which can contain one or more substituents from the group halogen, hydroxy, $C_{1-4}$-alkyl, $C_{1-4}$-alkoxy, trifluoromethyl, phenyl, $C_{1-4}$-alkylphenyl, halophenyl, nitro, amino, acylamino, benzyloxy and O-phosphate, or a monocyclic or polycyclic cycloalkyl group, which contains up to 13 carbon atoms in the cyclic structure and which, when it is monocyclic, can carry one or two condensed benzene rings, or a heterocyclic group,

$R^5$ signifies hydrogen or fluorine,

m signifies 0, 1 or 2,

X signifies oxygen or NH,

Y signifies a direct bond, -CH=CH-, -C≡C- or a group of the formula

-(Z)n-A- (a)

in which A represents a $C_{1-8}$-alkylene group which can be substituted by one or two phenyl groups,

Z signifies oxygen, sulphur, SO or $SO_2$ and n signifies O or 1, with the proviso that $R^1$ is different from iodine when $R^2$ represents hydroxy or benzoyloxy, $R^3$ represents hydrogen, $R^4$ represents unsubstituted phenyl, $R^5$ represents hydrogen, m represents O, X represents oxygen and Y represents a direct bond,

and their tautomers, characterized by

(a) reacting a compound of the general formula

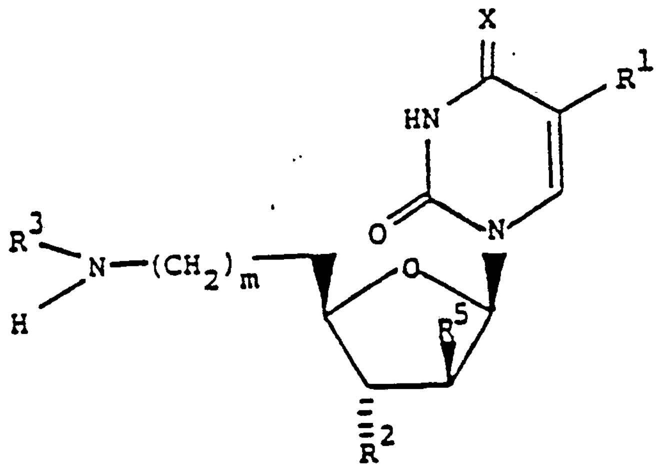

II

wherein $R^1$, $R^2$, $R^3$, $R^5$ m and X have the above significance,
or a tautomer thereof, in which a 4-amino group which may be present is optionally protected, with a reactive derivative of an acid of the general formula

$R^4$-Y-COOH III

wherein $R^4$ and Y have the above significance,
and cleaving off a protecting group which may be present in the product, or
(b) for the manufacture of compounds of formula I or a tautomer thereof in which X represents oxygen and $R^2$ represents acyloxy, acylating a compound of formula I or a tautomer thereof in which X is oxygen and $R^2$ is hydroxy, or
(c) for the manufacture of a compound of formula I or a tautomer thereof in which X is oxygen and $R^2$ is hydroxy, deacylating a compound of formula I or a tautomer thereof in which X is oxygen and $R^2$ is acyloxy, or
(d) for the manufacture of a compound of formula I or a tautomer thereof in which Y is a group of formula (a), Z is SO or $SO_2$ and n is 1, oxidizing a compound of formula I or a tautomer thereof in which Y is a group of formula (a), Z is sulphur and n is 1, and
(e) if desired, functionally modifying a reactive substituent present in $R^4$.

2. A process according to claim 1, wherein $R^1$ is fluorine, chlorine, bromine, $C_{1-4}$-alkyl or halo-($C_{1-4}$-alkyl), $R^4$ is a carbocyclic or aromatic heterocyclic group, $R^5$ is hydrogen and m is O.

3. A process according to claim 1 or 2, wherein $R^1$ is $C_{1-4}$-alkyl.

4. A process according to claims 1-3, wherein $R^2$ is hydroxy.

5. A process according to claims 1-4, wherein $R^3$ is hydrogen.

6. A process according to claims 1-5, wherein $R^4$ is phenyl which can be substituted by one or more substituents from the group halogen, $C_{1-4}$-alkyl, $C_{1-4}$-alkoxy, trifluoromethyl, phenyl and nitro.

7. A process according to claims 1 or 3-6, wherein m = O.

8. A process according to claims 1-7, wherein X is oxygen.

9. A process according to claims 1 - 8, wherein Y is a group of formula (a).

10. A process according to claims 1-7, in which $R^1$ is ethyl, $R^2$ is hydroxy, $R^3$ is a hydrogen, $R^4$ is 2-bromophenyl, 2,6-dichlorophenyl or 4-biphenylyl, m = O, X is oxygen and Y is a group of formula (a) in which A is-$CH_2$ or -$CH(CH_3)$- and n is equal to O.

11. A process according to claims 1-7, in which $R^1$ is ethyl or propyl, $R^2$ is hydroxy, $R^3$ is hydrogen, $R^4$ is 2-biphenylyl, 2,4-dichlorophenyl, 2,4,5-trichlorophenyl, 4-chloro-2-nitrophenyl or 2,4-dichloro-5-methoxyphenyl, m is equal to O, X is oxygen and Y is a group of formula (a) in which A is -CH($CH_3$) or -CH-(phenyl)-, Z is oxygen and n is equal to 1.

12. A process according to claim 1 for the.manufacture of the compounds

5'-[2-(2-bromophenyl)acetamido]-2',5'-dideoxy-5-ethyluridine,
5'-[2-(2,6-dichlorophenyl)acetamido]-2',5'-dideoxy-5-ethyluridine,
5'-[2(RS)-(2,4-dichlorophenoxy)propionamido]-2',5'-dideoxy-5-ethyluridine,
5'-[2-(4-biphenylyl)acetamido]-2',5'-dideoxy-5-ethyluridine,
2',5'-dideoxy-5-ethyl-5'- [2(RS)-(2-phenylphenoxy)-propionamido]uridine,
1-[5-[2-(RS)-(2,4-dichlorophenoxy)propionamido]-2,5-dideoxy-2-fluoro-β-D-arabinofuranosyl]-5-ethyluracil,
5'-[2(RS)-(2,4,5-trichlorophenoxy)propionamido]-2',5'-dideoxy-5-ethyluridine,
5'-[2(RS)-(4-chloro-2-nitrophenoxy)propionamido]-2',5'-dideoxy-5-ethyluridine,
5'-[2(RS)-(2,4-dichlorophenoxy)-2-phenylacetamido]-2',5'-deoxy-5-ethyluridine,
5'-[2(RS)-(2,4-dichloro-5-methoxyphenoxy)propionamido]-2',5'-dideoxy-5-ethyluridine,
5'-[2(RS)-(2,4-dichlorophenoxy)propionamido]-2',5'-dideoxy-5-propyluridine..

13. A process according to claim 2, for the manufacture of the compounds

2',5'-dideoxy-5-ethyl-5'-(2-phenylacetamido]uridine,
5'-(4-bromobenzamido)-5'-deoxythymidine,
5'-deoxy-5'-(4-nitrobenzamido)thymidine,
5'-benzamido-5'-deoxythymidine,
5'-deoxy-5'-(2-fluorobenzamido)thymidine,
5'-deoxy-5'-(2-nitrobenzamido)thymidine,
5'-[2-(2-bromophenyl)acetamido]-5'deoxythymidine,
5'-deoxy-5'-[2-(4-nitrophenyl)acetamido]thymidine,
5'-deoxy-5'-(2-phenylacetamido)thymidine,
5'-deoxy-5'-[2-(4-trifluoromethylphenyl)acetamido]-thymidine,
5'-benzamido-2',5'-dideoxy-5-ethyluridine,
2',5'-dideoxy-5-ethyl-5'-(2-fluorobenzamido)uridine,
5'-(2-bromobenzamido)-2',5'-dideoxy-5-ethyluridine,
2',5'-dideoxy-5-ethyl-5'-(4-nitrobenzamido)uridine,
2',5'-dideoxy-5-ethyl-5'-(2-trifluoromethylbenzamido)uridine,
2',5'-dideoxy-5-ethyl-5'-[2-(2,6-dimethylphenyl)-acetamido]uridine,
5'-[2(RS)-(2-bromophenyl)propionamido]-2',5'-dideoxy-5-ethyluridine,
5'-[2-(2-chloro-3-nitrophenyl)acetamido]-2',5'-dideoxy-5-ethyluridine,
5'-[2(RS)-(2,6-dichlorophenyl)propionamido]-2',5'-dideoxy-5-ethyluridine,
2',5'-dideoxy-5-ethyl-5'-[2-(3,5-dimethylphenyl)acetamido]uridine,
2',5'-dideoxy-5'-[2-(3,5-dimethoxyphenyl)acetamido]-5-ethyluridine,
2',5'-dideoxy-5-ethyl-5'-[2-(2,3,5,6-tetramethylphenyl)acetamido]uridine,
2',5'-dideoxy-5-ethyl-5'-[2-(2-methoxyphenyl)acetamido]uridine,
2',5'-dideoxy-5-ethyl-5'-[2-(2-nitrophenyl)acetamido]-uridine,
5-bromo-5'-[2-(2-bromophenyl)acetamido]2',5'-dideoxyuridine,
3'-O-butyryl-5'-[2-(2,6-dichlorophenyl)acetamido]-2',5'-dideoxy-5-ethyluridine,
2',5'-dideoxy-5'-[2-(2,6-dichlorophenyl)acetamido]-3'-O-(3,3-dimethylbutyryl)-5-ethyluridine,
5'-[2-(2,6-dichlorophenyl)acetamido]2',5'-dideoxy-5-ethyl-3'-O-palmitoyluridine,
3'-O-acetyl-5'-[2-(2,6-dichlorophenyl)acetamido]-2',5'-dideoxy-5-ethyluridine,
3'-O-benzyl-5'-[2-(2,6-dichlorophenyl)acetamido]-2,'5'-dideoxy-5-ethyluridine,
5'-[2-(2,6-dichlorophenyl)acetamido]-2',5'-dideoxy-3'-O-ethyl-5-ethyluridine,
2',3',5'-trideoxy-5'-[2-(2,6-dichlorophenyl)acetamido]-5-ethyluridine,
5'-[2-(2-aminophenyl)acetamido]-2',5'-dideoxy-5-ethyluridine,
5'-[2-(2-acetamidophenyl)acetamido]-3'-O-acetyl-2',5'-dideoxy-5-ethyluridine,
5'-[2-(2-acetamidophenyl)acetamido]-2',5'-dideoxy-5-ethyluridine,
2',5'-dideoxy-5-ethyl-5'-[2-(4-hydroxy-2,6-dimethylphenyl)acetamido]uridine,

5-(2-chloroethyl)-5'-[2-(2,6-dichlorophenyl)acetamido]-2',5'-dideoxyuridine,
5'-[2-(2-bromophenyl)-N-methylacetamido]-2',5'-dideoxy-5-ethyluridine,
5'-[2-(2,6-dichlorophenyl)-N-methylacetamido]2',5'-dideoxy-5-ethyluridine,
5'-[2(RS)-(2-bromophenyl)-N-methylpropionamido]-2',5'-dideoxy-5-ethyluridine,
3'-O-acetyl-5'-[2-(2-bromophenyl)N-methylacetamido]-2',5'-dideoxy-5-ethyluridine,
2',5'-dideoxy-5-ethyl-5'-(2-naphthalamido)uridine,
2',5'-dideoxy-5-ethyl-5'-(2-phenylbenzamido)uridine,
2',5'-dideoxy-5-ethyl-5'-(3-phenylpropionamido)uridine,
2',5'-dideoxy-5-ethyl-5'-(4-phenylbutyramido)uridine,
2',5'-dideoxy-5'-cinnamamido-5-ethyluridine,
2',5'-dideoxy-5-ethyl-5'-(3-phenyl-2-propynamido)-uridine,
2',5'-dideoxy-5-ethyl-5'-[3-(phenylsulphonyl)propionamido]uridine,
5'-(2-bromocinnamamido)-2',5'-dideoxy-5-ethyluridine,
2',5'-dideoxy-5-ethyl-5'-[2-(2-thienyl)acetamido]-uridine,
2',5'-dideoxy-5-ethyl-5'[2-(3-thienyl)acetamido]-uridine,
5'-(1-adamantylcarboxamido)-2',5'-dideoxy-5-ethyl-uridine.
2',5'-dideoxy-5-ethyl-5'-(2-pyridylcarboxamido)-uridine,
2',5'-dideoxy-5-ethyl-5'-(3-pyridylcarboxamido)uridine,
2',5'-dideoxy-5-ethyl-5'-[2-(phenylsulphonyl)acetamido]uridine,
2',5'-dideoxy-5-ethyl-5'-(9-fluorenylcarboxamido)-uridine,
3'-O-acetyl-2',5'-dideoxy-5-ethyl-5'-(9-fluorenylcarboxamido)uridine,
3'-O-butyryl-2',5'-dideoxy-5-ethyl-5'-(9-fluorenylcarboxamido)uridine,
2',5'-dideoxy-5-ethyl-3'-O-(3,3-dimethylbutyryl)-5'-(9-fluorenylcarboxamido)uridine,
2',5'-dideoxy-5-ethyl-5'-(9-fluorenylcarboxamido)-3'-O-(hexadecanoyl)uridine,
2',5'-dideoxy-5-ethyl-3'-O-(9-fluorenylcarbonyl)-5'-(9-fluorenylcarboxamido)uridine,
2',5'-dideoxy-5-ethyl-5'-(2-phenylbutyramido)uridine,
2',5'-dideoxy-5-ethyl-5'-(3-phenylbutyramido)uridine,
2',5'-dideoxy-5-ethyl-5'-(2,2-diphenylacetamido)uridine,
5'-(2-cyclohexyl-2-phenylacetamido)-2',5'-dideoxy-5-ethyluridine,
2',5'-dideoxy-5-ethyl-5'-(triphenylacetamido)uridine,
2',5'-dideoxy-5-ethyl-5'-(2-methyl-2-phenylpropionamido)uridine,
2',5'-dideoxy-5-ethyl-5'-[2(RS)-phenylpropionamido]-uridine,
5'-[2(RS)-(2,4-dichlorophenoxy)propionamido]-2',5'-dideoxy-5-ethyluridine,
5'-[2(RS)-(2,6-dichlorophenoxy)propionamido)-2',5'-dideoxy-5-ethyluridine,
5'-[2(RS)-(3,5 -dichlorophenoxy)propionamido]-2',5'-dideoxy-5-ethyluridine,
5'-[2(RS)-(2-chlorophenoxy)propionamido]-2',5'-dideoxy-5-ethyluridine,
5'-[2-(2,6-dichlorophenoxy)acetamido]-2',5'-dideoxy-5-ethyluridine,
5'-[2-(2,4-dichlorophenoxy)acetamido]-2',5'-dideoxy-5-ethyluridine,
5'-[4-(2,6-dichlorophenoxy)butyramido]-2',5'-dideoxy-5-ethyluridine,
5'-[6-(2,6-dichlorophenoxy)hexanamido]-2',5'-dideoxy-5-ethyluridine,
5'-[5-(2,6-dichlorophenoxy)valeramido]-2',5'-dideoxy-5-ethyluridine,
5'-[2(RS)-(2-chloro-4-nitrophenoxy)propionamido]-2',5'-dideoxy-5-ethyluridine,
5'-[2(RS)-(2-chloro-4-phenylphenoxy)propionamido]-2',5'-dideoxy-5-ethyluridine,
5-bromo-5'-[2-(2-bromophenyl)acetamido]-2',5'-dideoxycytidine and
5'-[2-(2-bromophenyl)acetamido]-2',5'-dideoxy-5-ethylcytidine.

**14.** A process according to claim 1 for the manufacture of the compounds

5'-[2-(2,6-dichlorophenyl)acetamido]-5'-deoxythymidine,
5-bromo-5'-[2(RS)-(2,4-dichlorophenoxy)propionamidol-2',5'-dideoxyuridine,
5-bromo-5'-[2-(2,6-dichlorophenyl)acetamido]-2',5'-dideoxyrnridine,
5'-benzamido-5-bromo-2',5'-dideoxyuridine,
5'-[2(RS)-(2,4-dichlorophenoxy)propionamido]-2',5'-dideoxy-5-iodouridine,
5'-[2(RS)-(2,4,5-trichlorophenoxy)propionamido]-2',5'-dideoxy-5-iodouridine,
5'-[2-(2,6-dichlorophenyl)acetamido]-2',5'-dideoxy-5-iodouridine,
3'-O-benzyl-5'-[2(RS)-(2,4-dichlorophenoxy)propionamido]-2',5'-dideoxy-5-ethyluridine,
5'-[2(RS)-(2,4-dichlorophenoxy)propionamido]-2',5'-dideoxy-3'-O-ethyl-5-ethyluridine,

5'-[2(RS)-(2,4-dichlorophenoxy)-N-methylpropionamido]-2',5'-dideoxy-5-ethyluridine,
5'-[2(R)-(2,4-dichlorophenoxy)propionamido]-2',5'-dideoxy-5-ethyluridine,
5'-[2(S)-(2,4-dichlorophenoxy)propionamido]-2',5'-dideoxy-5-ethyluridine,
5'-[2(RS)-(2,4-dichlorophenoxy)butyramido]-2',5'-dideoxy-5-ethyluridine,
5'-[2(RS)-(4-acetamido-2-chlorophenoxy)propionamido]-2',5'-dideoxy-5-ethyluridine,
5'-[2(RS)-(2-chloro-4-methoxyphenoxy)propionamido]-2',5'-dideoxy-5-ethyluridine,
2',5'-dideoxy-5-ethyl-5'-[2(RS)-(2-methylbiphenylyloxy)-propionamido]uridine,
5'-[2(RS)-(2'4-dichlorophenoxy)propionamido]-5'-deoxythymidine,
5'-[2-(2,4-dichlorophenoxy)-2-methylpropionamido]-2',5'-dideoxy-5-ethyluridine,
2',5'-dideoxy-5-ethyl-5'-[2(RS)-phenoxypropionamido]-uridine,
2',5'-dideoxy-5-ethyl-5'-[2(RS)-(2-fluorophenoxy)propionamido]uridine,
2',5'-dideoxy-5-ethyl-5'-[2(RS)-(2-trifluoromethylphenoxy)propionamido]uridine,
1-[5-[2(RS)-(2,4-dichlorophenoxy)propionamido]-2,5-dideoxy-2-fluoro-ß-D-arabinofuranosyl]thymine,
5'-[2(RS)-(2,4-dichlorophenylsulphinyl)propionamido]-2',5'-dideoxy-5-ethyluridine,
5'-[2(RS)-(2,4-dichlorophenylsulphonyl)propionamido]-2',5'-dideoxy-5-ethyluridine,
5'-[2-(2,6-dichlorophenylacetamido)ethyl]-2',5'-dideoxy-5-ethyluridine,
5'-[2-[2(RS)-(2,4,5-trichlorophenoxy)propionamido]-ethyl]-2',5'-dideoxy-5-ethyluridine,
5'-[2-[2(RS)-(2,4-dichlorophenoxy)propionamido]ethyl]-2',5'-dideoxy-5-ethyluridine,
5'-[2(RS)-(2,6-dichlorobenzyl)propionamido]-2',5'-dideoxy-5-ethyluridine,
5'-[2(RS)-(2,4-dichlorobenzyl)propionamido]-2',5'-dideoxy-5-ethyluridine,
5'-[5-chloro-2,3-dihydro-2(RS)-benzofuranylcarboxamido]-2',5'-dideoxy-5-ethyluridine,
5-(6-chloro-2H-1-benzopyran-2-ylcarboxamido)-2',5'-dideoxy-5-ethyluridine,
5'-[2-(4-benzyloxy-2,6-dimethylphenyl)acetamido]-2',5'-dideoxy-5-ethyluridine,
3'-O-acetyl-5'-[2-(4-benzyloxy-2,6-dimethylphenyl)acetamido]-2',5'-dideoxy-5-ethyluridine,
3'-O-acetyl-2',5'-dideoxy-5-ethyl-5'-[2-(4-hydroxy-2,6-dimethylphenyl)acetamido]uridine,
2',5'-dideoxy-5-ethyl-5'-[2-(2,6-dimethyl-4-phosphatophenyl)acetamido]uridine,
5-(2-chloroethyl)-2',5'-dideoxy-5-'[2-(2,4-dichlorophenoxy)propionamido]uridine,
5'-benzamido-5-(2-chloroethyl)-2',5'-dideoxyuridine,
5-(2-chloroethyl)-5'-[2-(2,4,5-trichlorophenoxy)propionamido]-2',5'-dideoxyuridine,
5'-[2-(2,6-dichlorophenyl)acetamido]-2',5'-dideoxy-5-propyluridine,
5'-[2(RS)-(2,4-dichlorophenoxy)propionamido]-2',5'-dideoxy-5-propyluridine and
5-acetyl-5'-[2-(2,6-dichlorophenyl)acetamido]-2',5'-dideoxyuridine.

**15.** A process for the manufacture of pharmaceutical preparations, especially such having anti-viral activity, characterized by bringing a compound of formula I or a tautomer thereof into a galenical administration form.

**16.** The use of a compound of formula I or of a tautomer thereof for the manufacture of medicaments for the control or prophylaxis of viral infections.

## Revendications

**Revendications pour les Etats contractants suivants: BE, CH, DE, FR, GB, IT, LI, LU, NL, SE.**

**1.** Composés de formule générale

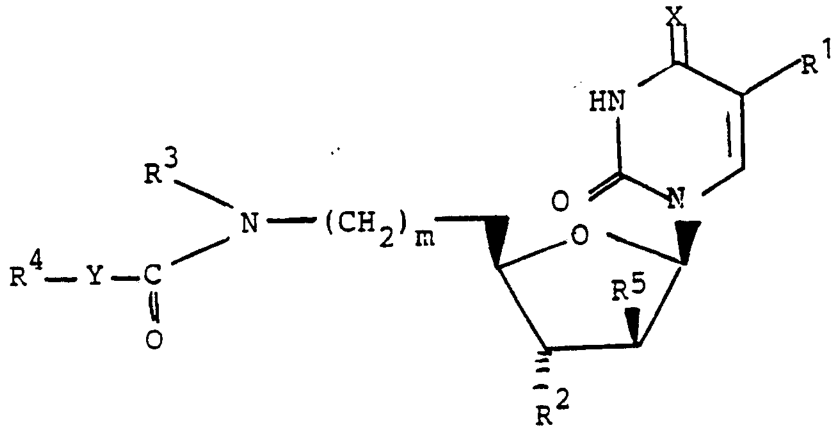

I

dans laquelle

$R^1$ représente un halogène, un groupe alkyle en C1-C4, halogénoalkyle en C1-C4 ou alcanoyle en C2-C4,

$R^2$ représente l'hydrogène, un groupe hydroxy, alcoxy en C1-C4, alkylthio en C1-C4 ou phénylalcoxy en C1-C4, ou bien encore, lorsque X représente l'oxygène, un groupe acyloxy,

$R^3$ représente l'hydrogène ou un groupe alkyle en C1-C4,

$R^4$ représente un groupe carbocyclique, à savoir un groupe hydrocarboné aromatique monocyclique ou polycyclique contenant jusqu'à 14 atomes de carbone dans la structure cyclique et qui peut porter un ou plusieurs substituants du groupe formé par les halogènes, les groupes hydroxy, alkyle en C1-C4, alcoxy en C1-C4, trifluorométhyle, phényle, (alkyle en C1-C4)-phényle, halogénophényle, nitro, amino, acylamino, benzyloxy et O-phosphate, ou un groupe cycloalkyle monocyclique ou polycyclique, contenant jusqu' à 13 atomes de carbone dans la structure cyclique et qui, lorsqu' il est monocyclique, peut porter un ou plusieurs cycles benzéniques condensés, ou un groupe hétérocyclique,

$R^5$ représente l'hydrogène ou le fluor,

m est égal à 0, 1 ou 2,

X représente l'oxygène ou le groupe NH,

Y représente une liaison directe, un groupe -CH=CH-,-C≡C- ou un groupe de formule

$(Z)_n$-A- (a)

dans laquelle

A représente un groupe alkylène en C1-C8 qui peut être substitué par un ou deux groupes phényle,

z représente l'oxygène, le soufre, un groupe SO ou $SO_2$ et n est égal à 0 ou 1, sous réserve que $R^1$ ne peut représenter l'iode lorsque $R^2$ représente un groupe hydroxy ou benzoyloxy $R^3$ l'hydrogène, $R^4$ un groupe phényle non substitué, $R^5$ l'hydrogène, m est égal à 0, X représente l'oxygène et Y une liaison directe,

et leurs tautomères.

**2.** Composés selon la revendication 1, dans lesquels $R^1$ représente le fluor, le chlore, le brome, un groupe alkyle en C1-C4 ou halogénoalkyle en C1-C4, $R^4$ représente un groupe carbocyclique ou aromatique-hétérocyclique, $R^5$ représente l'hydrogène et m est égal à 0.

**3.** Composés selon la revendication 1 ou 2, dans lesquels $R^1$ représente un groupe alkyle en C1-C4.

**4.** Composés selon les revendications 1 à 3, dans lesquels $R^2$ représente un groupe hydroxy.

**5.** Composés selon les revendications 1 à 4, dans lesquels $R^3$ représente l'hydrogène.

**6.** Composés selon les revendications 1 à 5, dans lesquels $R^4$ représente un groupe phényle qui peut porter un ou plusieurs substituants choisis parmi les halogènes, les groupes alkyle en C1-C4, alcoxy en

C1-C4, trifluorométhyle, phényle et nitro.

7. Composés selon les revendications 1 ou 3 à 6, dans lesquels m = 0.

8. Composés selon les revendications 1 à 7, dans lesquels X représente l'oxygène.

9. Composés selon les revendications 1 à 8, dans lesquels Y représente un groupe de formule (a).

10. Composés selon les revendications 1 à 7, dans lesquels $R^1$ représente un groupe éthyle, $R^2$ un groupe hydroxy, $R^3$ l'hydrogène, $R^4$ un groupe 2-bromophényle, 2,6-dichlorophényle ou 4-biphénylyle, m = 0, X représente l'oxygène et Y un groupe de formule (a) dans laquelle A représente $-CH_2-$ ou $-CH(CH_3)-$ et n = 0.

11. Composés selon les revendications 1 à 7, dans lesquels $R^1$ représente un groupe éthyle ou propyle, $R^2$ un groupe hydroxy, $R^3$ l'hydrogène, $R^4$ un groupe 2-biphénylyle, 2,4-dichlorophényle, 2,4,5-trichlorophényle, 4-chloro-2-nitrophényle ou 2,4-dichloro-5-méthoxyphényle, m est égal à 0, X représente l'oxygène et Y un groupe de formule (a) dans laquelle A représente $-CH(CH_3)-$ ou-CH(phényle)-, Z représente l'oxygène et n est égal à 1.

12. Les composés selon la revendication 1,
5'-[2-(2-bromophényl)-acétamido]-2',5'-didésoxy-5-éthyluridine,
5'-[2-(2,6-dichlorophényl)-acétamido]-2',5'-didésoxy-5-éthyluridine,
5'-[2(RS)-(2,4-dichlorophénoxy)-propionamido]-2',5'-didésoxy-5-éthyluridine,
5'-[2-(4-biphénylyl)-acétamido]-2',5'-didésoxy-5-éthyluridine,
2',5'-didésoxy-5-éthyl-5'-[2(RS)-(2-phénylphénoxy)-propionamido]-uridine,
1-[5-[2(RS)-(2,4-dichlorophénoxy)-propionamido]-2,5-didésoxy-2-fluoro-bêta-D-arabinofurannosyl]-5-éthyluracile,
5'-[2(RS)-(2,4,5-trichlorophénoxy)-propionamido]-2',5'-didésoxy-5-éthyluridine,
5'-[2(RS)-(4-chloro-2-nitrophénoxy)-propionamido]-2',5'-didésoxy-5-éthyluridine,
5'-[2(RS)-(2,4-dichlorophénoxy)-2-phénylacétamido]-2',5'-désoxy-5-éthyluridine,
5'-[2(RS)-(2,4 -dichloro-5-méthoxyphénoxy)-propionamido]-2',5'-didésoxy-5-éthyluridine et
5'-[2(RS)-(2,4-dichlorophénoxy)-propionamido]-2',5'-didésoxy-5-propyluridine.

13. Les composés selon la revendication 2,
2',5'-didésoxy-5-éthyl-5'-(2-phénylacétamido)-uridine,
5'-(4-bromobenzamido)-5'-désoxythymidine,
5'-désoxy-5'-(4-nitrobenzamido)-thymidine,
5'-benzamido-5'-désoxythymidine,
5'-désoxy-5'-(2-fluorobenzamido)-thymidine,
5'-désoxy-5'-(2-nitrobenzamido)-thymidine,
5'-[2-(2-bromophényl)-acétamido]-5'-désoxythymidine,
5'-désoxy-5'-[2-(4-nitrophényl)-acétamido]-thymidine,
5'-désoxy-5'-(2-phénylacétamido)-thymidine,
5'-désoxy-5'-[2-(4-trifluorométhylphényl)acétamido]-thymidine,
5'-benzamido-2',5'-didésoxy-5-éthyluridine,
2',5'-didésoxy-5-éthyl-5'-(2-fluorobenzamido)-uridine,
5'-(2-bromobenzamido)-2',5'-didésoxy-5-éthyluridine,
2',5'-didésoxy-5-éthyl-5'-(4-nitrobenzamido)-uridine,
2',5'-didésoxy-5-éthyl-5'-(2-trifluorométhylbenzamido)-uridine,
2',5'-didésoxy-5-éthyl-5'-[2-(2,6-diméthylphényl)-acétamido]-uridine,
5'-[2(RS)-(2-bromophényl)-propionamido]-2',5'-didésoxy-5-éthyluridine,
5'-[2-(2-chloro-3-nitrophényl)-acétamido]-2',5'-didésoxy-5-éthyluridine,
5'-[2(RS)-(2,6-dichlorophényl)-propionamido]-2',5'-didésoxy-5-éthyluridine,
2',5'-didésoxy-5-éthyl-5'-[2-(3,5-diméthylphényl)-acétamido]-uridine,
2',5'-didésoxy-5'-[2-(3,5-diméthoxyphényl)-acétamido]-5-éthyluridine,
2',5'-didésoxy-5-éthyl-5'-[2-(2,3,5,6-tétraméthylphényl)-acétamido]-uridine,
2',5'-didésoxy-5-éthyl-5'-[2-(2-méthoxyphényl)acétamido]-uridine,
2',5'-didésoxy-5-éthyl-5'-[2-(2-nitrophényl)]-acétamido-uridine,

5-bromo-5'-[2-(2-bromophényl)acétamido]-2',5'-didésoxy-uridine,
3'-O-butyryl-5'-[2-(2,6-dichlorophényl)acétamido]-2',5'-didésoxy-5-éthyluridine,
2',5'-didésoxy-5'-[2-(2,6-dichlorophényl)acétamido]-3'-O-(3,3-diméthylbutyryl)-5-éthyluridine,
5'-[2-(2,6-dichlorophényl)acétamido]-2',5'-didésoxy-5-éthyl-3'-O-palmitoyluridine,
3'-O-acétyl-5'-[2-(2,6-dichlorophényl)acétamido]-2',5'-didésoxy-5-éthyluridine,
3'-O-benzyl-5'-[2-(2,6-dichlorophényl)acétamido]-2',5'-didésoxy-5-éthyluridine,
5'-[2-(2,6-dichlorophényl)acétamido]-2',5'-didésoxy-3'-O-éthyl-5-éthyluridine,
2',3',5'-tridésoxy-5'-[2-(2,6-dichlorophényl)acétamido]-5-éthyluridine,
5'-[2-(2-aminophényl)acétamido]-2',5'-didésoxy-5-éthyl-uridine,
5'-[2-(2-acétamidophényl)acétamido]-3'-O-acétyl-2',5'-didésoxy-5-éthyluridine,
5'-[2-(2-acétamidophényl)acétamido]-2',5'-didésoxy-5-éthyluridine,
2',5'-didésoxy-5-éthyl-5'[2-(4-hydroxy-2,6-diméthyl-phényl)acétamido]-uridine,
5-(2-chloroéthyl)-5'-[2-(2,6-dichlorophényl)acétamido]-2',5'-didésoxyuridine,
5'-[2-(2-bromophényl)-N-méthylacétamido]-2',5'-didésoxy-5-éthyluridine,
5'-[2-(2,6-dichlorophényl)-N-méthylacétamido]-2',5'-didésoxy-5-éthyluridine,
5'-[2(RS)-(2-bromophényl)-N-méthylpropionamido]-2',5'-didésoxy-5-éthyluridine,
3'-O-acétyl-5'-[2-(2-bromophényl)-N-méthylacétamido]-2',5'-didésoxy-5-éthyluridine,
2',5'-didésoxy-5-éthyl-5'-(2-naphtalamido)-uridine,
2',5'-didésoxy-5-éthyl-5'-(2-phénylbenzamido)-uridine,
2',5'-didésoxy-5-éthyl-5'-(3-phénylpropionamido)-uridine,
2',5'-didésoxy-5-éthyl-5'-(4-phénylbutyramido)-uridine,
2',5'-didésoxy-5'-cinnamamido-5-éthyluridine,
2',5'-didésoxy-5-éthyl-5'-(3-phényl-2-propynamido)-uridine,
2',5'-didésoxy-5-éthyl-5'-[3-(phénylsulfonyl)-propion-amido]-uridine,
5'-(2-bromocinnamamido)-2',5'-didésoxy-5-éthyluridine,
2',5'-didésoxy-5-éthyl-5'-[2-(2-thiényl)-acétamido]-uridine,
2',5'-didésoxy-5-éthyl-5'-[2-(3-thiényl)-acétamido]-uridine,
5'-(1-adamantylcarboxamido)-2',5'-didésoxy-5-éthyl-uridine,
2',5'-didésoxy-5-éthyl-5'-(2-pyridylcarboxamido)-uridine,
2',5'-didésoxy-5-éthyl-5'-(3-pyridylcarboxamido)-uridine,
2',5'-didésoxy-5-éthyl-5'-[2-(phénylsulfonyl)acétamido]-uridine,
2',5'-didésoxy-5-éthyl-5'-(9-fluorénylcarboxamido)-uridine,
3'-O-acétyl-2',5'-didésoxy-5-éthyl-5'-(9-fluorénylcarboxamido)-uridine,
3'-O-butyryl-2',5'-didésoxy-5-éthyl-5'-(9-fluorénylcarboxamido)-uridine,
2',5'-didésoxy-5-éthyl-3'-O-(3,3-diméthylbutyryl)-5'-(9-fluorénylcarboxamido)uridine,
2',5'-didésoxy-5-éthyl-5'-(9-fluorénylcarboxamido)-3'-O-(hexadécanoyl)-uridine,
2',5'-didésoxy-5-éthyl-3'-O-(9-fluorénylcarbonyl)-5'-(9-fluorénylcarboxamido)uridine,
2',5'-didésoxy-5-éthyl-5'-(2-phénylbutyramido)-uridine,
2',5'-didésoxy-5-éthyl-5'-(3-phénylbutyramido)-uridine,
2',5'-didésoxy-5-éthyl-5'-(2,2-diphénylacétamido)-uridine,
5'-(2-cyclohexyl-2-phénylacétamido)-2',5'-didésoxy-5-éthyluridine,
2',5'-didésoxy-5'-éthyl-5'-(triphénylacétamido)-uridine,
2',5'-didésoxy-5-éthyl-5'-(2-méthyl-2-phénylpropionamido)-uridine,
2',5'-didésoxy-5-éthyl-5'-[2(RS)-phénylpropionamido)]-uridine,
5'-[2(RS)-(2,4-dichlorophénoxy)propionamido]-2',5'-didésoxy-5-éthyluridine,
5'-[2(RS)-(2,6-dichlorophénoxy)-propionamido]-2',5'-didésoxy-5-éthyluridine,
5'-[2(RS)-(3,5-dichlorophénoxy)propionamido]-2',5'-didésoxy-5-éthyluridine,
5'-[2(RS)-(2-chlorophénoxy)propionamido]-2',5'-didésoxy-5-éthyluridine,
5'-[2-(2,6-dichlorophénoxy)acétamido]-2',5'-didésoxy-5-éthyluridine,
5'-[2-(2,4-dichlorophénoxy)-acétamido]-2',5'-didésoxy-5-éthyluridine,
5'-[4-(2,6-dichlorophénoxy)-butyramido]-2',5'-didésoxy-5-éthyluridine,
5'-[6-(2,6-dichlorophénoxy)-hexanamido]-2',5'-didésoxy-5-éthyluridine,
5'-[5-(2,6-dichlorophénoxy)-valéramido]-2',5'-didésoxy-5-éthyluridine,
5'-[2(RS)-(2-chloro-4-nitrophénoxy)-propionamido]-2',5'-didésoxy-5-éthyluridine,
5'-[2(RS)-(2-chloro-4-phénylphénoxy)propionamido]-2',5'-didésoxy-5-éthyluridine,
5-bromo-5'-[2-(2-bromophényl)acétamido]-2',5'-didésoxy-cytidine et
5'-[2-(2-bromophényl)-acétamido]-2',5'-didésoxy-5-éthyl-cytidine.

14. Les composés selon la revendication 1,
5'-[2-(2,6-dichlorophényl)-acétamido]-5'-désoxythymidine,
5-bromo-5'-[2(RS)-(2,4-dichlorophénoxy)propionamido]-2',5'-didésoxyuridine,
5-bromo-5'-[2-(2,6-dichlorophényl)-acétamido]-2',5'-didésoxyuridine,
5'-benzamido-5-bromo-2',5'-didésoxyuridine.
5'-[2(RS)-(2,4-dichlorophénoxy)-propionamido]-2',5'-didésoxy-5-iodouridine,
5'-[2(RS)-(2,4,5-trichlorophénoxy)propionamido]-2',5'-didésoxy-5-iodouridine,
5'-[2-(2,6-dichlorophényl)-acétamido]-2',5'-didésoxy-5-iodouridine,
3'-O-benzyl-5'-[2(RS)-(2,4-dichlorophénoxy)-propionamido]-2',5'-didésoxy-5-éthyluridine,
5'-[2(RS)-(2,4-dichlorophénoxy)-propionamido]-2',5'-didésoxy-3'-O-éthyl-5-éthyluridine,
5'-[2(RS)-(2,4-dichlorophénoxy)-N-méthylpropionamido]-2',5'-didésoxy-5-éthyluridine,
5'-[2(R)-(2,4-dichlorophénoxy)propionamido]-2',5'-didésoxy-5-éthyluridine,
5'-[2(S)-(2,4-dichlorophénoxy)propionamido]-2',5'-didésoxy-5-éthyluridine,
5'-[2(RS)-(2,4-dichlorophénoxy)butyramido]-2',5'-didésoxy-5-éthyluridine,
5'-[2(RS)-(4-acétamido-2-chlorophénoxy)propionamido]-2',5'-didésoxy-5-éthyluridine,
5'-[2(RS)-(2-chloro-4-méthoxyphénoxy)propionamido]-2',5'-didésoxy-5-éthyluridine,
2',5'-didésoxy-5-éthyl-5'-[2(RS)-(2-méthylbiphénylyloxy)-propionamido]-uridine,
5'-[2(RS)-(2,4-dichlorophénoxy)propionamido]-5'-désoxythymidine,
5'-[2-(2,4-dichlorophénoxy)-2-méthylpropionamido]-2',5'-didésoxy-5-éthyluridine,
2',5'-didésoxy-5-éthyl-5'-[2(RS)-phénoxypropionamido]-uridine,
2',5'-didésoxy-5-éthyl-5'-[2(RS)-(2-fluorophénoxy)-propionamido]-uridine,
2',5'-didésoxy-5-éthyl-5'-[2(RS)-(2-trifluorométhyl)-phénoxy)propionamido]-uridine,
1-[5-[2(RS)-(2,4-dichlorophénoxy)propionamido]-2,5-didésoxy-2-fluoro-bêta-D-arabinofurannosyl]-thymine,
5'-[2(RS)-(2,4-dichlorophénylsulfinyl)propionamido]-2',5'-didésoxy-5-éthyluridine,
5'-[2(RS)-(2,4-dichlorophénylsulfonyl)propionamido]-2',5'-didésoxy-5-éthyluridine,
5'-[2-(2,6-dichlorophénylacétamido)éthyl]-2',5'-didésoxy-5-éthyluridine,
5'-[2-[2(RS)-(2,4,5-trichlorophénoxy)propionamido]-éthyl]-2',5'-didésoxy-5-éthyluridine,
5'-[2-[2(RS)-(2,4-dichlorophénoxy)propionamido]-éthyl]-2',5'-didésoxy-5-éthyluridine,
5'-[2(RS)-(2,6-dichlorobenzyl)-propionamido]-2',5'-didésoxy-5-éthyluridine,
5'-[2(RS)-(2,4-dichlorobenzyl)propionamo]-2',5'-didésoxy-5-éthyluridine,
5'-[2-chloro-2,3-dihydro-2(RS)-benzofurannylcarboxamido]-2',5'-didésoxy-5-éthyluridine,
5-(6-chloro-2H-1-benzopyranne-2-ylcarboxamido)-2',5'-didésoxy-5-éthyluridine,
5'-[2-(4-benzyloxy-2,6-diméthylphényl)acétamido]-2',5'-didésoxy-5-éthyluridine,
3'-O-acétyl-5'-[2-(4-benzyloxy-2,6-diméthylphényl)acétamido]-2',5'-didésoxy-5-éthyluridine,
3'-O-acétyl-2',5'-didésoxy-5-éthyl-5'-[2-(4-hydroxy-2,6-diméthylphényl)acétamido]-uridine,
2',5'-didésoxy-5-éthyl-5'-[2-(2,6-diméthyl-4-phosphatophényl)-acétamido]-uridine,
5-(2-chloroéthyl)-2',5'-didésoxy-5'-[2-(2,4-dichlorophénoxy)propionamido]-uridine,
5'-benzamido-5-(2-chloréthyl)-2',5'-didésoxyuridine,
5-(2-chloréthyl)-5'-[2-(2,4,5-trichlorophénoxy)-propionamido]-2',5'-didésoxyuridine,
5'-[2-(2,6-dichlorophényl)acétamido]-2',5'-didésoxy-5-propyluridine,
5'-[2(RS)-(2,4-dichlorophénoxy)propionamido]-2',5'-didésoxy-5-propyluridine et
5-acétyl-5'-[2-(2,6-dichlorophényl)acétamido]-2',5'-didésoxyuridine.

15. Les composés selon les revendications 1 à 4 pour l'utilisation en tant que médicaments, en particulier en tant qu'agents contre les virus.

16. Procédé de préparation des composés de formule générale I selon la revendication 1 et de leurs tautomères, caractérisé en ce que
a) on fait réagir un composé de formule générale

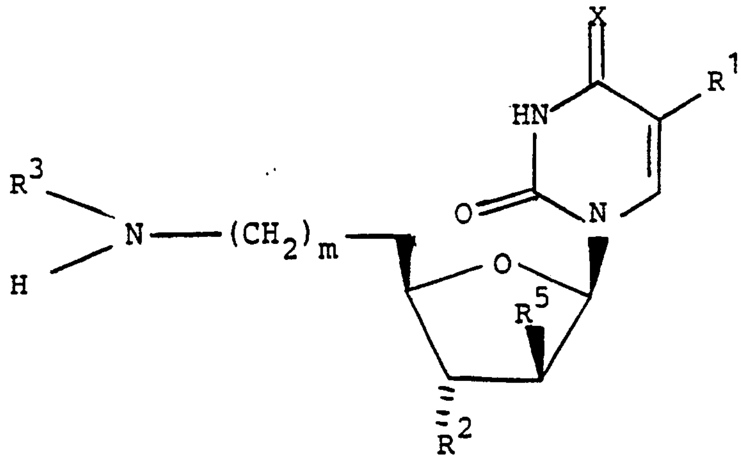

II

dans laquelle $R^1$, $R^2$, $R^3$, $R^5$, m et X ont les significations indiquées ci-dessus, ou un tautomère de ce composé, dans lequel un groupe 4-amino éventuellement présent est protégé si on le désire, avec un dérivé réactif d'un acide de formule générale

$R^4$-Y-COOH III

dans laquelle $R^4$ et Y ont les significations indiquées ci-dessus,
et on élimine un groupe protecteur éventuellement présent dans le produit obtenu, ou bien
b) pour préparer les composés de formule I ou un tautomère de l'un de ces composés, dans lesquels X représente l'oxygène et $R^2$ un groupe acyloxy, on acyle un composé de formule I ou un tautomère d'un tel composé, dans lequel X représente l'oxygène et $R^2$ un groupe hydroxy, ou bien
c) pour préparer un composé de formule I ou un tautomère d'un tel composé, dans lequel X représente l'oxygène et $R^2$ un groupe hydroxy, on soumet à désacylation un composé de formule I ou un tautomère d'un tel composé, dans lequel X représente l'oxygène et $R^2$ un groupe acyloxy, ou bien
d) pour préparer un composé de formule I ou un tautomère d'un tel composé, dans lequel Y représente un groupe de formule (a), Z représente SO ou $SO_2$ et n est égal à 1, on oxyde un composé de formule I ou un tautomère d'un tel composé, dans lequel Y représente un groupe de formule (a), Z représente le soufre et n est égal à 1, et
e) si on le désire, on soumet un substituant réactif présent dans $R^4$ à une modification fonctionnelle.

**17.** Compositions pharmaceutiques, en particulier à activité antivirale, contenant un composé selon les revendications 1 à 14 et un véhicule pharmaceutique.

**18.** Utilisation d'un composé selon les revendications 1 à 14 pour la préparation de médicaments prévus pour le traitement ou la prophylaxie des infections virales.

**REVENDICATIONS pour les Etats Contractants: AT,ES,GR**

**1.** Procédé de préparation des composés de formule générale

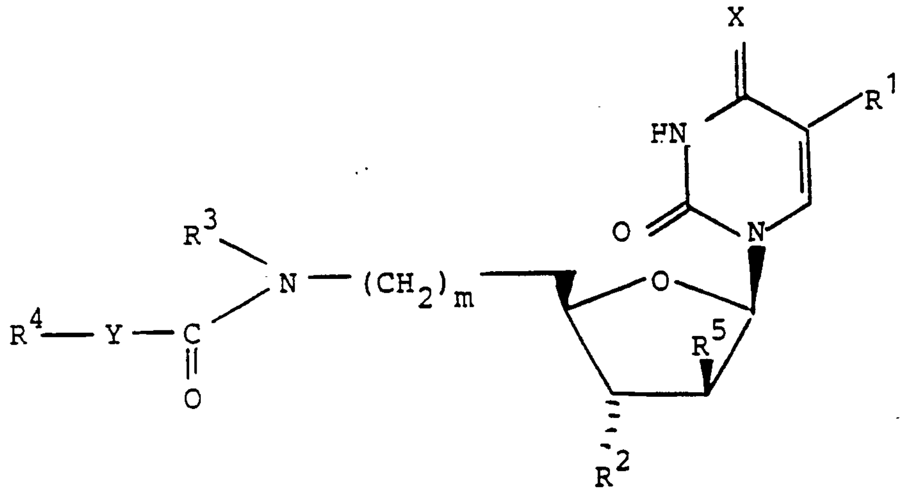

I

dans laquelle

$R^1$ représente un halogène, un groupe alkyle en C1-C4, halogénoalkyle en C1-C4 ou alcanoyle en C2-C4,

$R^2$ représente l'hydrogène, un groupe hydroxy, alcoxy en C1-C4, alkylthio en C1-C4 ou phénylalcoxy en C1-C4, ou bien encore, lorsque X représente l'oxygène, un groupe acyloxy;

$R^3$ représente l'hydrogène ou un groupe alkyle en C1-C4,

$R^4$ représente un groupe carbocyclique, à savoir un groupe hydrocarboné aromatique monocyclique ou polycyclique contenant jusqu'à 14 atomes de carbone dans la structure cyclique et qui peut porter un ou plusieurs substituants choisis parmi les halogènes, les groupes hydroxy, alkyle en C1-C4, alcoxy en C1-C4, trifluorométhyle, phényle, (alkyle en C1-C4)-phényle, halogénophényle, nitro, amino, acylamino, benzyloxy et O-phosphate, ou un groupe cycloalkyle monocyclique ou polycyclique contenant jusqu'à 13 atomes de carbone dans la structure cyclique et qui, lorsqu'il est monocyclique, peut porter un ou plusieurs cycles benzéniques condensés, ou un groupe hétérocyclique,

$R^5$ représente l'hydrogène ou le fluor,

m est égal à 0, 1 ou 2,

X représente l'oxygène ou le groupe NH,

Y représente une liaison directe, un groupe -CH=CH-,-C≡C- ou un groupe de formule

$(Z)_n$-A- (a)

dans laquelle

A représente un groupe alkylène en C1-C8 oui peut être substitué par un ou deux groupes phényle,

Z représente l'oxygène, le soufre, un groupe SO ou $SO_2$ et n est égal à 0 ou 1, sous réserve que $R^1$ ne peut représenter l'iode lorsque $R^2$ représente un groupe hydroxy ou benzoyloxy, $R^3$ l'hydrogène, $R^4$ un groupe phényle non substitué, $R^5$ l'hydrogène, m est égal à 0, X représente l'oxygène et Y une liaison directe,

et de leurs tautomères, caractérisé en ce que

a) ob fait réagir un composé de formule générale

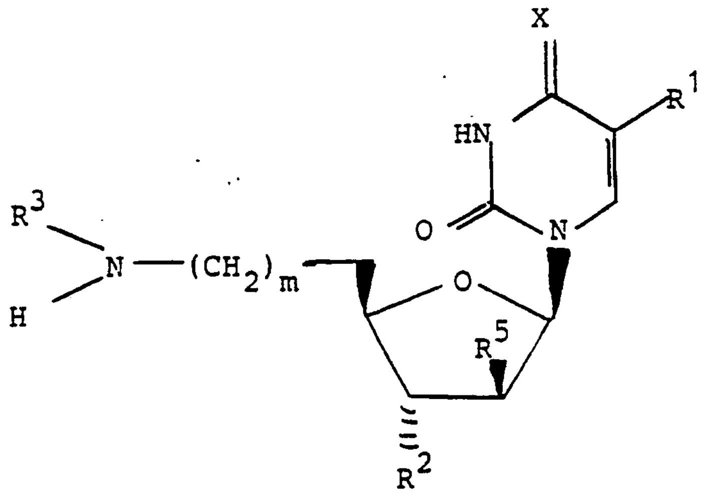

II

dans laquelle $R^1$, $R^2$, $R^3$, $R^5$, m et X ont les significations indiquées ci-dessus, ou un tautomère d'un tel composé, dans lequel un groupe 4-amino éventuellement présent est protégé si on le désire, avec un dérivé réactif d'un acide de formule générale

$R^4$-Y-COOH III

dans laquelle $R^4$ et Y ont les significations indiquées ci-dessus, et on élimine un groupe protecteur éventuellement présent dans le produit, ou bien

b) pour préparer les composés de formule I ou un tautomère d'un tel composé, dans lesquels X représente l'oxygène et $R^2$ un groupe acyloxy, on acyle un composé de formule I ou un tautomère d'un tel composé, dans lequel X représente l'oxygène et $R^2$ un groupe hydroxy, ou bien

c) pour préparer un composé de formule I ou un tautomère d'un tel composé, dans lequel X représente l'oxygène et $R^2$ un groupe hydroxy, on soumet à désacylation un composé de formule I ou un tautomère d'un tel composé, dans lequel X représente l'oxygène et $R^2$ un groupe acyloxy, ou bien

d) pour préparer un composé de formule I ou un tautomère d'un tel composé, dans lequel Y représente un groupe de formule (a), Z représente SO ou $SO_2$ et n est égal à 1, on oxyde un composé de formule I ou un tautomère d'un tel composé, dans lequel Y représente un groupe de formule (a), Z représente le soufre et n est égal à 1, et

e) si on le désire, on soumet un substituant réactif présent dans $R^4$ à une modification fonctionnelle.

2. Procédé selon la revendication 1, dans lequel $R^1$ représente le fluor, le chlore, le brome, un groupe alkyle en C1-C4 ou halogénoalkyle en C1-C4, $R^4$ représente un groupe carbocyclique ou aromatique-hétérocyclique, $R^5$ représente l'hydrogène et m est égal à 0.

3. Procédé selon la revendication 1 ou 2, dans lequel $R^1$ représente un groupe alkyle en C1-C4.

4. Procédé selon les revendications 1 à 3, dans lequel $R^2$ représente un groupe hydroxy.

5. Procédé selon les revendications 1 à 4, dans lequel $R^3$ représente l'hydrogène.

6. Procédé selon les revendications 1 à 5, dans lequel $R^4$ représente un groupe phényle qui peut porter un ou plusieurs substituants choisis parmi les halogènes, les groupes alkyle en C1-C4, alcoxy en C1-C4, trifluorométhyle, phényle et nitro.

7. Procédé selon les revendications 1 ou 3 à 6, dans lequel m = 0.

8. Procédé selon les revendications 1 à 7, dans lequel X représente l'oxygène.

9. Procédé selon les revendications 1 à 8, dans lequel Y représente un groupe de formule (a).

10. Procédé selon les revendications 1 à 7, dans lequel $R^1$ représente un groupe éthyle, $R^2$ un groupe hydroxy, $R^3$ l'hydrogène, $R^4$ un groupe 2-bromophényle, 2,6-dichlorophényle ou 4-biphénylyle, n = 0, X représente l'oxygène et Y un groupe de formule (a) dans laquelle A représente $-CH_2-$ ou $-CH(CH_3)-$ et n = 0.

11. Procédé selon les revendications 1 à.7 dans lequel $R^1$ représente un groupe éthyle ou propyle, $R^2$ un groupe hydroxy, $R^3$ l'hydrogène, $R^4$ un groupe 2-biphénylyle, 2,4-dichlorophényle, 2,4,5-trichlorophényle, 4-chloro-2-nitrophényle ou 2,4-dichloro-5-méthoxyphényle, m = 0, X représente l'oxygène et Y un groupe de formule (a) dans laquelle A représente $-CH(CH_3)-$ ou -CH(phényle)-, Z représente l'oxygène et n = 1.

12. Procédé selon la revendication 1 pour la préparation des composés :
5'-[2-(2-bromophényl)-acétamido]-2',5'-didésoxy-5-éthyl-uridine,
5'-[2-(2,6-dichlorophényl)-acétamido]-2',5'-didésoxy-5-éthyluridine,
5'-[2(RS)-(2,4-dichlorophénoxy)-propionamido]-2',5'-didésoxy-5-éthyluridine,
5'-[2-(4-biphénylyl)-acétamido]-2',5'-didésoxy-5-éthyl-uridine,
2',5'-didésoxy-5-éthyl-5'-[2(RS)-(2-phénylphénoxy)-propionamido]-uridine,
1-[5-[5(RS)-(2,4-dichlorophénoxy)-propionamido]-2,5-didésoxy-2-fluoro-bêta-D-arabinofurannosyl]-5-éthyl-uracile,
5'-[2(RS)-(2,4,5-trichlorophénoxy)-propionamido]-2',5'-didésoxy-5-éthyluridine,
5'-[2(RS)-(4-chloro-2-nitrophénoxy)-propionamido]-2',5'-didésoxy-5-éthyluridine,
5'-[2(RS)-(2,4-dichlorophénoxy)-2-phénylacétamido]-2',5'-désoxy-5-éthyluridine,
5'-[2(RS)-(2,4-dichloro-5-méthoxyphénoxy)-propionamido]-2',5'-didésoxy-5-éthyluridine et
5'-[2(RS)-(2,4-dichlorophénoxy)-propionamido]-2',5'-didésoxy-5-propyluridine.

13. procédé selon la revendication 2, pour la préparation des composés
2',5'-didésoxy-5-éthyl-5'-(2-phénylacétamido)-uridine,
5'-(4-bromobenzamido)-5'-désoxythymidine,
5'-désoxy-5'-(4-nitrobenzamido)-thymidine,
5'-benzamido-5'-désoxythymidine,
5'-désoxy-5'-(2-fluorobenzamido)-thymidine,
5'-désoxy-5'-(2-nitrobenzamido)-thymidine,
5'-[2-(2-bromophényl)-acétamido]-5'-désoxythymidine,
5'-désoxy-5'-[2-(4-nitrophényl)-acétamido]-thymidine,
5'-désoxy-5'-(2-phénylacétamido)-thymidine,
5'-désoxy-5'-[2-(4-trifluorométhylphényl)acétamido]-thymidine,
5'-benzamido-2',5'-didésoxy-5-éthyluridine,
2',5'-didésoxy-5-éthyl-5'-(2-fluorobenzamido)-uridine,
5'-(2-bromobenzamido)-2',5'-didésoxy-5-éthyluridine,
2',5'-didésoxy-5-éthyl-5'-(4-nitrobenzamido)-uridine,
2',5'-didésoxy-5-éthyl-5'-(2-trifluorométhylbenzamido)-uridine,
2',5'-didésoxy-5-éthyl-5'-[2-(2,6-diméthylphényl)-acétamido]-uridine,
5'-[2(RS)-(2-bromophényl)-propionamido]-2',5'-didésoxy-5-éthyluridine,
5'-[2-(2-chloro-3-nitrophényl)-acétamido]-2',5'-didésoxy-5-éthyluridine,
5'-[2(RS)-(2,6-dichlorophényl)-propionamido]-2',5'-didésoxy-5-éthyluridine,
2',5'-didésoxy-5-éthyl-5'-[2-(3,5-diméthylphényl)-acétamido]-uridine,
2',5'-didésoxy-5'-[2-(3,5-diméthoxyphényl)-acétamido]-5-éthyluridine,
2',5'-didésoxy-5-éthyl-5'-[2-(2,3,5,6)-tétraméthylphényl)-acétamido]-uridine,
2',5'-didésoxy-5-éthyl-5'-[2-(2-méthoxyphényl)acétamido]-uridine,
2',5'-didésoxy-5-éthyl-5'-[2-(2-nitrophényl)]-acétamido-uridine,
5-bromo-5'-[2-(2-bromophényl)-acétamido]-2',5'-didésoxy-uridine,
3'-O-butyryl-5'-[2-(2,6-dichlorophényl)acétamido]-2',5'-didésoxy-5-éthyluridine,
2',5'-didésoxy-5'-[2-(2,6-dichlorophényl)acétamido]-3'-O-(3,3-diméthylbutyryl)-5-éthyluridine,
5'-[2-(2,6-dichlorophényl)acétamido]-2',5'-didésoxy-5-éthyl-3'-O-palmitoyluridine,
3'-O-acétyl-5'-[2-(2,6-dichlorophényl)-acétamido]-2',5'-didésoxy-5-éthyluridine,
3'-O-benzyl-5'-[2-(2,6-dichlorophényl)acétamido]-2',5'-didésoxy-5-éthyluridine,
5'-[2-(2,6-dichlorophényl)acétamido]-2',5'-didésoxy-3'-O-éthyl-5-éthyluridine,
2',3',5'-tridésoxy-5'-[2-(2,6-dichlorophényl)acétamido]-5-éthyluridine,

5'-[2-(2-aminophényl)acétamido]-2',5'-didésoxy-5-éthyl-uridine,
5'-[2-(2-acétamidophényl)acétamido]-3'-O-acétyl-2',5'-didésoxy-5-éthyluridine,
5'-[2-(2-acétamidophényl)acétamido]-2',5'-didésoxy-5-éthyluridine,
2',5'-didésoxy-5-éthyl-5'-[2-(4-hydroxy-2,6-diméthyl-phényl)acétamido]-uridine,
5-(2-chloroéthyl)-5'-[2-(2,6-dichlorophényl)acétamido]-2',5'-didésoxyuridine,
5'-[2-(2-bromophényl)-N-méthylacétamido]-2',5'-didésoxy-5-éthyluridine,
5'-[2-(2,6-dichlorophényl)-N-méthylacétamido]-2',5'-didésoxy-5-éthyluridine,
5'-[2(RS)-(2-bromophényl)-N-méthylpropionamido]-2',5'-didésoxy-5-éthyluridine,
3'-O-acétyl-5'-[2-(2-bromophényl)-N-méthylacétamido]-2',5'-didésoxy-5-éthyluridine,
2',5'-didésoxy-5-éthyl-5'-(2-naphtalamido)-uridine,
2',5'-didésoxy-5-éthyl-5'-(2-phénylbenzamido)-uridine,
2',5'-didésoxy-5-éthyl-5'-(3-phénylpropionamido)-uridine,
2',5'-didésoxy-5-éthyl-5'-(4-phénylbutyramido)-uridine,
2',5'-didésoxy-5'-cinnamamido-5-éthyluridine,
2',5'-didésoxy-5-éthyl-5'-(3-phényl-2-propynamido)-uridine,
2',5'-didésoxy-5-éthyl-5'-[3-(phénylsulfonyl)-propionamido]-uridine,
5'-(2-bromocinnamamido)-2',5'-didésoxy-5-éthyluridine,
2',5'-didésoxy-5-éthyl-5'-[2-(2-thiényl)-acétamido]-uridine,
2',5'-didésoxy-5-éthyl-5'-[2-(3-thiényl)acétamido]-uridine,
5'-(1-adamantylcarboxamido)-2',5'-didésoxy-5-éthyluridine,
2',5'-didésoxy-5-éthyl-5'-(2-pyridylcarboxamido)-uridine,
2',5'-didésoxy-5-éthyl-5'-(3-pyridylcarboxamido)-uridine,
2',5'-didésoxy-5-éthyl-5'-[2-(phénylsulfonyl)acétamido]-uridine,
2',5'-didésoxy-5-éthyl-5'-(9-fluorénylcarboxamido)-uridine,
3'-O-acétyl-2',5'-didésoxy-5-éthyl-5'-(9-fluorénylcarboxamido)-uridine,
3'-O-butyryl-2',5'-didésoxy-5-éthyl-5'-(9-fluorénylcarboxamido)-uridine,
2',5'-didésoxy-5-éthyl-3'-O-(3,3-diméthylbutyryl)-5'-(9-fluorénylcarboxamido)uridine,
2',5'-didésoxy-5-éthyl-5'-(9-fluorénylcarboxamido)-3'-O-(hexadécanoyl)-uridine,
2',5'-didésoxy-5-éthyl-3'-O-(9-fluorénylcarbonyl)-5'-(9-fluorénylcarboxamido)-uridine,
2',5'-didésoxy-5-éthyl-5'-(2-Phénylbutyramido)-uridine,
2',5'-didésoxy-5-éthyl-5'-(3-phénylbutyramido)-uridine,
2',5'-didésoxy-5-éthyl-5'-(2,2-diphénylacétamido)-uridine,
5'-(2-cyclohexyl-2-phénylacétamido)-2',5'-didésoxy-5-éthyluridine,
2',5'-didésoxy-5'-éthyl-5'-(triphénylacétamido)-uridine,
2',5'-didésoxy-5-éthyl-5'-(2-méthyl-2-phénylpropionamido)-uridine,
2',5'-didésoxy-5-éthyl-5'-[2(RS)-phénylpropionamido)]-uridine,
5'-[2(RS)-(2,4-dichlorophénoxy)propionamido]-2',5'-didésoxy-5-éthyluridine,
5'-[2(RS)-(2,6-dichlorophénoxy)-propionamido]-2',5'-didésoxy-5-éthyluridine,
5'-[2(RS)-(3,5-dichlorophénoxy)propionamido]-2',5'-didésoxy-5-éthyluridine,
5'-[2(RS)-(2-chlorophénoxy)propionamido]-2',5'-didésoxy-5-éthyluridine,
5'-[2-(2,6-dichlorophénoxy)acétamido]-2',5'-didésoxy-5-éthyluridine,
5'-[2-(2,4-dichlorophénoxy)-acétamido]-2',5'-didésoxy-5-éthyluridine,
5'-[4-(2,6-dichlorophénoxy)-butyramido]-2',5'-didésoxy-5-éthyluridine,
5'-[6-(2,6-dichlorophénoxy)-hexanamido]-2',5'-didésoxy-5-éthyluridine,
5'-[5-(2,6-dichlorophénoxy)-valéramido]-2',5'-didésoxy-5-éthyluridine,
5'-[2(RS)-(2-chloro-4-nitrophénoxy)-propionamido]-2',5'-didésoxy-5-éthyluridine,
5'-[2(RS)-(2-chloro-4-phénylphénoxy)propionamido]-2',5'-didésoxy-5-éthyluridine,
5-bromo-5'-[2-(2-bromophényl)acétamido]-2',5'-didésoxy-cytidine et
5'-[2-(2-bromophényl)-acétamido]-2',5'-didésoxy-5-éthyl-cytidine.

14. Procédé selon la revendication 1 pour la préparation des composés
5'-[2-(2,6-dichlorophényl)-acétamido]-5'-désoxythymidine,
5-bromo-5'-[2(RS)-(2,4-dichlorophénoxy)propionamido]-2',5'-didésoxyuridine,
5-bromo-5'-[2-(2,6-dichlorophényl)-acétamido]-2',5'-didésoxyuridine,
5'-benzamido-5-bromo-2',5'-didésoxyuridine,
5'-[2(RS)-(2,4-dichlorophénoxy)-propionamido]-2',5'-didésoxy-5-iodouridine,
5'-[2(RS)-(2,4,5-trichlorophénoxy)propionamido]-2',5'-didésoxy-5-iodouridine,
5'-[2-(2,6-dichlorophényl)-acétamido]-2',5'-didésoxy-5-iodouridine,

3'-O-benzyl-5'-[2(RS)-(2,4-dichlorophénoxy)-propionamido]-2',5'-didésoxy-5-éthyluridine,
5'-[2(RS)-(2,4-dichlorophénoxy)-propionamido]-2',5'-didésoxy-3'-O-éthyl-5-éthyluridine,
5'-[2(RS)-(2,4-dichlorophénoxy)-N-méthylpropionamido]-2',5'-didésoxy-5-éthyluridine,
5'-[2(R)-(2,4-dichlorophénoxy)propionamido]-2',5'-didésoxy-5-éthyluridine,
5'-[2(S)-(2,4-dichlorophénoxy)-propionamido]-2',5'-didésoxy-5-éthyluridine,
5'-[2(RS)-(2,4-dichlorophénoxy)-butyramido]-2',5'-didésoxy-5-éthyluridine,
5'-[2(RS)-(4-acétamido-2-chlorophénoxy)-propionamido]-2',5'-didésoxy-5-éthyluridine,
5'-[2(RS)-(2-chloro-4-méthoxyphénoxy)-propionamido]-2',5'-didésoxy-5-éthyluridine,
2',5'-didésoxy-5-éthyl-5'-[2(RS)-(2-méthylbiphénylyloxy)-propionamido]-uridine,
5'-[2(RS)-(2,4-dichlorophénoxy)propionamido]-5'-désoxy-thymidine,
5'-[2-(2,4-dichlorophénoxy)-2-méthylpropionamido]-2',5'-didésoxy-5-éthyluridine,
2',5'-didésoxy-5-éthyl-5'-[2(RS)-phénoxypropionamido]-uridine,
2',5'-didésoxy-5-éthyl-5'-[2(RS)-(2-fluorophénoxy)-propionamido]-uridine,
2',5'-didésoxy-5-éthyl-5'-[2(RS)-(2-trifluorométhyl)-phénoxy)propionamido]-uridine,
1-[5-[2(RS)-(2,4-dichlorophénoxy)propionamido]-2,5-didésoxy-2-fluoro-bêta-D-arabinofurannosyl]-thymine,
5'-[2(RS)-(2,4-dichlorophénylsulfinyl)propionamido]-2',5'-didésoxy-5-éthyluridine,
5'-[2(RS)-(2,4-dichlorophénylsulfonyl)-propionamido]-2',5'-didésoxy-5-éthyluridine,
5'-[2-(2,6-dichlorophénylacétamido )éthyl]-2',5'-didésoxy-5-éthyluridine,
5'-[2-[2(RS)-(2,4,5-trichlorophénoxy)-propionamido]-éthyl]-2',5'-didésoxy-5-éthyluridine,
5'-[2-[2(RS)-(2,4-dichlorophénoxy)-propionamido]-éthyl]-2',5'-didésoxy-5-éthyluridine,
5'-[2(RS)-(2,6-dichlorobenzyl)-propionamido]-2',5'-didésoxy-5-éthyluridine,
5'-[2(RS)-(2,4-dichlorobenzyl)-propionamido]-2',5'-didésoxy-5-éthyluridine,
5'-[5-chloro-2,3-dihydro-2(RS)-benzofurannylcarboxamido]-2',5'-didésoxy-5-éthyluridine,
5-(6-chloro-2H-1-benzopyranne-2-ylcarboxamido)-2',5'-didésoxy-5-éthyluridine,
5'-[2-(4-benzyloxy-2,6-diméthylphényl)-acétamido]-2',5'-didésoxy-5-éthyluridine,
3'-O-acétyl-5'-[2-(4-benzyloxy-2,6-diméthylphényl)-acétamido]-2',5'-didésoxy-5-éthyluridine,
3'-O-acétyl-2',5'-didésoxy-5-éthyl-5'-[2-(4-hydroxy-2,6-diméthylphényl)-acétamido]-uridine,
2',5'-didésoxy-5-éthyl-5'-[2-(2,6-diméthyl-4-phosphatophényl)-acétamido]-uridine,
5-(2-chloroéthyl)-2',5'-didésoxy-5'-[2-(2,4-dichlorophénoxy)-propionamido]-uridine,
5'-benzamido-5-(2-chloréthyl)-2',5'-didésoxyuridine,
5-(2-chloréthyl)-5'-[2-(2,4,5-trichlorophénoxy)-propionamido]-2',5'-didésoxyuridine,
5'-[2-(2,6-dichlorophényl)-acétamido]-2',5'-didésoxy-5-propyluridine,
5'-[2(RS)-(2,4-dichlorophénoxy)propionamido]-2',5'-didésoxy-5-propyluridine et
5-acétyl-5'-[2-(2,6-dichlorophényl)-acétamido]-2',5'-didésoxyuridine.

**15.** Procédé de préparation de compositions pharmaceutiques, en particulier de compositions pharmaceutiques à activité antivirale, caractérisé en ce que l'on met un composé de formule I ou un tautomère d'un tel composé sous une forme d'administration galénique.

**16.** Utilisation d'un composé de formule I ou d'un tautomère d'un tel composé pour la préparation de médicaments pour le traitement ou la prophylaxie des infections virales.